(19) Europäisches Patentamt / European Patent Office / Office européen des brevets

(11) **EP 4 559 896 A1**

(12) **EUROPEAN PATENT APPLICATION**
published in accordance with Art. 153(4) EPC


(43) Date of publication:
**28.05.2025 Bulletin 2025/22**

(21) Application number: **23842694.4**

(22) Date of filing: **31.05.2023**

(51) International Patent Classification (IPC):
***C07C 237/08** (2006.01)* ***A61K 9/51** (2006.01)*
***A61K 31/7088** (2006.01)* ***A61K 31/7105** (2006.01)*
***A61K 47/10** (2017.01)* ***A61K 47/18** (2017.01)*
***A61K 47/28** (2006.01)* ***A61K 47/34** (2017.01)*
***A61K 48/00** (2006.01)* ***C07C 231/02** (2006.01)*
***C12N 15/88** (2006.01)*

(52) Cooperative Patent Classification (CPC):
**C07C 237/06; A61K 9/51; A61K 31/7088; A61K 31/7105; A61K 47/10; A61K 47/18; A61K 47/28; A61K 47/34; A61K 48/00; C07C 231/02; C07C 237/08; C07D 207/16; C07D 209/14; C07D 209/16; C07D 211/62;** (Cont.)

(86) International application number:
**PCT/JP2023/020385**

(87) International publication number:
**WO 2024/018762 (25.01.2024 Gazette 2024/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.07.2022 JP 2022115015**



(71) Applicant: **National University Corporation Hokkaido University**
**Hokkaido 060-0808 (JP)**

(72) Inventors:
- **SATO, Yusuke**
**Sapporo-shi, Hokkaido 060-0808 (JP)**
- **HARASHIMA, Hideyoshi**
**Sapporo-shi, Hokkaido 060-0808 (JP)**
- **SUZUKI, Yuichi**
**Sapporo-shi, Hokkaido 060-0808 (JP)**

(74) Representative: **Müller-Boré & Partner Patentanwälte PartG mbB**
**Friedenheimer Brücke 21**
**80639 München (DE)**


(54) **PH-SENSITIVE CATIONIC LIPID AND LIPID NANOPARTICLE**


(57) The present invention addresses the problem of providing, without using relatively expensive starting materials, a more conveniently synthesizable pH-sensitive cationic lipid and a lipid nanoparticle that contains this pH-sensitive cationic lipid. The present invention is a pH-sensitive cationic lipid represented by general formula (I). [$R^1$ is a C1-22 hydrocarbon group; the three $R^1$ groups in one molecule may be the same as or different from each other; $Z^1$ is a C1-6 alkylene group or a single bond; X is $-N(R^2)(R^3)$ or is a 5- to 7-membered nonaromatic heterocyclic group (with the proviso that this group is bonded to $Z^1$ via a carbon atom); and $R^2$ and $R^3$ are each independently a hydrogen atom or a C1-4 hydrocarbon group, or $R^2$ and $R^3$ may be bonded to each other to form a 5- to 7-membered nonaromatic heterocycle.]

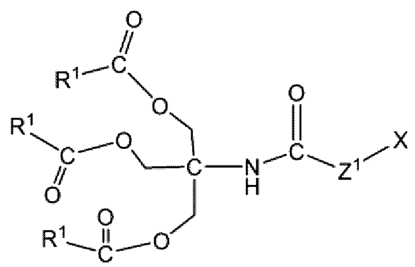
(I)


EP 4 559 896 A1

(52) Cooperative Patent Classification (CPC): (Cont.)
**C07D 213/38; C07D 295/088; C07D 295/108; C07D 413/04; C07D 471/10; C12N 15/88**

## Description

### Technical Field

The present invention relates to a pH-sensitive cationic lipid having three hydrophobic chains and a lipid nanoparticle containing the pH-sensitive cationic lipid.

This application claims priority to Japanese Patent Application No. 2022-115015 filed in Japan on July 19, 2022, the contents of which are incorporated herein by reference.

### Background Art

Lipid nanoparticles (LNPs) are used as carriers to encapsulate fat-soluble drugs and nucleic acids such as siRNA (short interfering RNA) and mRNA and deliver them to target cells. For example, as lipid nanoparticles that serve as carriers for efficiently delivering nucleic acids such as siRNA into target cells, lipid nanoparticles have been reported that contain pH-sensitive cationic lipids, which are electrically neutral at physiological pH and change to cationic in a weakly acidic pH environment such as an endosome, as constituent lipids (Patent Literature 1). Lipid nanoparticles carrying a positive charge in the blood interact non-specifically with plasma proteins and are rapidly cleared from the blood by the reticuloendothelial system. In contrast, lipid nanoparticles containing pH-sensitive cationic lipids as constituent lipids are not charged under neutral conditions in the blood, but are positively charged under weakly acidic conditions in endosomes and can efficiently escape from endosomes.

As a pH-sensitive cationic lipid, for example, Jayaraman et al. developed DLin-MC3-DMA and achieved an $ED_{50}$ of 0.005 mg siRNA/kg in factor VII (F7) knockdown in mouse liver (Non-patent Literature 1). The present inventors have also previously developed their own pH-sensitive cationic lipids YSK05 and YSK13-C3, achieving $ED_{50}$s of 0.06 and 0.015 mg siRNA/kg, respectively, in F7 knockdown (Non-patent Literatures 2-4). The present inventors have further developed compounds having two hydrocarbon chains and one hydrophilic group, such as 7-(4-(diisopropylamino)butyl)-7-hydroxytridecane-1,13-diyl dioleate (CL4H6), as pH-sensitive cationic lipids (Patent Literature 1).

On the other hand, as a method for producing lipid nanoparticles encapsulating nucleic acids and the like, there is a method based on the principle of alcohol dilution using a flow path. For example, it has been reported that lipid nanoparticles having a diameter of about 30 nm can be reproducibly produced by using a microchannel with a built-in three-dimensional micromixer capable of achieving instantaneous mixing of two liquids (Non-patent Literature 5). It has also been reported that by using a simple two-dimensional flow path structure in which baffles of a certain width are arranged alternately from both sides in a micro-sized flow path through which a raw material solution flows, it is possible to form a nano-sized lipid particle formation system with higher particle size controllability than the conventional flow path structure using a three-dimensional mixer (Patent Literature 2). Recently, these methods for producing nanoparticle formulations have been mainly employed for the production of lipid nanoparticles (LNPs) loaded with fat-soluble drugs and nucleic acids such as siRNA (short interfering RNA) or mRNA.

### Citation List

#### Patent Literatures

Patent Literature 1: International Publication No. WO 2018/230710
Patent Literature 2: International Publication No. WO 2018/190423

#### Non Patent Literatures

Non Patent Literature 1: Jayaraman et al., Angewandte Chemie International Edition, 2012, vol. 51, pp. 8529-8533.
Non Patent Literature 2: Watanabe et al., Scientific Reports, 2014, 4:4750, DOI: 10.1038/srep04750.
Non Patent Literature 3: Yamamoto et al., Journal of Hepatology, 2016, vol. 64, pp. 547-555.
Non Patent Literature 4: Sato et al., Molecular Therapy, 2016, vol. 24, pp. 788-795.
Non Patent Literature 5: Leung et al., Journal of Physical Chemistry C Nanomater Interfaces, 2012, vol. 116(34), pp. 18440-18450.

### Summary of Invention

Problems to be solved by the invention

When a compound is mass-produced in a factory, it is preferable that it can be synthesized using inexpensive raw materials and in a small number of reaction steps from the viewpoints of production cost, quality stability, and the like. However, the synthesis reaction of pH-sensitive cationic lipids described in Patent Literature 1, such as CL4H6, is a multi-step reaction of as many as 6 to 7 steps and further requires a Grignard reaction using an organometallic compound. The following shows the outline of the synthesis reaction of CL4H6.

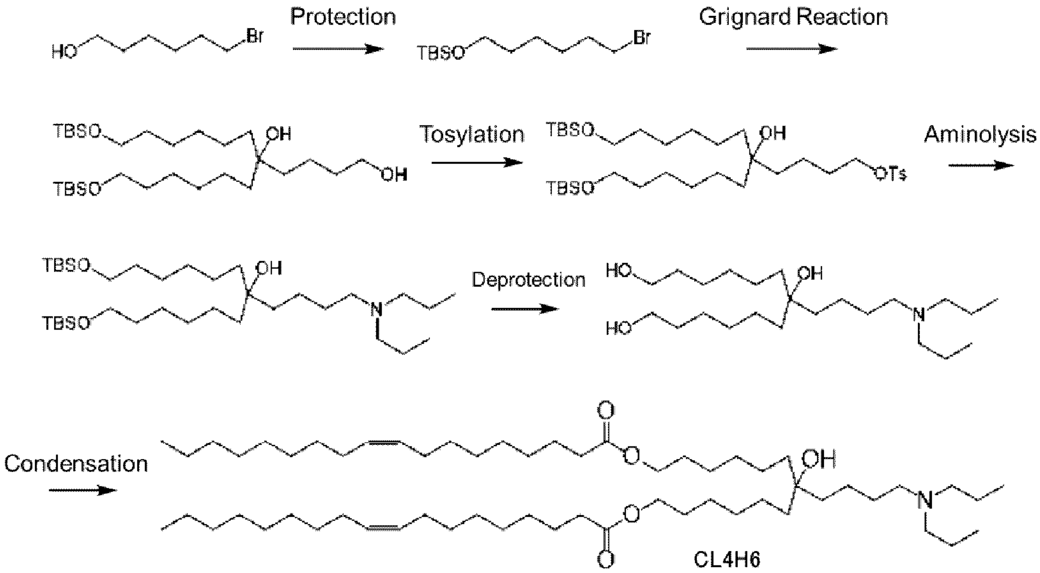

CL4H6

An object of the present invention is to provide a pH-sensitive cationic lipid that can be synthesized more easily in fewer steps without using an organometallic reaction or a relatively expensive raw material, and a lipid nanoparticle containing the pH-sensitive cationic lipid.

Means for solution of the problems

The present inventors have found that a pH-sensitive cationic lipid can be synthesized in a smaller number of steps without using a relatively expensive raw material by using tris(hydroxymethyl)aminomethane (CAS number: 77-86-1) (hereinafter abbreviated as "Tris") as a linker, bonding a hydrocarbon chain to each of the three hydroxyl groups of Tris, and bonding one hydrophilic group to the amino group of Tris, thereby completing the present invention.

That is, the present invention provides the following pH-sensitive cationic lipids and the like.

[1] A pH-sensitive cationic lipid comprising a compound represented by the following formula (I):

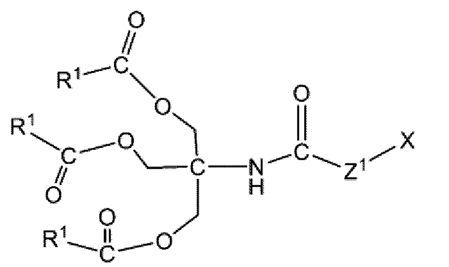
(I)

wherein, in formula (I), $R^1$ is a hydrocarbon group having 1 to 22 carbon atoms; three $R^1$ groups in one molecule may be the same as or different from each other; $Z^1$ is an alkylene group having 1 to 6 carbon atoms which may be substituted with one hydroxyalkyl group having 1 to 3 carbon atoms, or a single bond; X is $-N(R^2)(R^3)$ or a 5-to 7-membered nonaromatic heterocyclic group, wherein the group is bonded to $Z^1$ via a carbon atom, and the ring may be substituted with one or two hydrocarbon groups having 1 to 4 carbon atoms or amide groups; and $R^2$ and $R^3$ are each independently a hydrogen atom or a hydrocarbon group having 1 to 4 carbon atoms in which one or two hydrogen atoms may be substituted with a phenyl group, a pyrrolyl group, a pyrimidyl group, or an indolyl group, or $R^2$ and $R^3$

may be bonded to each other to form a 5- to 7-membered nonaromatic heterocycle in which one or two hydrogen atoms of the ring may be substituted with a hydrocarbon group having 1 to 4 carbon atoms, an amide group, or a nitrogen-containing cyclic group, and one carbon atom of the ring may be a carbon atom constituting other nitrogen-containing cyclic group.

[2] The pH-sensitive cationic lipid according to [1], wherein $-Z^1$-X in the general formula (I) is any one of the following formulas (H-1) to (H-8) and (H-16) to (H-26):

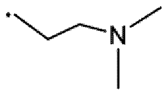
(H-1)

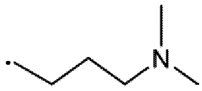
(H-2)

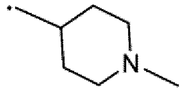
(H-3)

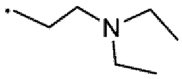
(H-4)

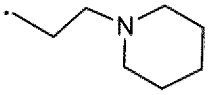
(H-5)

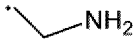
(H-6)

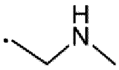
(H-7)

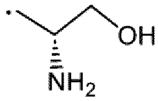
(H-8)

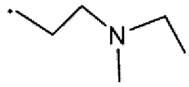
(H-16)

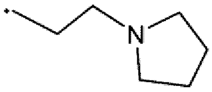
(H-17)

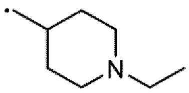
(H-18)

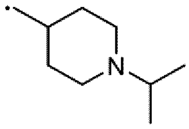
(H-19)

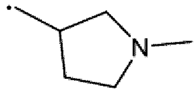
(H-20)

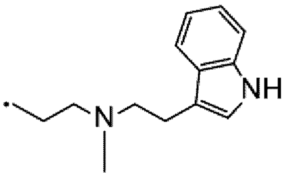
(H-21)

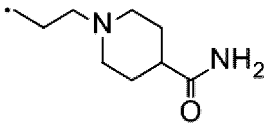
(H-22)

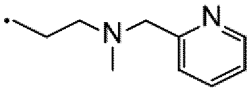
(H-23)

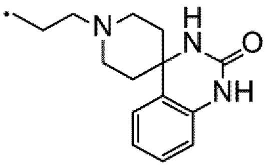
(H-24)

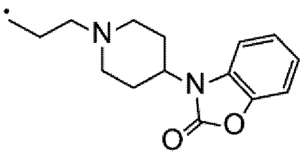
(H-25)

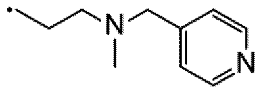
(H-26)

[3] The pH-sensitive cationic lipid according to [1] or [2], wherein the three $R^1$ groups in one molecule are all the same.

[4] The pH-sensitive cationic lipid according to any one of [1] to [3], wherein $R^1$ is an alkyl group having 15 to 22 carbon atoms or an alkenyl group having 15 to 22 carbon atoms.

[5] The pH-sensitive cationic lipid according to any one of [1] to [4], which is a compound represented by any one of the following general formulas (I-1) to (I-8) and (I-16) to (I-26):

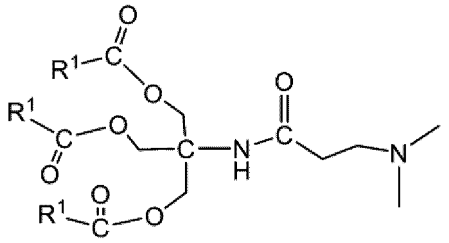

(I-1)

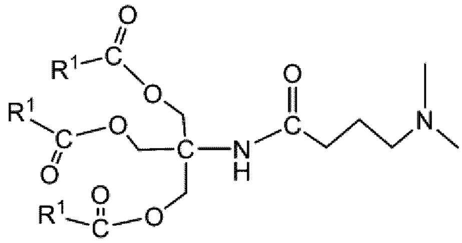

(I-2)

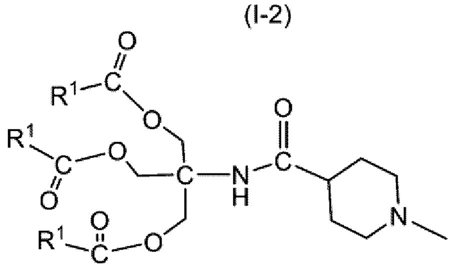

(I-3)

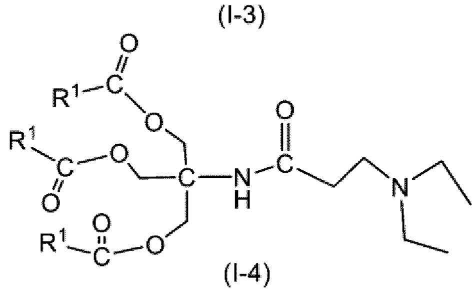

(I-4)

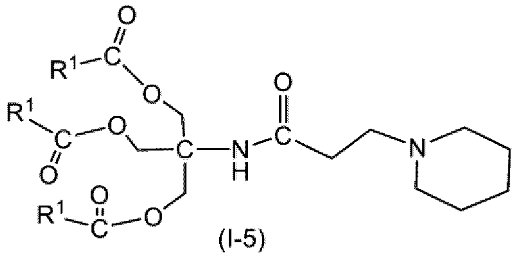

(I-5)

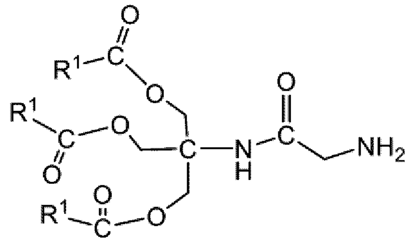

(I-6)

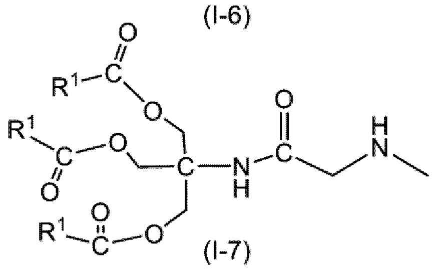

(I-7)

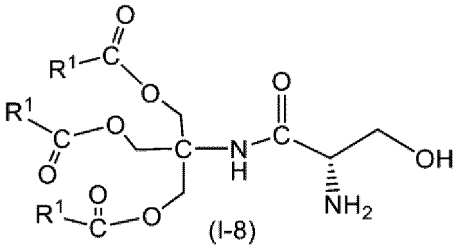

(I-8)

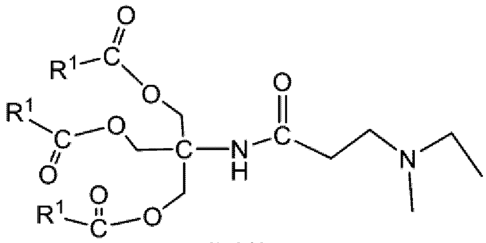

(I-16)

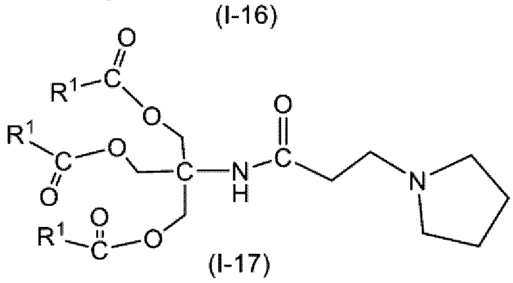

(I-17)

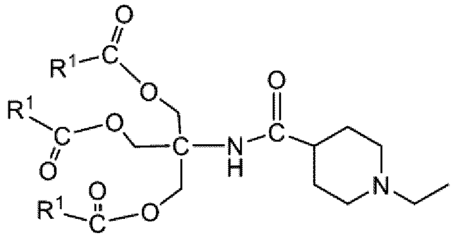

(I-18)

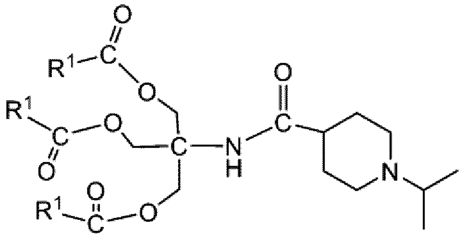

(I-19)

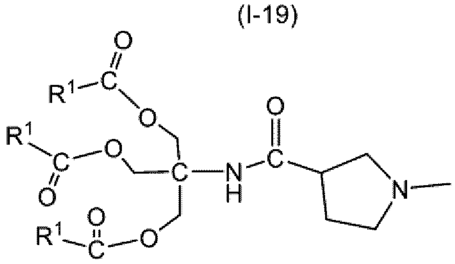

(I-20)

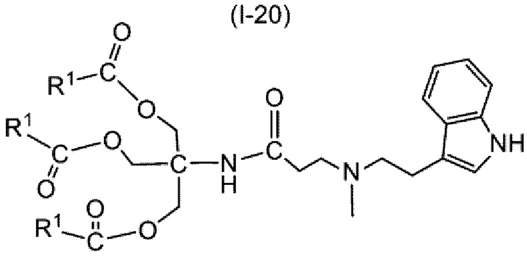

(I-21)

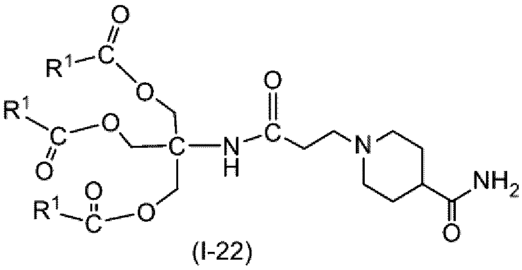

(I-22)

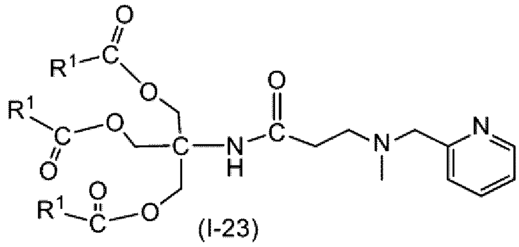

(I-23)

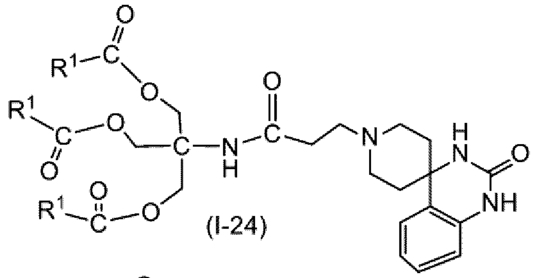

(I-24)

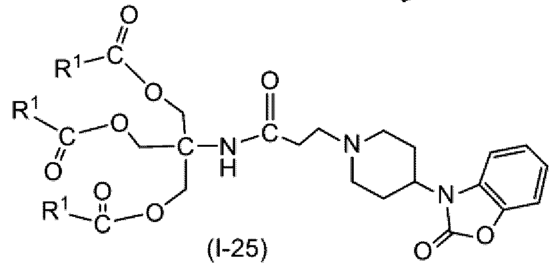

(I-25)

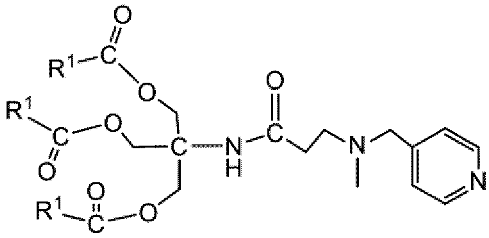

(I-26)

wherein $R^1$ is the same as defined above.

[6] A pH-sensitive cationic lipid comprising a compound having a Tris skeleton, wherein

a fatty acid residue is bonded to each of three oxygen atoms of the Tris skeleton, the fatty acid residue has 1 to 22 carbon atoms in a hydrocarbon chain moiety thereof and may have one or more unsaturated bonds, and the three fatty acid residues in one molecule may be the same group or two or more different groups, and

a primary to tertiary amine is linked to one nitrogen atom of the Tris skeleton via a carbonyl group or a carbonyl group followed by an alkylene group having 1 to 6 carbon atoms, and the alkylene group may be substituted with one hydroxyalkyl group having 1 to 3 carbon atoms

the compound has a pKa of 4.0 to 9.0.

[7] A method for producing a pH-sensitive cationic lipid, the method comprising condensing a compound represented by the following general formula (A), a compound represented by the following general formula (B), and tris(hydroxymethyl)aminomethane to produce a pH-sensitive cationic lipid comprising a compound represented by the following

general formula (I):

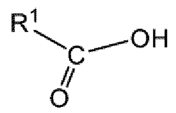

(A)

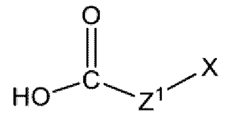

(B)

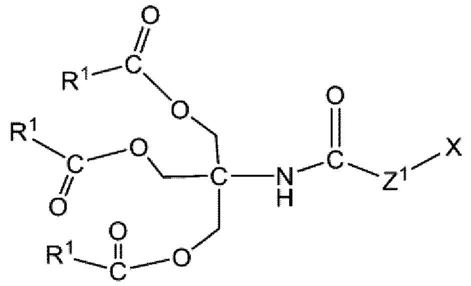

(I)

wherein, in formula (A), $R^1$ is an alkyl group having 1 to 22 carbon atoms or an alkenyl group having 2 to 22 carbon atoms;

wherein, in formula (B), $Z^1$ is an alkylene group having 1 to 6 carbon atoms which may be substituted with one hydroxyalkyl group having 1 to 3 carbon atoms, or a single bond; X is $-N(R^2)(R^3)$ or a 5- to 7-membered nonaromatic heterocyclic group, wherein the group is bonded to $Z^1$ via a carbon atom, and the ring may be substituted with one or two hydrocarbon groups having 1 to 4 carbon atoms or amide groups; and $R^2$ and $R^3$ are each independently a hydrogen atom or a hydrocarbon group having 1 to 4 carbon atoms in which one or two hydrogen atoms may be substituted with a phenyl group, a pyrrolyl group, a pyrimidyl group, or an indolyl group, or $R^2$ and $R^3$ may be bonded to each other to form a 5- to 7-membered nonaromatic heterocycle in which one or two hydrogen atoms of the ring may be substituted with a hydrocarbon group having 1 to 4 carbon atoms, an amide group, or a nitrogen-containing cyclic group, and one carbon atom of the ring may be a carbon atom constituting other nitrogen-containing cyclic group;

wherein, in formula (I), $R^1$, $Z^1$, and X are the same as defined above, but three $R^1$ groups in one molecule may be the same as or different from each other.

[8] A method for producing a pH-sensitive cationic lipid, the method comprising adding N-[tris(hydroxymethyl)methyl] acrylamide to a compound represented by the following general formula (C), and then further condensing a resulting compound with a compound represented by the following general formula (A) to produce a pH-sensitive cationic lipid comprising a compound represented by the following general formula (I'):

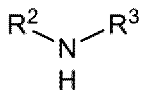

(C)

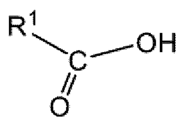

(A)

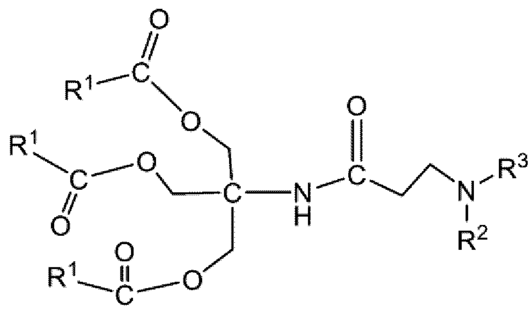

(I')

wherein, in formulas (C) and (I'), $R^2$ and $R^3$ are each independently a hydrogen atom or a hydrocarbon group having 1 to 4 carbon atoms in which one or two hydrogen atoms may be substituted with a phenyl group, a pyrrolyl group, a pyrimidyl group, or an indolyl group, or $R^2$ and $R^3$ may be bonded to each other to form a 5- to 7-membered nonaromatic heterocycle in which one or two hydrogen atoms of the ring may be substituted with a hydrocarbon group having 1 to 4 carbon atoms, an amide group, or a nitrogen-containing cyclic group, and one carbon atom of the ring may be a carbon atom constituting other nitrogen-containing cyclic group; wherein, in formula (A), $R^1$ is an alkyl group having 1 to 22 carbon atoms or an alkenyl group having 2 to 22 carbon atoms; wherein, in formula (I'), $R^1$ is the same as defined above, and three $R^1$ groups in one molecule may be the same as or different from each other.

[9] A lipid nanoparticle comprising the pH-sensitive cationic lipid according to any one of [1] to [6].

[10] The lipid nanoparticle according to [9], further comprising a sterol and a polyalkylene glycol-modified lipid.

[11] The lipid nanoparticle according to [9] or [10], further comprising phosphatidylcholine.

[12] The lipid nanoparticle according to any one of [9] to [11], containing a nucleic acid.

[13] The lipid nanoparticle according to [12], wherein the nucleic acid is siRNA, mRNA, or plasmid DNA.

[14] A pharmaceutical composition comprising the lipid nanoparticle containing the pH-sensitive cationic lipid according to any one of [1] to [6] as an active ingredient.

[15] A pharmaceutical composition comprising, as an active ingredient, the lipid nanoparticle containing the pH-sensitive cationic lipid according to any one of [1] to [6] and a nucleic acid.

[16] The pharmaceutical composition according to [14] or [15], which is used for gene therapy.

[17] A method for expressing a foreign gene, comprising administering to a subject animal (excluding human), the lipid nanoparticle according to [12], which encapsulates a foreign gene of interest to be expressed in a target cell, and expressing the foreign gene in the target cell of the subject animal.

Effects of Invention

The pH-sensitive cationic lipid according to the present invention is relatively easy to synthesize and is suitable for large-scale synthesis. Therefore, the pH-sensitive cationic lipid is useful as a constituent lipid of lipid nanoparticles used as an active ingredient of a pharmaceutical.

Brief Description of Drawings

Fig. 1 illustrates a plot diagram plotting Rf values of each lipid against apparent pKa values in Test Example 1. Fig. 1(A) shows the results of TLC using dichloromethane:methanol (9:1) as a mobile phase, and Fig. 1(B) shows the results of TLC using dichloromethane:methanol (19:1) as a mobile phase.

Fig. 2 illustrates a diagram showing the results of measurement of Fluc activity (RLU/mg protein) in the liver and spleen of mice administered with Fluc mRNA-loaded lipid nanoparticles in Example 11.

Fig. 3 illustrates a diagram showing the results of measurement of Fluc activity (RLU/mg protein) in the liver and spleen of mice administered with each Fluc mRNA-loaded lipid nanoparticle in Example 12.

Fig. 4 illustrates a diagram showing the results of measurement of Fluc activity (RLU/mg protein) in the liver and spleen of mice administered with each Fluc mRNA-loaded lipid nanoparticle in Example 13.

Fig. 5 illustrates a diagram showing the results of measurement of Fluc activity (RLU/mg protein) in the liver and spleen of mice administered with each Fluc mRNA-loaded lipid nanoparticle in Example 14.

Fig. 6 illustrates a diagram showing the results of measurement of Fluc activity (RLU/mg protein) in the liver and spleen of mice administered with each Fluc mRNA-loaded lipid nanoparticle in Example 15.

Fig. 7 illustrates a diagram showing the results of measurement of Fluc activity (RLU/mg protein) in the liver and spleen of mice administered with each Fluc mRNA-loaded lipid nanoparticle in Example 16.

Fig. 8 illustrates a diagram showing the results of measurement of Fluc activity (RLU/mg protein) in the liver and spleen of mice administered with each Fluc mRNA-loaded lipid nanoparticle in Example 17.

Fig. 9 illustrates a diagram showing the results of measurement of Fluc activity (RLU/mg protein) in the liver and spleen of mice administered with each Fluc mRNA-loaded lipid nanoparticle in Example 18.

Fig. 10 illustrates a diagram showing the results of measurement of Fluc activity (RLU/mg protein) in each tissue of mice administered with each Fluc mRNA-loaded lipid nanoparticle in Example 19.

Fig. 11 illustrates imaging results of measurement of luciferase-derived luminescence intensity of the whole body of mice administered with each Fluc mRNA-loaded lipid nanoparticle in Example 20.

Fig. 12 illustrates a diagram showing the results of time-course measurement of tumor volume in mice intravenously administered with Ova mRNA-loaded lipid nanoparticles 7 days and 10 days after tumor cell inoculation in Example 21.

Fig. 13 illustrates a diagram showing the measurement results of Fluc activity (RLU/mg protein) in the liver of mice administered with each Fluc mRNA-loaded lipid nanoparticle in Example 33.

Fig. 14 illustrates a diagram showing the measurement results of Fluc activity (RLU/mg protein) in the spleen of mice administered with each Fluc mRNA-loaded lipid nanoparticle in Example 33.

Fig. 15 illustrates a diagram showing the measurement results of Fluc activity (RLU/mg protein) in the lung of mice administered with each Fluc mRNA-loaded lipid nanoparticle in Example 33.

Fig. 16 illustrates a diagram showing the measurement results of Fluc activity (RLU/mg protein) in the brain of mice administered with each Fluc mRNA-loaded lipid nanoparticle in Example 33.

Fig. 17 illustrates a diagram showing the measurement results of Fluc activity (RLU/mg protein) in the heart of mice administered with each Fluc mRNA-loaded lipid nanoparticle in Example 33.

Fig. 18 illustrates a diagram showing the measurement results of Fluc activity (RLU/mg protein) in the muscle of mice administered with each Fluc mRNA-loaded lipid nanoparticle in Example 33.

Fig. 19 illustrates a diagram showing the measurement results of Fluc activity (RLU/mg protein) in the liver and spleen of mice administered with each Fluc mRNA-loaded lipid nanoparticle in Example 34.

Fig. 20 illustrates a diagram plotting specificity on the vertical axis and pKa on the horizontal axis (Fig. 20(A)) and a diagram plotting the relationship between specificity change and pKa (Fig. 20(B)) for Fluc mRNA-loaded lipid nanoparticles having a DSPC content of 3 mol% ("DSPC-3" in the figure) and Fluc mRNA-loaded lipid nanoparticles having a DSPC content of 15 mol% ("DSPC-15" in the figure) in Example 35.

Fig. 21 illustrates a diagram showing the measurement results of Fluc activity (RLU/mg protein) in the liver and spleen of wild-type mice and ApoE-deficient mice administered with Fluc mRNA-loaded lipid nanoparticles having a DSPC content of 3 mol% or 15 mol% in Example 36.

Fig. 22 illustrates a diagram showing, separately for each cell type, the results of measurement of DiD fluorescence intensity in the spleen taken up with DiD-modified Fluc mRNA-loaded lipid nanoparticles having different DSPC contents in Example 37.

Fig. 23 illustrates a diagram showing the measurement results of Fluc activity (RLU/mg protein) in splenic B cells of mice administered with Fluc mRNA-loaded lipid nanoparticles having various DSPC contents in Example 38.

Fig. 24 illustrates a diagram showing the measurement results of DiD fluorescence intensity in primary B cells taken up with DiD-modified Fluc mRNA-loaded lipid nanoparticles having a DSPC content of 3 mol% or 15 mol% under conditions of untreated serum and no EDTA addition, untreated serum and EDTA addition, or inactivated serum and no EDTA addition in Example 39.

Fig. 25 illustrates a diagram showing the results of measurement of DiD fluorescence intensity in B cells, MZB cells, and FOB cells of mice administered with DiD-modified Fluc mRNA-loaded lipid nanoparticles having a DSPC content of 3 mol% or 15 mol% in Example 40.

Description of Embodiments

Hereinafter, embodiments of the present invention will be specifically described. In this specification, "X1 to X2 (X1 and X2 are real numbers satisfying X1 < X2)" means "X1 or more and X2 or less." Also, "$C_{X3\text{-}X4}$ (X3 and X4 are integers satisfying X3 < X4)" means "having X3 or more and X4 or less carbon atoms."

[pH-Sensitive Cationic Lipid]

The pH-sensitive cationic lipid according to the present invention is a compound in which a hydrocarbon chain is bonded to each of the three hydroxyl groups of Tris, and one hydrophilic group is bonded to the amino group of Tris. It is a compound having three hydrocarbon chains and a hydrophilic moiety and has a high affinity for lipids such as phospholipids due to the similarity of its structure, and is suitable as a constituent lipid of lipid nanoparticles.

As an example of the pH-sensitive cationic lipid according to the present invention, a compound can be mentioned in which a fatty acid residue is bonded to each of the three oxygen atoms in the Tris skeleton, and a primary to tertiary amine is linked to one nitrogen atom in the Tris skeleton via a carbonyl group or a carbonyl group followed by an alkylene group having 1 to 6 carbon atoms. One nitrogen atom of the Tris skeleton forms an amide bond with the carbonyl group linked thereto. Here, the term "Tris skeleton" means a structure in which one hydrogen atom is removed from each of the three hydroxyl groups and the amino group of tris(hydroxymethyl)aminomethane. The three fatty acid residues bonded to the Tris skeleton have 1 to 22 carbon atoms in the hydrocarbon chain moiety and may have one or more unsaturated bonds. Further, a plurality of fatty acid residues in one molecule may be the same group or two or more different groups. The pH-sensitive cationic lipid according to the present invention preferably has all of the plurality of fatty acid residues in one molecule being the same group. The one nitrogen atom of the Tris skeleton and the amine are linked via a carbonyl group or via a carbonyl group followed by an alkylene group having 1 to 6 carbon atoms, and the alkylene group linked to the carbonyl group may be substituted with one hydroxyalkyl group having 1 to 3 carbon atoms. As the amine, specifically, the groups listed for X below can be used. The pKa of the compound depends on the structure of the terminal amine and is preferably about 4.0 to about 9.0, preferably about 4.5 to about 8.5, more preferably about 5.0 to about 8.5.

An example of the pH-sensitive cationic lipid according to the present invention is a compound represented by the following general formula (I):

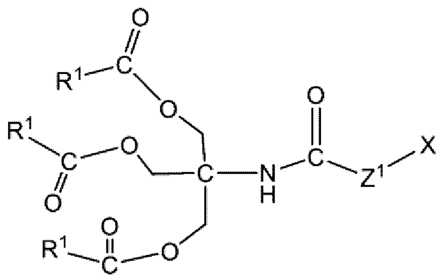

(I)

In the general formula (I), $R^1$ is a hydrocarbon group having 1 to 22 carbon atoms ($C_{1-22}$ hydrocarbon group). The $C_{1-22}$ hydrocarbon group may be linear or branched. The hydrocarbon group may be an alkyl group or an alkenyl group. The alkenyl group may be a group having one unsaturated bond, two unsaturated bonds, or three unsaturated bonds.

When $R^1$ is a $C_{1-22}$ alkyl group, examples of the alkyl group include:

methyl group, ethyl group, n-propyl group, isopropyl group, n-butyl group, isobutyl group, sec-butyl group, tert-butyl group;

n-pentyl group, 1-methylbutyl group, 2-methylbutyl group, 3-methylbutyl group, 1-ethylpropyl group, 1,1-dimethylpropyl group, 2,2-dimethylpropyl group;

n-hexyl group, 1-methylpentyl group, 2-methylpentyl group, 3-methylpentyl group, 4-methylpentyl group, 1-ethylbutyl group, 1,1-dimethylbutyl group, 2,2-dimethylbutyl group, 3,3-dimethylbutyl group, 1,2-dimethylbutyl group, 1-methyl-2,2-dimethylbutyl group;

n-heptyl group, 1-methylhexyl group, 2-methylhexyl group, 3-methylhexyl group, 4-methylhexyl group, 5-methylhexyl group, 1-ethylpentyl group, 1,1-dimethylpentyl group, 2,2-dimethylpentyl group, 3,3-dimethylpentyl group, 4,4-dimethylpentyl group, 1-methyl-3,3-dimethylbutyl group, 2-methyl-3,3-dimethylbutyl group;

n-octyl group, 1-methylheptyl group, 2-methylheptyl group, 3-methylheptyl group, 4-methylheptyl group, 5-methylheptyl group, 6-methylheptyl group, 1-ethylhexyl group, 1,1-dimethylhexyl group, 2,2-dimethylhexyl group, 3,3-dimethylhexyl group, 4,4-dimethylhexyl group, 5,5-dimethylhexyl group, 1-methyl-4,4-dimethylpentyl group, 2-methyl-4,4-dimethylpentyl group, 3-methyl-4,4-dimethylpentyl group;

n-nonyl group, 1-methyloctyl group, 2-methyloctyl group, 3-methyloctyl group, 4-methyloctyl group, 5-methyloctyl group, 6-methyloctyl group, 7-methyloctyl group, 1-ethylheptyl group, 1,1-dimethylheptyl group, 2,2-dimethylheptyl group, 3,3-dimethylheptyl group, 4,4-dimethylheptyl group, 5,5-dimethylheptyl group, 6,6-dimethylheptyl group, 1-methyl-5,5-dimethylhexyl group, 2-methyl-5,5-dimethylhexyl group, 3-methyl-5,5-dimethylhexyl group, 4-methyl-5,5-dimethylhexyl group;

n-decyl group, 1-methylnonyl group, 2-methylnonyl group, 3-methylnonyl group, 4-methylnonyl group, 5-methylnonyl group, 6-methylnonyl group, 7-methylnonyl group, 8-methylnonyl group, 1-ethyloctyl group, 1,1-dimethyloctyl group, 2,2-dimethyloctyl group, 3,3-dimethyloctyl group, 4,4-dimethyloctyl group, 5,5-dimethyloctyl group, 6,6-dimethyloctyl group, 7,7-dimethyloctyl group, 1-methyl-6,6-dimethylheptyl group, 2-methyl-6,6-dimethylheptyl group, 3-methyl-6,6-dimethylheptyl group, 4-methyl-6,6-dimethylheptyl group, 5-methyl-6,6-dimethylheptyl group;

n-undecyl group, 1-methyldecyl group, 2-methyldecyl group, 3-methyldecyl group, 4-methyldecyl group, 5-methyldecyl group, 6-methyldecyl group, 7-methyldecyl group, 8-methyldecyl group, 9-methyldecyl group, 1-ethylnonyl group, 1,1-dimethylnonyl group, 2,2-dimethylnonyl group, 3,3-dimethylnonyl group, 4,4-dimethylnonyl group, 5,5-dimethylnonyl group, 6,6-dimethylnonyl group, 7,7-dimethylnonyl group, 8,8-dimethylnonyl group, 1-methyl-7,7-dimethyloctyl group, 2-methyl-7,7-dimethyloctyl group, 3-methyl-7,7-dimethyloctyl group, 4-methyl-7,7-dimethyloctyl group, 5-methyl-7,7-dimethyloctyl group, 6-methyl-7,7-dimethyloctyl group;

n-dodecyl group, 1-methylundecyl group, 2-methylundecyl group, 3-methylundecyl group, 4-methylundecyl group, 5-methylundecyl group, 6-methylundecyl group, 7-methylundecyl group, 8-methylundecyl group, 9-methylundecyl group, 10-methylundecyl group, 1-ethyldecyl group, 1,1-dimethyldecyl group, 2,2-dimethyldecyl group, 3,3-dimethyldecyl group, 4,4-dimethyldecyl group, 5,5-dimethyldecyl group, 6,6-dimethyldecyl group, 7,7-dimethyldecyl group, 8,8-dimethyldecyl group, 9,9-dimethyldecyl group, 1-methyl-8,8-dimethylnonyl group, 2-methyl-8,8-dimethylnonyl group, 3-methyl-8,8-dimethylnonyl group, 4-methyl-8,8-dimethylnonyl group, 5-methyl-8,8-dimethylnonyl group, 6-methyl-8,8-dimethylnonyl group, 7-methyl-8,8-dimethylnonyl group;

n-tridecyl group, 1-methyldodecyl group, 2-methyldodecyl group, 3-methyldodecyl group, 4-methyldodecyl group, 5-methyldodecyl group, 6-methyldodecyl group, 7-methyldodecyl group, 8-methyldodecyl group, 9-methyldodecyl group, 10-methyldodecyl group, 11-methyldodecyl group, 1-ethylundecyl group, 1,1-dimethylundecyl group, 2,2-dimethylundecyl group, 3,3-dimethylundecyl group, 4,4-dimethylundecyl group, 5,5-dimethylundecyl group, 6,6-dimethylundecyl group, 7,7-dimethylundecyl group, 8,8-dimethylundecyl group, 9,9-dimethylundecyl group, 10,10-dimethylundecyl group, 1-methyl-9,9-dimethyldecyl group, 2-methyl-9,9-dimethyldecyl group, 3-methyl-9,9-dimethyldecyl group, 4-methyl-9,9-dimethyldecyl group, 5-methyl-9,9-dimethyldecyl group, 6-methyl-9,9-dimethyldecyl group, 7-methyl-9,9-dimethyldecyl group, 8-methyl-9,9-dimethyldecyl group;
n-tetradecyl group, 1-methyltridecyl group, 2-methyltridecyl group, 3-methyltridecyl group, 4-methyltridecyl group, 5-methyltridecyl group, 6-methyltridecyl group, 7-methyltridecyl group, 8-methyltridecyl group, 9-methyltridecyl group, 10-methyltridecyl group, 11-methyltridecyl group, 12-methyltridecyl group, 1-ethyldodecyl group, 1,1-dimethyldodecyl group, 2,2-dimethyldodecyl group, 3,3-dimethyldodecyl group, 4,4-dimethyldodecyl group, 5,5-dimethyldodecyl group, 6,6-dimethyldodecyl group, 7,7-dimethyldodecyl group, 8,8-dimethyldodecyl group, 9,9-dimethyldodecyl group, 10,10-dimethyldodecyl group, 11,11-dimethyldodecyl group, 1-methyl-10,10-dimethylundecyl group, 2-methyl-10,10-dimethylundecyl group, 3-methyl-10,10-dimethylundecyl group, 4-methyl-10,10-dimethylundecyl group, 5-methyl-10,10-dimethylundecyl group, 6-methyl-10,10-dimethylundecyl group, 7-methyl-10,10-dimethylundecyl group, 8-methyl-10,10-dimethylundecyl group, 9-methyl-10,10-dimethylundecyl group;
n-pentadecyl group, 1-methyltetradecyl group, 2-methyltetradecyl group, 3-methyltetradecyl group, 4-methyltetradecyl group, 5-methyltetradecyl group, 6-methyltetradecyl group, 7-methyltetradecyl group, 8-methyltetradecyl group, 9-methyltetradecyl group, 10-methyltetradecyl group, 11-methyltetradecyl group, 12-methyltetradecyl group, 13-methyltetradecyl group, 1-ethyltridecyl group, 1,1-dimethyltridecyl group, 2,2-dimethyltridecyl group, 3,3-dimethyltridecyl group, 4,4-dimethyltridecyl group, 5,5-dimethyltridecyl group, 6,6-dimethyltridecyl group, 7,7-dimethyltridecyl group, 8,8-dimethyltridecyl group, 9,9-dimethyltridecyl group, 10,10-dimethyltridecyl group, 11,11-dimethyltridecyl group, 12,12-dimethyltridecyl group, 1-methyl-11,11-dimethyldodecyl group, 2-methyl-11,11-dimethyldodecyl group, 3-methyl-11,11-dimethyldodecyl group, 4-methyl-11,11-dimethyldodecyl group, 5-methyl-11,11-dimethyldodecyl group, 6-methyl-11,11-dimethyldodecyl group, 7-methyl-11,11-dimethyldodecyl group, 8-methyl-11,11-dimethyldodecyl group, 9-methyl-11,11-dimethyldodecyl group, 10-methyl-11,11-dimethyldodecyl group;
n-hexadecyl group, 1-methylpentadecyl group, 2-methylpentadecyl group, 3-methylpentadecyl group, 4-methylpentadecyl group, 5-methylpentadecyl group, 6-methylpentadecyl group, 7-methylpentadecyl group, 8-methylpentadecyl group, 9-methylpentadecyl group, 10-methylpentadecyl group, 11-methylpentadecyl group, 12-methylpentadecyl group, 13-methylpentadecyl group, 14-methylpentadecyl group;
n-heptadecyl group, 1-methylhexadecyl group, 2-methylhexadecyl group, 3-methylhexadecyl group, 4-methylhexadecyl group, 5-methylhexadecyl group, 6-methylhexadecyl group, 7-methylhexadecyl group, 8-methylhexadecyl group, 9-methylhexadecyl group, 10-methylhexadecyl group, 11-methylhexadecyl group, 12-methylhexadecyl group, 13-methylhexadecyl group, 14-methylhexadecyl group, 15-methylhexadecyl group;
n-octadecyl group, 1-methylheptadecyl group, 2-methylheptadecyl group, 3-methylheptadecyl group, 4-methylheptadecyl group, 5-methylheptadecyl group, 6-methylheptadecyl group, 7-methylheptadecyl group, 8-methylheptadecyl group, 9-methylheptadecyl group, 10-methylheptadecyl group, 11-methylheptadecyl group, 12-methylheptadecyl group, 13-methylheptadecyl group, 14-methylheptadecyl group, 15-methylheptadecyl group, 16-methylheptadecyl group;
n-nonadecyl group, 1-methyloctadecyl group, 2-methyloctadecyl group, 3-methyloctadecyl group, 4-methyloctadecyl group, 5-methyloctadecyl group, 6-methyloctadecyl group, 7-methyloctadecyl group, 8-methyloctadecyl group, 9-methyloctadecyl group, 10-methyloctadecyl group, 11-methyloctadecyl group, 12-methyloctadecyl group, 13-methyloctadecyl group, 14-methyloctadecyl group, 15-methyloctadecyl group, 16-methyloctadecyl group, 17-methyloctadecyl group;
n-icosyl group, 1-methylnonadecyl group, 2-methylnonadecyl group, 3-methylnonadecyl group, 4-methylnonadecyl group, 5-methylnonadecyl group, 6-methylnonadecyl group, 7-methylnonadecyl group, 8-methylnonadecyl group, 9-methylnonadecyl group, 10-methylnonadecyl group, 11-methylnonadecyl group, 12-methylnonadecyl group, 13-methylnonadecyl group, 14-methylnonadecyl group, 15-methylnonadecyl group, 16-methylnonadecyl group, 17-methylnonadecyl group, 18-methylnonadecyl group;
n-henicosyl group, 1-methylicosyl group, 2-methylicosyl group, 3-methylicosyl group, 4-methylicosyl group, 5-methylicosyl group, 6-methylicosyl group, 7-methylicosyl group, 8-methylicosyl group, 9-methylicosyl group, 10-methylicosyl group, 11-methylicosyl group, 12-methylicosyl group, 13-methylicosyl group, 14-methylicosyl group, 15-methylicosyl group, 16-methylicosyl group, 17-methylicosyl group, 18-methylicosyl group, 19-methylicosyl group;
n-docosyl group, 1-methylhenicosyl group, 2-methylhenicosyl group, 3-methylhenicosyl group, 4-methylhenicosyl group, 5-methylhenicosyl group, 6-methylhenicosyl group, 7-methylhenicosyl group, 8-methylhenicosyl group, 9-methylhenicosyl group, 10-methylhenicosyl group, 11-methylhenicosyl group, 12-methylhenicosyl group, 13-methylhenicosyl group, 14-methylhenicosyl group, 15-methylhenicosyl group, 16-methylhenicosyl group, 17-methylhenicosyl group, 18-methylhenicosyl group, 19-methylhenicosyl group, 20-methylhenicosyl group; and the like.

When $R^1$ is a $C_{2-22}$ alkenyl group, examples of the alkenyl group include groups in which 1 to 3 of the saturated bonds between carbon molecules in the groups listed as the $C_{2-22}$ alkyl group are replaced by unsaturated bonds.
In the general formula (I), three $R^1$ groups in one molecule may be the same as or different from each other. In the

pH-sensitive cationic lipid according to the present invention, from the viewpoint of ease of synthesis and quality stability, it is preferable that all three $R^1$ groups in one molecule are the same.

In the pH-sensitive cationic lipid according to the present invention, $R^1$ is preferably a linear $C_{1-22}$ alkyl group or a linear $C_{2-22}$ alkenyl group, and more preferably a linear $C_{15-22}$ alkyl group or a linear $C_{15-22}$ alkenyl group. In the pH-sensitive cationic lipid according to the present invention, it is more preferable that the three $R^1$ groups in one molecule are each the same group or different group selected from the group consisting of n-pentadecyl group, n-hexadecyl group, n-heptadecyl group, n-octadecyl group, n-nonadecyl group, n-icosyl group, n-henicosyl group, n-docosyl group, n-pentadecenyl group, n-hexadecenyl group, n-heptadecenyl group, n-octadecenyl group, n-nonadecenyl group, n-icosenyl group, n-henicosenyl group, and n-docosenyl group, and it is still more preferable that all three $R^1$ groups in one molecule are each the same group selected from the group consisting of n-pentadecyl group, n-hexadecyl group, n-heptadecyl group, n-octadecyl group, n-nonadecyl group, n-icosyl group, n-henicosyl group, n-docosyl group, n-pentadecenyl group, n-hexadecenyl group, n-heptadecenyl group, n-octadecenyl group, n-nonadecenyl group, n-icosenyl group, n-henicosenyl group, and n-docosenyl group.

In the general formula (I), $Z^1$ is a $C_{1-6}$ alkylene group which may be substituted with one hydroxyl group, or a single bond. The $C_{1-6}$ alkylene group may be linear or branched. Examples of the $C_{1-6}$ alkylene group include a methylene group, an ethylene group, a 1-methylmethylene group, a 1-hydroxymethylmethylene group, an n-propylene group, a 1-methylethylene group, a 1-hydroxymethylethylene group, a 2-methylethylene group, an n-butylene group, a 1-methylpropylene group, a 1-hydroxymethylpropylene group, a 2-methylpropylene group, a 3-methylpropylene group, a 1-ethylethylene group, a 2-ethylethylene group, an n-pentylene group, a 1-methylbutylene group, a 2-methylbutylene group, a 3-methylbutylene group, a 4-methylbutylene group, a 1-ethylpropylene group, a 2-ethylpropylene group, a 3-ethylpropylene group, an n-hexylene group, a 1-methylpentylene group, a 2-methylpentylene group, a 3-methylpentylene group, a 4-methylpentylene group, a 1-ethylbutylene group, a 2-ethylbutylene group, and a 3-ethylbutylene group. In the pH-sensitive cationic lipid according to the present invention, a compound in which $Z^1$ is a methylene group, an ethylene group, an n-propylene group, a 1-hydroxymethylmethylene group, a 1-methylmethylene group, a 1-methylethylene group, a 1-hydroxymethylethylene group, or a 2-methylethylene group is preferable, and a compound in which $Z^1$ is a methylene group, an ethylene group, an n-propylene group, or a 1-hydroxymethylmethylene group is more preferable.

In the general formula (I), X is $-N(R^2)(R^3)$ or a 5- to 7-membered nonaromatic heterocyclic group. The 5- to 7-membered nonaromatic heterocyclic group represented by X is bonded to $Z^1$ via a carbon atom.

In the general formula (I), when X is $-N(R^2)(R^3)$, $R^2$ and $R^3$ are each independently a hydrogen atom or a $C_{1-4}$ hydrocarbon group in which one or two hydrogen atoms may be substituted with a phenyl group, a pyrrolyl group, a pyrimidyl group, or an indolyl group. The $C_{1-4}$ hydrocarbon group may be a linear or branched $C_{1-4}$ alkyl group or a linear or branched $C_{2-4}$ alkenyl group.

When $R^2$ and $R^3$ are a $C_{1-4}$ alkyl group, examples of the $C_{1-4}$ alkyl group include a methyl group, an ethyl group, a propyl group, an isopropyl group, an n-butyl group, an isobutyl group, and a tert-butyl group. When $R^2$ and $R^3$ are a $C_{2-4}$ alkenyl group, examples of the $C_{2-4}$ alkenyl group include a vinyl group, a 1-propenyl group, a 2-propenyl group, a 1-methylvinyl group, a 2-methyl-1-propenyl group, a 1-butenyl group, a 2-butenyl group, and a 3-butenyl group. In the pH-sensitive cationic lipid according to the present invention, in the general formula (I), when X is $-N(R^2)(R^3)$, a compound in which $R^2$ and $R^3$ are each independently a hydrogen atom or a linear or branched $C_{1-4}$ alkyl group is preferable, a compound in which $R^2$ and $R^3$ are each independently a hydrogen atom or a linear $C_{1-4}$ alkyl group is more preferable, and a compound in which $R^2$ and $R^3$ are each independently a hydrogen atom, a methyl group, an ethyl group, or an n-propyl group is still more preferable.

In the general formula (I), when X is $-N(R^2)(R^3)$, $R^2$ and $R^3$ may be bonded to each other to form a 5- to 7-membered nonaromatic heterocycle. Examples of the 5- to 7-membered nonaromatic heterocycle formed by $R^2$ and $R^3$ being bonded to each other include a 1-pyrrolidinyl group, a 1-piperidinyl group, a 1-morpholinyl group, and a 1-piperazinyl group.

When X is $-N(R^2)(R^3)$ and $R^2$ and $R^3$ are bonded to each other to form a 5- to 7-membered nonaromatic heterocycle, one or two hydrogen atoms in the formed 5- to 7-membered nonaromatic heterocycle may be substituted with a $C_{1-4}$ hydrocarbon group, an amide group, or a nitrogen-containing cyclic group. The $C_{1-4}$ hydrocarbon group is preferably a $C_{1-4}$ alkyl group or a $C_{2-4}$ alkenyl group. When two hydrogen atoms in the ring are substituted with a $C_{1-4}$ alkyl group or a $C_{2-4}$ alkenyl group, they may be substituted with the same group or with different groups. The nitrogen-containing cyclic group is preferably a group derived from a ring in which a carbonyl group is adjacent to a nitrogen atom, and examples thereof include a group derived from a nitrogen-containing ring such as benzo[d]oxazol-2(3H)-one, 1,3-dihydro-2H-benzo[d]imidazol-2-one, indolin-2-one, 1,3-dihydro-2H-pyrrol-2-one, 1,3-dihydro-2H-imidazol-2-one, oxazol-2(3H)-one, oxazolidin-2-one, imidazolidin-2-one, pyrrolidin-2-one, 1,4-dihydroisoquinolin-3(2H)-one, 3,4-dihydro-2H-benzo[e][1,3]oxazin-2-one, 3,4-dihydroquinazolin-2(1H)-one, 3,4-dihydroquinolin-2(1H)-one, 1,4-dihydro-2H-benzo[d][1,3]oxazin-2-one, 3,4-dihydropyridin-2(1H)-one, 3,6-dihydropyridin-2(1H)-one, 3,6-dihydro-2H-1,3-oxazin-2-one, 3,4-dihydro-2H-1,3-oxazin-2-one, 3,4-dihydropyrimidin-2(1H)-one, piperidin-2-one, 1,3-oxazinan-2-one, or tetrahydropyrimidin-2(1H)-one.

When X is $-N(R^2)(R^3)$ and $R^2$ and $R^3$ are bonded to each other to form a 5- to 7-membered nonaromatic heterocycle, one of the carbon atoms constituting the 5- to 7-membered nonaromatic heterocycle may be a carbon atom constituting other nitrogen-containing cyclic group. The other nitrogen-containing cyclic group is preferably a group derived from a ring in which a carbonyl group is adjacent to a nitrogen atom, and specific examples thereof include the same groups as described above.

In the general formula (I), when X is a 5- to 7-membered nonaromatic heterocyclic group, examples of heteroatoms contained in the heterocyclic group include a nitrogen atom, an oxygen atom, and a sulfur atom. The heterocyclic group may have one heteroatom constituting the heterocycle, or two or more heteroatoms which may be the same or different. The heterocycle in the heterocyclic group may be a saturated heterocycle or may contain one or two or more double bonds, but the heterocycle is not an aromatic ring.

When X is a 5- to 7-membered nonaromatic heterocyclic group, the heterocyclic group is bonded to $Z^1$ via a carbon atom, and the ring may be substituted with one or two $C_{1-4}$ hydrocarbon groups or amide groups. The $C_{1-4}$ hydrocarbon group may be a linear or branched $C_{1-4}$ alkyl group or a linear or branched $C_{2-4}$ alkenyl group. As the linear or branched $C_{1-4}$ alkyl group and the linear or branched $C_{2-4}$ alkenyl group, those mentioned above can be used.

In the general formula (I), $-Z^1$-X is preferably any one group of the following formulas (H-1) to (H-8), (H-16), and (H-17). $-Z^1$-X in the general formula (I) is also preferably any one group of the following formulas (H-18) to (H-26). In the following formulas, black circles represent bonding arms.

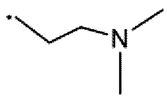
(H-1)

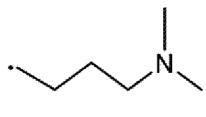
(H-2)

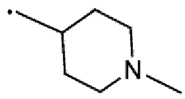
(H-3)

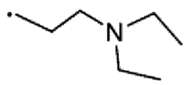
(H-4)

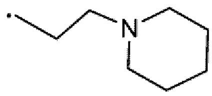
(H-5)

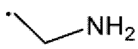
(H-6)

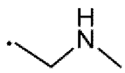
(H-7)

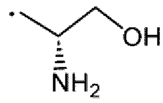
(H-8)

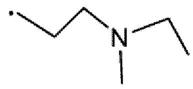
(H-16)

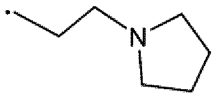
(H-17)

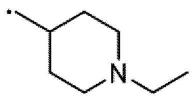
(H-18)

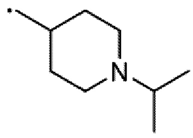
(H-19)

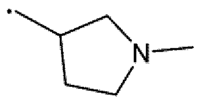
(H-20)

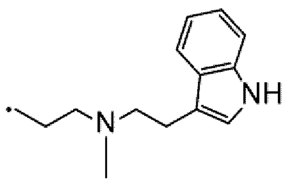
(H-21)

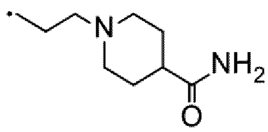
(H-22)

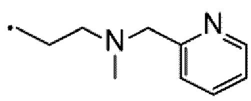
(H-23)

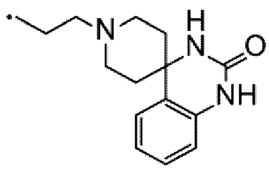
(H-24)

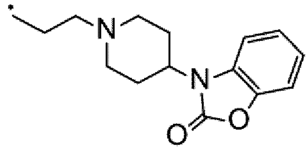
(H-25)

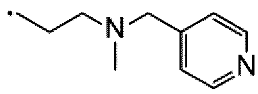
(H-26)

The pH-sensitive cationic lipid according to the present invention is preferably a compound in which $-Z^1$-X in the general formula (I) is any one group of the formulas (H-1) to (H-8), (H-16), and (H-17), more preferably a compound in

which $R^1$ in the general formula (I) is a linear $C_{1-22}$ alkyl group or a linear $C_{2-22}$ alkenyl group and $-Z^1$-X is any one group of the formulas (H-1) to (H-8), (H-16), and (H-17), further preferably a compound in which $R^1$ in the general formula (I) is a linear $C_{15-22}$ alkyl group or a linear $C_{15-22}$ alkenyl group and $-Z^1$-X is any one group of the formulas (H-1) to (H-8), (H-16), and (H-17), and still more preferably a compound in which $R^1$ in the general formula (I) is a linear $C_{15-22}$ alkyl group or a linear $C_{15-22}$ alkenyl group, all three $R^1$ groups in one molecule are the same, and $-Z^1$-X is any one group of the formulas (H-1) to (H-8), (H-16), and (H-17). Among them, the pH-sensitive cationic lipid according to the present invention is particularly preferably a compound in which all three $R^1$ groups in one molecule in the general formula (I) are any one group of an n-pentadecyl group, an n-hexadecyl group, an n-heptadecyl group, an n-octadecyl group, an n-nonadecyl group, an n-icosyl group, an n-henicosyl group, an n-docosyl group, an n-pentadecenyl group, an n-hexadecenyl group, an n-heptadecenyl group, an n-octadecenyl group, an n-nonadecenyl group, an n-icosenyl group, an n-henicosenyl group, and an n-docosenyl group, and $-Z^1$-X is any one group of the formulas (H-1) to (H-8), (H-16), and (H-17).

The pH-sensitive cationic lipid according to the present invention is preferably a compound in which $-Z^1$-X in the general formula (I) is any one group of the formulas (H-18) to (H-26), more preferably a compound in which $R^1$ in the general formula (I) is a linear $C_{1-22}$ alkyl group or a linear $C_{2-22}$ alkenyl group and $-Z^1$-X is any one group of the formulas (H-18) to (H-26), further preferably a compound in which $R^1$ in the general formula (I) is a linear $C_{15-22}$ alkyl group or a linear $C_{15-22}$ alkenyl group and $-Z^1$-X is any one group of the formulas (H-18) to (H-26), and still more preferably a compound in which $R^1$ in the general formula (I) is a linear $C_{15-22}$ alkyl group or a linear $C_{15-22}$ alkenyl group, all three $R^1$ groups in one molecule are the same, and $-Z^1$-X is any one group of the formulas (H-18) to (H-26). Among them, the pH-sensitive cationic lipid according to the present invention is particularly preferably a compound in which all three $R^1$ groups in one molecule in the general formula (I) are any one group of an n-pentadecyl group, an n-hexadecyl group, an n-heptadecyl group, an n-octadecyl group, an n-nonadecyl group, an n-icosyl group, an n-henicosyl group, an n-docosyl group, an n-pentadecenyl group, an n-hexadecenyl group, an n-heptadecenyl group, an n-octadecenyl group, an n-nonadecenyl group, an n-icosenyl group, an n-henicosenyl group, and an n-docosenyl group, and $-Z^1$-X is any one group of the formulas (H-18) to (H-26).

Examples of the pH-sensitive cationic lipid represented by the general formula (I) include compounds represented by the following general formulas (I-1) to (I-8), (I-16), and (I-17). In the formulas, $R^1$ is the same as in the general formula (I). The apparent pKa of each compound is also shown. In the pH-sensitive cationic lipid represented by the general formula (I), X is a hydrophilic moiety, and the pKa of the pH-sensitive cationic lipid is affected by the structure of this moiety.

apparent pKa

(I-1) 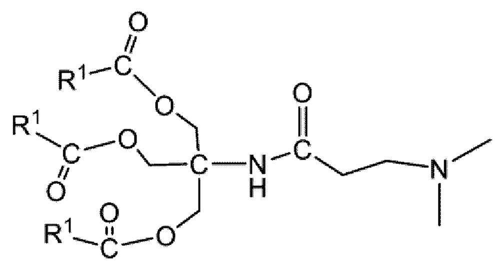 7.10

(I-2) 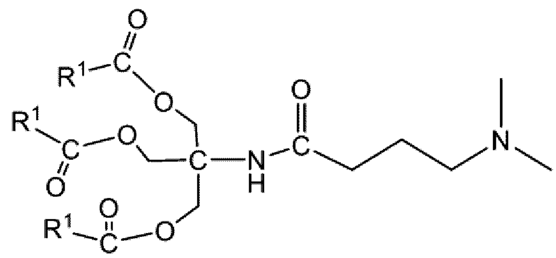 8.15

(I-3) 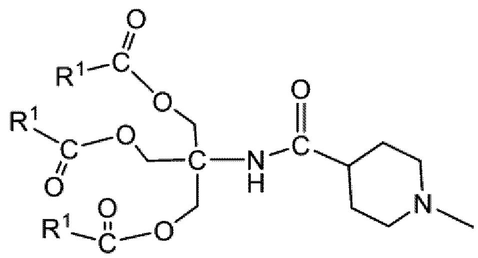 8.05

(I-4) 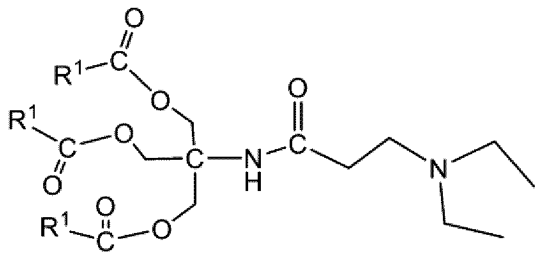 6.50

(I-5) 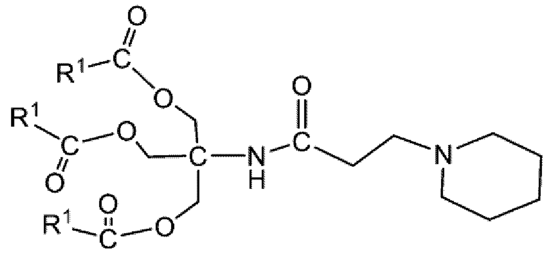 6.25

apparent pKa

(I-6) 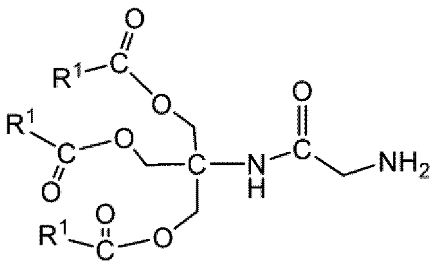 7.35

(I-7) 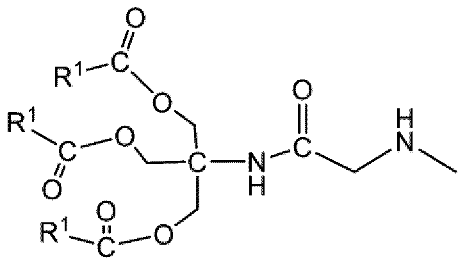 7.30

(I-8) 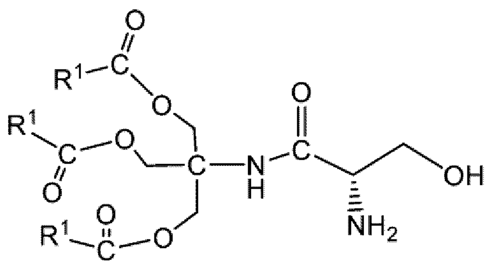 6.75

(I-16) 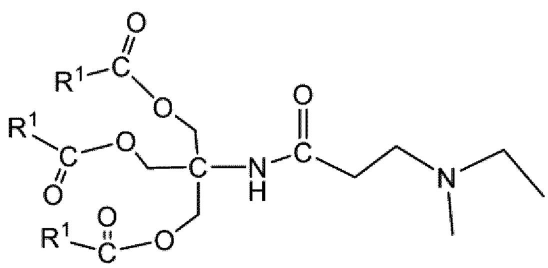 6.60

(I-17) 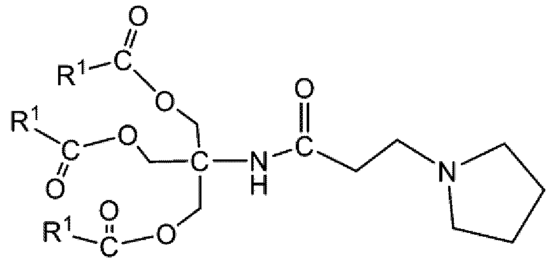 6.20

Examples of the pH-sensitive cationic lipid represented by the general formula (I) include compounds represented by the following general formulas (I-18) to (I-26). In the formulas, $R^1$ is the same as in the general formula (I).

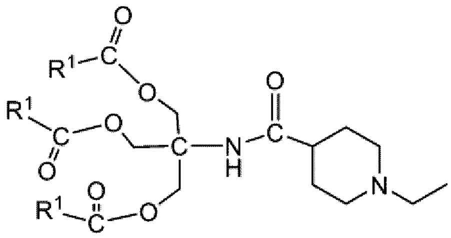

(I-18)

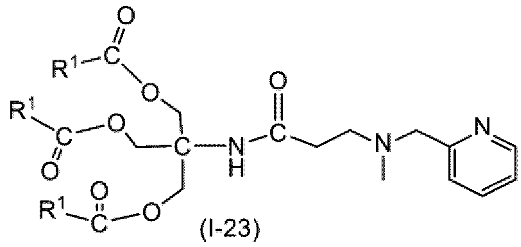

(I-23)

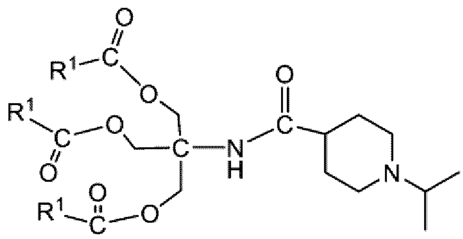

(I-19)

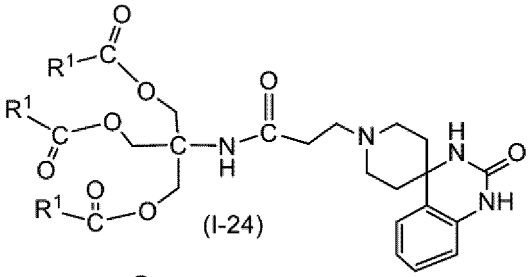

(I-24)

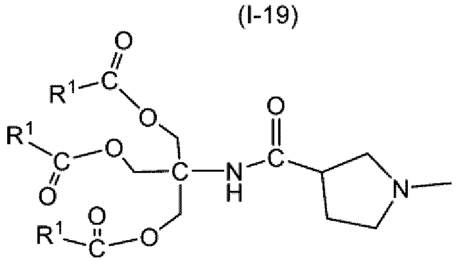

(I-20)

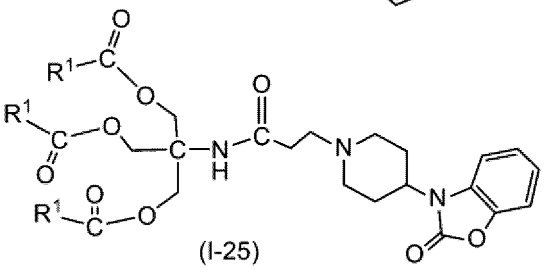

(I-25)

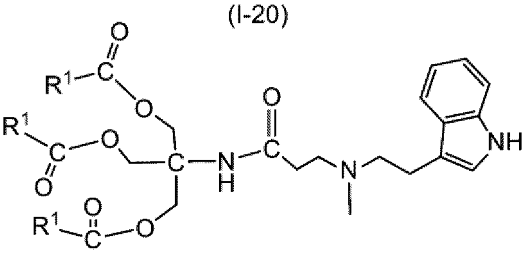

(I-21)

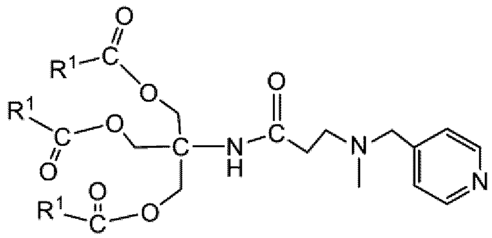

(I-26)

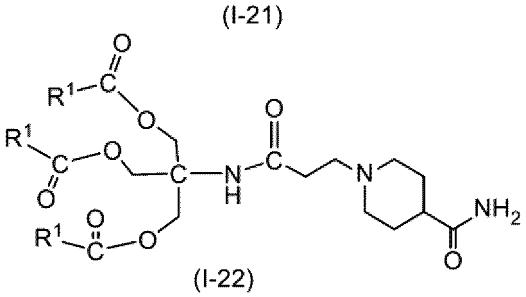

(I-22)

In the pH-sensitive cationic lipid according to the present invention, three hydrocarbon chains composed of $R^1$ extend from one sp3 carbon, and a hydrophilic group composed of X is further linked to this sp3 carbon via $Z^1$. Therefore,

the pH-sensitive cationic lipid according to the present invention can take an orientation in which the hydrophilic group composed of X is sterically shielded by the three hydrocarbon chains composed of $R^1$. On the other hand, the pH-sensitive cationic lipid described in Patent Literature 1 such as CL4H6 has two hydrophobic chains and a hydrophilic group, and the hydrophilic group and the hydrophobic chains are in a positional relationship such that they are separated from each other. Because of these differences in steric structure, the pH-sensitive cationic lipid according to the present invention can be expected to have different properties from the conventional pH-sensitive cationic lipid such as CL4H6 when used as a constituent lipid of a lipid nanoparticle.

The pKa of the pH-sensitive cationic lipid represented by the general formula (I) is not particularly limited, but can be selected, for example, in the range of about 4.0 to about 9.0, preferably about 4.5 to about 8.5, more preferably about 5.0 to about 8.5, and it is preferable to select the type of each substituent so as to give a pKa within this range. The pKa of the pH-sensitive cationic lipid is particularly affected by $-Z^1-X$ in the general formula (I). For example, a pH-sensitive cationic lipid having a desired pKa can be obtained by setting $-Z^1-X$ in the general formula (I) to a group having a pKa close to the desired pKa from among the formulas (H-1) to (H-8) and (H-16) to (H-26).

The pH-sensitive cationic lipid according to the present invention can be produced, for example, by condensing a compound represented by the following general formula (A) (hereinafter sometimes referred to as "compound (A)"), a compound represented by the following general formula (B) (hereinafter sometimes referred to as "compound (B)"), and tris(hydroxymethyl)aminomethane. $R^1$ in the general formula (A) is the same as $R^1$ in the general formula (I). $Z^1$ and X in the general formula (B) are the same as $Z^1$ and X in the general formula (I), respectively.

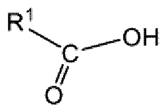
(A)

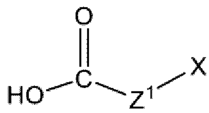
(B)

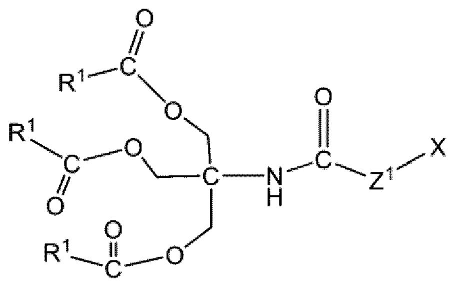
(I)

The pH-sensitive cationic lipid represented by the general formula (I) is synthesized by subjecting the carboxylic acid group of compound (A) and the hydroxyl group of Tris to dehydration condensation to form an ester bond, and further subjecting the carboxylic acid group of compound (B) and the amino group of Tris to dehydration condensation to form an amide bond. The dehydration condensation reaction of the carboxylic acid group of compound (A) with the hydroxyl group of Tris can be generally carried out under the same reaction conditions as the dehydration condensation reaction in forming an ester of a carboxylic acid and an alcohol. The dehydration condensation reaction of the carboxylic acid group of compound (B) with the amino group of Tris can be generally carried out under the same reaction conditions as the dehydration condensation reaction in forming an amide of a carboxylic acid and an amine. That is, the pH-sensitive cationic lipid represented by the general formula (I) can be relatively easily synthesized by a general dehydration condensation reaction using Tris as a linker.

The pH-sensitive cationic lipid represented by the general formula (I) can be synthesized by reacting Tris with compound (A) to carry out a dehydration condensation reaction with the hydroxyl group of Tris, and then reacting the resulting ester with compound (B) to carry out a dehydration condensation reaction with the Tris-derived amino group. The pH-sensitive cationic lipid represented by the general formula (I) can also be synthesized by reacting Tris with compound (B) to carry out a dehydration condensation reaction with the amino group of Tris, and then reacting the resulting amide with compound (A) to carry out a dehydration condensation reaction with the Tris-derived hydroxyl group.

Among the compounds represented by the general formula (I), a compound in which $Z^1$ is an ethylene group and X is $-N(R^2)(R^3)$ (a compound represented by the general formula (I')) can be synthesized by a simpler synthesis method using, for example, N-[tris(hydroxymethyl)methyl]acrylamide. Examples of the pH-sensitive cationic lipid represented by the general formula (I') include compounds in which $-Z^1-X$ in the general formula (I) is the formula (H-1), (H-4), (H-5), (H-16), (H-17), (H-21), (H-22), (H-23), (H-24), (H-25), or (H-26). Specifically, it can be produced by adding a secondary amine compound represented by the following general formula (C) (hereinafter sometimes referred to as "compound (C)") to N-[tris(hydroxymethyl)methyl]acrylamide and then condensing a resulting compound represented by the general formula (D) (hereinafter "compound (D)") with compound (A). $R^2$ and $R^3$ in the general formula (I'), the general formula (C), and the general formula (D) are the same as $R^2$ and $R^3$ in X of the general formula (I), respectively.

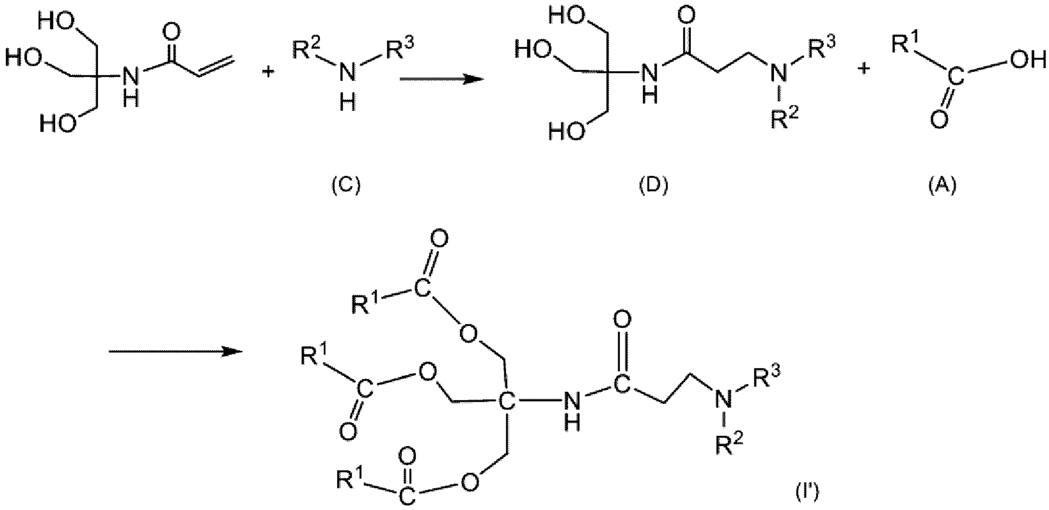

(C) (D) (A)

(I')

The addition reaction of compound (C) to the unsaturated bond in N-[tris(hydroxymethyl)methyl]acrylamide can be generally carried out under the same reaction conditions as the addition reaction in adding a secondary amine to an alkene. The dehydration condensation reaction of the carboxylic acid group of compound (A) with the hydroxyl group of compound (D) can be generally carried out under the same reaction conditions as the dehydration condensation reaction in forming an ester of a carboxylic acid and an alcohol. That is, the pH-sensitive cationic lipid represented by the general formula (I') can be relatively easily synthesized by a general addition reaction and dehydration condensation reaction using N-[tris(hydroxymethyl)methyl]acrylamide.

The pH-sensitive cationic lipid represented by the general formula (I) can be easily produced, for example, by the method specifically shown in the Examples of the present specification. By referring to this production method and appropriately selecting starting compounds, reagents, reaction conditions, and the like, those skilled in the art can easily produce any lipid included in the scope of the general formula (I).

[Lipid Nanoparticle]

The lipid nanoparticle according to the present invention comprises the pH-sensitive cationic lipid according to the present invention as a constituent lipid. The lipid nanoparticle according to the present invention may comprise only one kind or two or more kinds of the pH-sensitive cationic lipid according to the present invention. When the lipid nanoparticle according to the present invention comprises two or more kinds of the pH-sensitive cationic lipid according to the present invention, the amount of the pH-sensitive cationic lipid according to the present invention means the total amount of lipid molecules corresponding to the pH-sensitive cationic lipid according to the present invention among the lipid molecules constituting the lipid nanoparticle.

The proportion of the pH-sensitive cationic lipid according to the present invention in the lipid molecules constituting the lipid nanoparticle is not particularly limited. For example, in the lipid nanoparticle according to the present invention, the ratio of the amount of the pH-sensitive cationic lipid according to the present invention to the total lipid amount constituting the lipid nanoparticle ([amount of pH-sensitive cationic lipid according to the present invention (mol)]/([total amount of lipid constituting the lipid nanoparticle (mol)])$\times$100%) is preferably 5 mol% or more, more preferably 10 mol% or more, and further preferably 20 mol% or more. On the other hand, if the proportion of the pH-sensitive cationic lipid in the lipid molecules constituting the lipid nanoparticle is too high, the content of other lipids for imparting desired properties to the lipid nanoparticle may be too low. Therefore, the ratio of the amount of the pH-sensitive cationic lipid according to the present invention to the total lipid amount constituting the lipid nanoparticle in the lipid nanoparticle according to the present invention is preferably 90 mol% or less, more preferably 80 mol% or less, and further preferably 70 mol% or less.

As the lipid other than the pH-sensitive cationic lipid according to the present invention among the constituent lipids of the lipid nanoparticle according to the present invention, lipids generally used in forming liposomes can be used. Examples of such lipids include phospholipids, sterols, glycolipids, and saturated or unsaturated fatty acids. These can be used alone or in combination of two or more.

Examples of phospholipids include glycerophospholipids such as phosphatidylserine, phosphatidylinositol, phosphatidylglycerol, phosphatidylethanolamine, phosphatidylcholine, cardiolipin, plasmalogen, ceramide phosphorylglycerol phosphate, and phosphatidic acid; and sphingophospholipids such as sphingomyelin, ceramide phosphorylglycerol, and ceramide phosphorylethanolamine. Naturally occurring phospholipids such as egg yolk lecithin and soybean lecithin can also be used. The fatty acid residues in glycerophospholipids and sphingophospholipids are not particularly

limited, but examples thereof include saturated or unsaturated fatty acid residues having 12 to 24 carbon atoms, and saturated or unsaturated fatty acid residues having 14 to 20 carbon atoms are preferable. Specific examples include acyl groups derived from fatty acids such as lauric acid, myristic acid, palmitic acid, palmitoleic acid, stearic acid, oleic acid, linoleic acid, linolenic acid, arachidic acid, arachidonic acid, behenic acid, and lignoceric acid. When these glycerolipids or sphingolipids have two or more fatty acid residues, all the fatty acid residues may be the same group or different groups.

Examples of sterols include animal-derived sterols such as cholesterol, cholesterol hemisuccinate, lanosterol, dihydrolanosterol, desmosterol, and dihydrocholesterol; plant-derived sterols (phytosterols) such as stigmasterol, sitosterol, campesterol, and brassicasterol; and microorganism-derived sterols such as timosterol and ergosterol. Examples of glycolipids include glycosylglycerols such as sulfoxyribosyl glyceride, diglycosyl diglyceride, digalactosyl diglyceride, galactosyl diglyceride, and glycosyl diglyceride; and glycosphingolipids such as galactosylceramide, lactosylceramide, and gangliosides. Examples of saturated or unsaturated fatty acids include saturated or unsaturated fatty acids having 12 to 20 carbon atoms such as palmitic acid, oleic acid, stearic acid, arachidonic acid, and myristic acid.

It is preferable that the constituent lipids of the lipid nanoparticle according to the present invention include a neutral lipid in addition to the pH-sensitive cationic lipid according to the present invention, more preferably a phospholipid or a sterol, further preferably a sterol, and still more preferably cholesterol.

The lipid nanoparticle according to the present invention preferably contains a polyalkylene glycol-modified lipid as a lipid component. Since polyalkylene glycol is a hydrophilic polymer, the surface of a lipid nanoparticle can be modified with polyalkylene glycol by constructing the lipid nanoparticle using a polyalkylene glycol-modified lipid as a constituent lipid of a lipid membrane. Surface modification with polyalkylene glycol may enhance the stability of the lipid nanoparticle such as the blood retention property.

As the polyalkylene glycol, for example, polyethylene glycol, polypropylene glycol, polytetramethylene glycol, polyhexamethylene glycol, or the like can be used. The molecular weight of the polyalkylene glycol is, for example, about 300 to about 10,000, preferably about 500 to about 10,000, and further preferably about 1,000 to about 5,000.

For example, stearylated polyethylene glycol (e.g., stearic acid PEG45 (STR-PEG45)) can be used for modification of a lipid with polyethylene glycol. In addition, polyethylene glycol derivatives such as N-[carbonyl-methoxypolyethylene glycol-2000]-1,2-dipalmitoyl-sn-glycero-3-phosphoethanolamine, N-[carbonyl-methoxypolyethylene glycol-5000]-1,2-dipalmitoyl-sn-glycero-3-phosphoethanolamine, N-[carbonyl-methoxypolyethylene glycol-750]-1,2-distearoyl-sn-glycero-3-phosphoethanolamine, N-[carbonyl-methoxypolyethylene glycol-2000]-1,2-distearoyl-sn-glycero-3-phosphoethanolamine, N-[carbonyl-methoxypolyethylene glycol-5000]-1,2-distearoyl-sn-glycero-3-phosphoethanolamine, and 1,2-dimyristoyl-rac-glycero-3-methoxypolyethylene glycol-2000 (PEG-DMG) can also be used, but polyalkylene glycolated lipids are not limited thereto.

The proportion of the polyalkylene glycol-modified lipid to the total lipid amount constituting the lipid nanoparticle according to the present invention is not particularly limited as long as it does not impair the selectivity of the pH-sensitive cationic lipid according to the present invention to the target tissue, specifically, the target tissue-specific gene expression activity when the lipid nanoparticle according to the present invention is used as a gene expression carrier. For example, the proportion of the polyalkylene glycol-modified lipid to the total lipid amount constituting the lipid nanoparticle is preferably 0.5 to 3 mol%.

The lipid nanoparticle according to the present invention can be subjected to appropriate surface modification or the like as necessary.

The blood retention property of the lipid nanoparticle according to the present invention can be enhanced by modifying the surface with a hydrophilic polymer or the like. Surface modification may be performed by using a lipid modified with these modifying groups as a constituent lipid of the lipid nanoparticle.

In producing the lipid nanoparticle according to the present invention, for example, glycophorin, ganglioside GM1, phosphatidylinositol, ganglioside GM3, glucuronic acid derivative, glutamic acid derivative, polyglycerin phospholipid derivative, or the like can also be used as a lipid derivative for enhancing the blood retention property. In addition to polyalkylene glycol, dextran, pullulan, Ficoll, polyvinyl alcohol, styrenemaleic anhydride alternating copolymer, divinyl ether-maleic anhydride alternating copolymer, amylose, amylopectin, chitosan, mannan, cyclodextrin, pectin, carrageenan, or the like can also be used for surface modification as the hydrophilic polymer for enhancing the blood retention property.

Further, in order to promote nuclear translocation of the lipid nanoparticle according to the present invention, for example, the surface of the lipid nanoparticle can be modified with an oligosaccharide compound having three or more saccharides. The type of the oligosaccharide compound having three or more saccharides is not particularly limited, but for example, an oligosaccharide compound in which about 3 to about 10 sugar units are bonded can be used, and preferably an oligosaccharide compound in which about 3 to about 6 sugar units are bonded can be used. Among them, preferably, an oligosaccharide compound which is a trimer to hexamer of glucose can be used, and more preferably, an oligosaccharide compound which is a trimer or tetramer of glucose can be used. More specifically, isomaltotriose, isopanose, maltotriose, maltotetraose, maltopentaose, or maltohexaose can be preferably used, and among them, maltotriose, maltotetraose, maltopentaose, or maltohexaose in which glucose is $\alpha$1-4 bonded is more preferable. Particularly preferable is maltotriose

or maltotetraose, and most preferable is maltotriose. The amount of the surface modification of the lipid nanoparticle with the oligosaccharide compound is not particularly limited, but is, for example, about 1 to about 30 mol%, preferably about 2 to about 20 mol%, and more preferably about 5 to about 10 mol% with respect to the total lipid amount.

The method for modifying the surface of the lipid nanoparticle with an oligosaccharide compound is not particularly limited; for example, a liposome in which the surface of the lipid nanoparticle is modified with a monosaccharide such as galactose or mannose (International Publication No. WO 2007/102481) is known, and thus the surface modification method described in this publication can be employed. All of the disclosure of the above-mentioned publication is included in the disclosure of this specification by reference.

Further, the lipid nanoparticle according to the present invention can be imparted with, for example, any one or two or more functions such as a temperature change-sensitive function, a membrane permeation function, a gene expression function, and a pH-sensitive function. By appropriately adding these functions, it is possible to improve the retention property of the lipid nanoparticle in blood, efficiently release the lipid nanoparticle from the endosome after endocytosis in the target cell, and more efficiently express the encapsulated nucleic acid in the target cell.

The lipid nanoparticle according to the present invention may contain one or more substances selected from the group consisting of antioxidants such as tocopherol, propyl gallate, ascorbyl palmitate, or butylated hydroxytoluene, charged substances, and membrane polypeptides. Examples of the charged substances for imparting a positive charge include saturated or unsaturated aliphatic amines such as stearylamine and oleylamine, and examples of the charged substances for imparting a negative charge include dicetyl phosphate, cholesteryl hemisuccinate, phosphatidylserine, phosphatidylinositol, and phosphatidic acid. Examples of membrane polypeptides include peripheral membrane polypeptides and integral membrane polypeptides. The blending amount of these substances is not particularly limited and can be appropriately selected depending on the purpose.

The size of the lipid nanoparticle according to the present invention is preferably an average particle size of 400 nm or less, more preferably an average particle size of 300 nm or less, further preferably an average particle size of 200 nm or less, and still more preferably 150 nm or less, since a high delivery efficiency to target cells in vivo can be easily obtained. The average particle size of the lipid nanoparticle means a number average particle size measured by dynamic light scattering (DLS). Measurement by dynamic light scattering can be carried out by a conventional method using a commercially available DLS apparatus or the like.

The polydispersity index (PDI) of the lipid nanoparticle according to the present invention is about 0.01 to about 0.7, preferably about 0.01 to about 0.6, and further preferably about 0.01 to about 0.3. The zeta potential can be in the range of -50 mV to 5 mV, preferably in the range of -10 mV to 5 mV.

The form of the lipid nanoparticle according to the present invention is not particularly limited, and examples thereof include a unilamellar liposome, a multilamellar liposome, a spherical micelle, and an amorphous layered structure, as a form dispersed in an aqueous solvent. The lipid nanoparticle according to the present invention is preferably a unilamellar liposome or a multilamellar liposome.

The lipid nanoparticle according to the present invention preferably encapsulates a component of interest to be delivered into a target cell inside the particle covered with a lipid membrane. The component encapsulated inside the particle by the lipid nanoparticle according to the present invention is not particularly limited as long as it has a size that can be encapsulated. Any substance such as a nucleic acid, a saccharide, a peptide, a low molecular weight compound, and a metal compound can be encapsulated in the lipid nanoparticle according to the present invention.

A nucleic acid is preferable as the component to be encapsulated in the lipid nanoparticle according to the present invention. The nucleic acid may be DNA, RNA, or an analog or derivative thereof (e.g., peptide nucleic acid (PNA) or phosphorothioate DNA). The nucleic acid to be encapsulated in the lipid nanoparticle according to the present invention may be a single-stranded nucleic acid or a double-stranded nucleic acid, and may be linear or circular.

The nucleic acid to be encapsulated in the lipid nanoparticle according to the present invention preferably contains a foreign gene to be expressed in a target cell, and more preferably is a nucleic acid that functions to express the foreign gene in a cell by being taken up into the cell. The foreign gene may be a gene originally contained in the genomic DNA of the target cell or a gene not contained in the genomic DNA. Examples of such nucleic acids include gene expression vectors containing a nucleic acid comprising a base sequence encoding a gene to be expressed. The gene expression vector may exist as an extrachromosomal gene in a transfected cell, or may be integrated into genomic DNA by homologous recombination.

The gene expression vector to be encapsulated in the lipid nanoparticle according to the present invention is not particularly limited, and a vector generally used in gene therapy or the like can be used. The gene expression vector to be encapsulated in the lipid nanoparticle according to the present invention is preferably a nucleic acid vector such as a plasmid vector. The plasmid vector may be in a circular form, or may be encapsulated in the lipid nanoparticle according to the present invention in a state of being linearly cleaved in advance. The gene expression vector can be designed by a conventional method using generally used molecular biological tools based on the base sequence information of the target gene to be expressed, and can be produced by various known methods.

The nucleic acid to be encapsulated in the lipid nanoparticle according to the present invention is also preferably a

functional nucleic acid that controls the expression of a target gene present in a target cell. Examples of the functional nucleic acid include antisense oligonucleotides, antisense DNA, antisense RNA, siRNA, microRNA, and mRNA. It may also be plasmid DNA (pDNA) serving as an siRNA expression vector that expresses siRNA in a cell. The siRNA expression vector can be prepared from a commercially available siRNA expression vector, or can be appropriately modified. The nucleic acid to be encapsulated in the lipid nanoparticle according to the present invention is preferably mRNA or pDNA because of its particularly good selectivity for tissues in vivo.

The method for producing the lipid nanoparticle according to the present invention is not particularly limited, and any method available to those skilled in the art can be employed. It can be produced as follows: for example, all lipid components are dissolved in an organic solvent such as chloroform, and a lipid membrane is formed by drying under reduced pressure using an evaporator or spray drying using a spray dryer, and then a component to be encapsulated in the lipid nanoparticle, for example, an aqueous solvent containing a nucleic acid or the like, is added to the dried mixture, and further emulsified by an emulsifier such as a homogenizer, an ultrasonic emulsifier, or a high-pressure jet emulsifier. It can also be produced by a method well known as a method for producing liposomes, such as a reverse phase evaporation method. When it is desired to control the size of the lipid nanoparticle, extrusion through a membrane filter having a uniform pore size or the like under high pressure may be performed.

The composition of the aqueous solvent (dispersion medium) is not particularly limited, but examples thereof include buffer solutions such as phosphate buffer, citrate buffer, and phosphate buffered saline, physiological saline, and cell culture media. These aqueous solvents (dispersion media) can stably disperse the lipid nanoparticles, but further, sugars (aqueous solutions) such as monosaccharides such as glucose, galactose, mannose, fructose, inositol, ribose, and xylose, disaccharides such as lactose, sucrose, cellobiose, trehalose, and maltose, trisaccharides such as raffinose and melezitose, polysaccharides such as cyclodextrin, and sugar alcohols such as erythritol, xylitol, sorbitol, mannitol, and maltitol, and polyhydric alcohols (aqueous solutions) such as glycerin, diglycerin, polyglycerin, propylene glycol, polypropylene glycol, ethylene glycol, diethylene glycol, triethylene glycol, polyethylene glycol, ethylene glycol monoalkyl ether, diethylene glycol monoalkyl ether, and 1,3-butylene glycol may be added. In order to stably store the lipid nanoparticles dispersed in this aqueous solvent for a long period of time, it is desirable to exclude electrolytes in the aqueous solvent as much as possible from the viewpoint of physical stability such as aggregation suppression. From the viewpoint of chemical stability of the lipid, it is desirable to set the pH of the aqueous solvent to near neutral from weakly acidic (about pH 3.0 to 8.0) and/or to remove dissolved oxygen by nitrogen bubbling or the like.

The lipid nanoparticle according to the present invention can also be produced by an alcohol dilution method using a flow path. The method is a method for producing lipid nanoparticles by introducing a solution in which lipid components are dissolved in an alcohol solvent and a solution in which a water-soluble component to be included in the lipid nanoparticle is dissolved in an aqueous solvent from separate flow paths and merging them. By using a microchannel with a built-in three-dimensional micromixer capable of achieving instantaneous mixing of two liquids, lipid nanoparticles having a diameter of about 30 nm can be reproducibly produced (Non-patent Literature 5). As the flow path used for production, it is preferable to use a simple two-dimensional flow path structure in which baffles of a certain width are arranged alternately from both sides in a micro-sized flow path through which a raw material solution flows, as described in Patent Literature 2, since it can form a nano-sized lipid particle formation system with high particle size controllability. As the aqueous solvent used in the alcohol dilution method, the solvents described above can be used.

When the resulting aqueous dispersion of the lipid nanoparticle is freeze-dried or spray-dried, for example, a use of sugars (aqueous solutions) such as monosaccharides such as glucose, galactose, mannose, fructose, inositol, ribose, and xylose, disaccharides such as lactose, sucrose, cellobiose, trehalose, and maltose, trisaccharides such as raffinose and melezitose, polysaccharides such as cyclodextrin, and sugar alcohols such as erythritol, xylitol, sorbitol, mannitol, and maltitol may improve stability. Further, when the aqueous dispersion is frozen, for example, a use of the above-mentioned sugars or polyhydric alcohols (aqueous solutions) such as glycerin, diglycerin, polyglycerin, propylene glycol, polypropylene glycol, ethylene glycol, diethylene glycol, triethylene glycol, polyethylene glycol, ethylene glycol monoalkyl ether, diethylene glycol monoalkyl ether, and 1,3-butylene glycol may improve stability.

The lipid nanoparticle according to the present invention functions as a gene expression carrier to a target tissue. By encapsulating a foreign gene of interest to be expressed in a target cell in the lipid nanoparticle according to the present invention and administering the lipid nanoparticle to a subject animal, the foreign gene is expressed in the target tissue of the subject animal. The lipid nanoparticle according to the present invention can also encapsulate a medicinal ingredient other than a nucleic acid and introduce the medicinal ingredient into a target tissue, and is also useful as a drug delivery carrier. Therefore, the lipid nanoparticle according to the present invention is useful as an active ingredient of a pharmaceutical composition used for various treatments including gene therapy.

Highly liver-selective lipid nanoparticles can be produced by using a compound having high tissue specificity for the liver among the pH-sensitive cationic lipids according to the present invention as a constituent lipid and selecting and using other constituent lipids that are easily taken up into the liver. Similarly, highly spleen-selective lipid nanoparticles can be produced by using a compound having high tissue specificity for the spleen among the pH-sensitive cationic lipids according to the present invention as a constituent lipid and selecting and using other constituent lipids that are easily

taken up into the spleen. When such highly liver-selective lipid nanoparticles or highly spleen-selective lipid nanoparticles encapsulating a gene expression vector are administered to an animal, the gene expression vector encapsulated in the lipid nanoparticles is selectively expressed in the liver or spleen rather than in other organs. Similarly, when highly liver-selective lipid nanoparticles or highly spleen-selective lipid nanoparticles among the lipid nanoparticles according to the present invention, encapsulating an siRNA expression vector, are administered to an animal, the siRNA expression vector encapsulated in the lipid nanoparticles is selectively expressed in the liver or spleen rather than in other organs, and the expression of the gene targeted by the expression vector is suppressed.

Highly liver-selective lipid nanoparticles include those in which phosphatidylcholine has a content ratio of 7 mol% or less, preferably 2 to 7 mol%, more preferably 3 to 5 mol%, based on the total constituent lipids and an apparent pKa of 7.5 or less, preferably 7.2 or less. Lipid nanoparticles having an apparent pKa within the above range can be prepared, for example, by using a lipid having a pKa of 7.5 or less, preferably 6.0 to 7.4, among the pH-sensitive cationic lipids according to the present invention as a constituent lipid.

Highly spleen-selective lipid nanoparticles include those in which phosphatidylcholine has a content ratio of 8 mol% or more, preferably 10 mol% or more, more preferably 10 to 25 mol%, further preferably 10 to 20 mol%, based on the total constituent lipids and a lipid having a pKa of about 6.2 to about 6.6 among the pH-sensitive cationic lipids according to the present invention is used as a constituent lipid. Among them, those in which phosphatidylcholine has a content ratio of 8 mol% or more based on the total constituent lipids and a lipid in which $-Z^1$-X is the general formula (H-5), (H-16), or (H-4) among the pH-sensitive cationic lipids according to the present invention is used as a constituent lipid are preferable.

Lipid nanoparticles having a relatively high content ratio of phosphatidylcholine based on the total constituent lipids and a lipid having a pKa of about 6.2 to about 6.6 among the pH-sensitive cationic lipids according to the present invention as a constituent lipid have high selectivity for B cells and dendritic cells among cells constituting the spleen. For example, highly B cell-selective lipid nanoparticles include those in which phosphatidylcholine has a content ratio of 5 mol% or more, preferably 10 mol% or more, more preferably 10 to 25 mol%, further preferably 10 to 20 mol%, based on the total constituent lipids and a lipid having a pKa of about 6.2 to about 6.6, preferably a lipid in which $-Z^1$-X is the general formula (H-5), (H-16), or (H-4), among the pH-sensitive cationic lipids according to the present invention is used as a constituent lipid. Highly dendritic cell-selective lipid nanoparticles include those in which phosphatidylcholine has a content ratio of 3 mol% or more, preferably 5 mol% or more, more preferably 5 to 25 mol%, further preferably 5 to 20 mol%, based on the total constituent lipids and a lipid having a pKa of about 6.2 to about 6.6, preferably a lipid in which $-Z^1$-X is the general formula (H-5), (H-16), or (H-4), among the pH-sensitive cationic lipids according to the present invention is used as a constituent lipid.

Lipid nanoparticles for use as a carrier targeting the lung include those having an apparent pKa of 7.5 or more, preferably 7.5 to 8.5. Highly lung-selective lipid nanoparticles preferably include those in which a lipid in which $-Z^1$-X is the general formula (H-19), (H-18), (H-2), (H-3), or (H-4) among the pH-sensitive cationic lipids according to the present invention is used as a constituent lipid.

Lipid nanoparticles for use as a carrier targeting the brain preferably include, for example, those in which a lipid in which $-Z^1$-X is the general formula (H-4) among the pH-sensitive cationic lipids according to the present invention is used as a constituent lipid.

Lipid nanoparticles for use as a carrier targeting the heart preferably include, for example, those in which a lipid in which $-Z^1$-X is the general formula (H-19), (H-4), (H-16), or (H-7) among the pH-sensitive cationic lipids according to the present invention is used as a constituent lipid.

Lipid nanoparticles for use as a carrier targeting the muscle preferably include, for example, those in which a lipid in which $-Z^1$-X is the general formula (H-18), (H-19), (H-4), or (H-16) among the pH-sensitive cationic lipids according to the present invention is used as a constituent lipid.

The animal to which the lipid nanoparticle according to the present invention is administered is not particularly limited, and may be a human or an animal other than a human. Examples of non-human animals include mammals such as cattle, pigs, horses, sheep, goats, monkeys, dogs, cats, rabbits, mice, rats, hamsters, and guinea pigs, and birds such as chickens, quails, and ducks. The administration route for administering the lipid nanoparticle according to the present invention to an animal is not particularly limited, but is preferably parenteral administration such as intravenous administration, enteral administration, intramuscular administration, subcutaneous administration, transdermal administration, nasal administration, and pulmonary administration.

## Examples

Next, the present invention will be described in more detail with reference to Examples, but the present invention is not limited to the following Examples.

<NMR>

In the following experiments, unless otherwise specified, $^1H$ and $^{13}C$ NMR spectra were obtained using an NMR apparatus manufactured by JEOL Ltd. (ECA500 apparatus, ECZ400 apparatus, or ECX400P apparatus) with tetramethylsilane as an internal standard (0 ppm), wherein d is an abbreviation for doublet, t is an abbreviation for triplet, and m is an abbreviation for multiplet. Chemical shifts of the $^1H$ NMR spectra were reported in ppm on the $\sigma$ scale, downfield from tetramethylsilane.

<Constituent Lipids of Lipid Nanoparticles>

YSK05 synthesized by the method described in Non-patent Literature 2 was used. 7-(4-(Dimethylamino) butyl)-7-hydroxytridecane-1,13-diyl dioleate (CL1H6), 7-(4-(diisopropylamino)butyl)-7-hydroxytridecane-1,13-diyl dioleate (CL4H6), and 7-hydroxy-7-(4-((1-methylpiperidine-4-carbonyl)oxy)butyl)tridecane-1,13-diyl dioleate (CL15H6) synthesized by the methods described in Patent Literature 1 were used. CL4F6 (7-(4-(diisopropylamino)butyl)-13-((2-hexylnonanoyl)oxy)-7-hydroxytridecyl 2-hexyldecanoate) synthesized in Comparative Synthesis Example 1 below was used. 7-Hydroxy-7-(4-morpholinobutyl)tridecane-1,13-diyl dioleate (CL7H6) synthesized in Comparative Synthesis Example 2 below was used. SM-102 manufactured by Cayman Chemical, ALC-0315 manufactured by Ambeed, DLin-MC3-DMA (MC3) manufactured by Selleck Biotech, and cholesterol (Chol) manufactured by Sigma-Aldrich were respectively used. 1,2-Distearoyl-sn-glycero-3-phosphocholine (DSPC), 1,2-dipalmitoyl-sn-glycero-3-phosphocholine (DPPC), 1-palmitoyl-2-oleoyl-sn-glycero-3-phosphocholine (POPC), 1,2-dioleoyl-sn-glycero-3-phosphocholine (DOPC), 1-palmitoyl-2-oleoyl-sn-glycero-3-phosphoethanolamine (POPE), 1,2-dioleoyl-sn-glycero-3-phosphoethanolamine (DOPE), methoxyethylene glycol 2000-modified 2-dimyristoyl-rac-glycerol (PEG-DMG), and methoxyethylene glycol 2000-modified 2-distearoyl-rac-glycerol (PEG-DSG) manufactured by NOF CORPORATION were used.

<Thin Layer Chromatography (TLC)>

In the following experiments, unless otherwise specified, the reactants of all reactions were monitored by TLC using pre-coated TLC plates (manufactured by Millipore). TLC visualization was performed by UV light (254 nm), phosphomolybdic acid staining, or p-anisaldehyde staining. Reaction products were purified by an automated CombiFlash (registered trademark) Rf Chromatography System (manufactured by Teledyne ISCO) or a Selekt system (manufactured by Biotage).

<mRNA>

For the nucleic acids to be encapsulated in lipid nanoparticles, mRNA encoding firefly luciferase (Fluc) (CleanCap FLuc mRNA, 5moU) and mRNA encoding ovalbumin (OVA) (Ova mRNA) manufactured by TriLink Biotechnologies were used.

<Preparation of Lipid Nanoparticles>

In the following experiments, unless otherwise specified, lipid nanoparticles were prepared by an alcohol dilution method using a flow path.

For the preparation of lipid nanoparticles in Examples 11 to 18, a micromixer-integrated microfluidic device "iLiNP" (manufactured by Lilac Pharma) was used as the flow path. The flow rate of the device was controlled using a syringe pump (Harvard Apparatus).

Specifically, first, 80 $\mu$L of an ethanol solution containing each lipid component at a predetermined molar ratio and adjusted to a total lipid concentration of 8 mM and 240 $\mu$L of an acetate buffer (25 mM, pH 4.0) adjusted to a nucleic acid concentration of 55 $\mu$g/mL were fed into a microchannel, and a lipid nanoparticle solution discharged from the flow path was collected. The lipid nanoparticle solution was placed in a dialysis membrane (MWCO 12,000-14,000, manufactured by Spectrum Laboratories), dialyzed against PBS(-) (pH 7.4) as an external aqueous phase at 4°C for 2 hours or more, and then the lipid nanoparticle solution was collected from the dialysis membrane.

For the preparation of lipid nanoparticles in Examples 33 and subsequent ones, a baffle mixer-integrated microchannel iLiNP (manufactured by Lilac Pharma) was used.

Specifically, first, 13.75 $\mu$L of a 1 mg/mL mRNA solution was diluted with 25 mM acetate buffer (pH 4.0) to a volume of 250 $\mu$L, and the resulting mRNA solution was dispensed into a 1 mL syringe (manufactured by HAMILTON). Further, in the case of pH-sensitive cationic lipid/chol/DSPC/PEG-DSG=50/40/10/1.0 (mol%), 200 $\mu$L of a 10 mM pH-sensitive cationic lipid/ethanol solution, 160 $\mu$L of a 10 mM cholesterol/ethanol solution, 40 $\mu$L of a 10 mM DSPC/ethanol solution, 40 $\mu$L of a 1 mM PEG-DSG/ethanol solution, and 60 $\mu$L of ethanol were mixed to make a total volume of 500 $\mu$L,

and the resulting lipid solution (final lipid concentration: 8 mM, 100% ethanol) was dispensed into a 1 mL syringe (manufactured by HAMILTON). Each syringe was mounted on a syringe pump, the baffle mixer-integrated microchannel and the pump were connected, the RNA solution and the lipid solution were fed into the microchannel at a flow rate ratio of 3:1 (N/P=7.4) and a total flow rate of 500 μL/min, and a lipid nanoparticle solution discharged from the flow path was collected. The lipid nanoparticle solution was placed in a dialysis membrane (MWCO 12,000-14,000, manufactured by Spectrum Laboratories), dialyzed against PBS(-) (pH 7.4) as an external aqueous phase at 4°C for 2 hours or more to remove ethanol and exchange the buffer, and then the lipid nanoparticle solution was collected from the dialysis membrane.

<Measurement of Average Particle Size and Zeta Potential of Lipid Nanoparticles>

The average particle size (number average value), ζ-average particle size, and polydispersity index (PdI) of the lipid nanoparticles in PBS(-) (pH 7.4), and the zeta potential in 10 mM HEPES buffer (pH 7.4) were measured using an analyzer "Zetasizer Nano ZS ZEN3600" (manufactured by Malvern) utilizing dynamic light scattering.

<Encapsulation Rate (Encapsulation Efficiency) of Lipid Nanoparticles>

The encapsulation rate of the lipid nanoparticles was determined by measuring the amount of encapsulated nucleic acid (mRNA) using RiboGreen (manufactured by Life Technologies), which is an RNAintercalator. The amount of mRNA was quantified based on a calibration curve prepared from a dilution series solution of mRNA at a known concentration.

Specifically, 10 mM HEPES buffer (pH 7.4), 10 w/v% Triton X-100, and RiboGreen were mixed at 978.8 μL, 20 μL, and 1.25 μL per 10 wells, respectively, to obtain a Triton(+) buffer. A Triton(-) buffer was obtained in replacing Triton X-100 with 10 mM HEPES buffer (pH 7.4). 100 μL/well of the sample was added to a black 96-well plate for fluorescence measurement, and 100 μL/well of Triton(+) buffer or Triton(-) buffer was added. After the plate was gently shaken on a shaking incubator (SI-300, manufactured by AS ONE) (700 rpm, 30 seconds), the fluorescence intensity of each well was measured using a microplate reader (VARIOSKAN LUX, manufactured by Thermo Fisher Scientific) (wavelength Ex/Em=500 nm/525 nm), and the nucleic acid encapsulation rate (%) was calculated.

<Measurement of Apparent pKa of Lipid Nanoparticles>

The apparent pKa of the lipid nanoparticles was measured using p-toluenesulfonic acid (TNS). First, TNS (final concentration: 0.75 μM) and lipid nanoparticles (final concentration: 30 μM) were mixed in a buffer (200 mL) adjusted to each pH (pH 3.5 to 9.5). As the buffer, 20 mM citrate buffer, 20 mM sodium phosphate buffer, or 20 mM Tris-HCl buffer containing 130 mM NaCl was used. The fluorescence intensity of the prepared mixture was measured using a fluorescence spectrophotometer (Varioskan Lux, manufactured by Thermo Fisher Scientific) with the settings of λex=321 nm and λem=447 nm. Among the measured values, the highest value and the lowest value were set as 100% and 0% ionization degree, respectively, and the pH showing a 50% ionization degree was calculated as the apparent pKa. The content ratio (%) of cationically charged pH-sensitive cationic lipid was calculated using the Henderson-Hasselbalch equation.

<Administration of Lipid Nanoparticles to Mice>

Lipid nanoparticles were administered to mice by intravenous injection into the tail vein using a 27G needle. Unless otherwise specified, BALB/c mice (4 to 5 weeks old, female) were used.

<Measurement of Fluc activity>

First, BALB/c mice (female, 4 weeks old) were intravenously injected with lipid nanoparticles encapsulating Fluc mRNA (Fluc mRNA-loaded lipid nanoparticles) at a dose of 0.01 mg mRNA/kg. Six hours after intravenous injection, the mice were euthanized, each tissue was collected, frozen in liquid nitrogen, and stored at -80°C. The tissues were homogenized in passive lysis buffer (manufactured by Promega) using a bead-type cell disruptor (Micro Smash MS-100R, manufactured by TOMY Seiko). Tissue debris was removed by centrifugation, and the supernatant was collected. Fluc activity in the supernatant was measured using the Luciferase Assay System (E1500, manufactured by Promega) according to the manufacturer's protocol. Luminescence was measured using a luminometer (Luminescencer-PSN, manufactured by ATTO). Protein concentration was determined using a commercially available assay kit (BCA Protein Assay Kit, manufactured by Pierce). Fluc activity was expressed as relative light units (RLU) per mg of protein.

[Comparative Synthesis Example 1] Synthesis of CL4F6

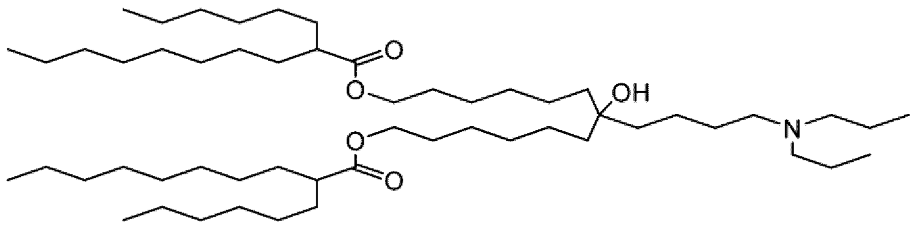

CL4F6

7-(4-(Diisopropylamino)butyl)tridecane-1,7,13-triol (1.0 mmol) synthesized by the method described in Patent Literature 1 was dissolved in 5 mL of dichloromethane, followed by an addition of 2-hexyldecanoic acid (2.20 mmol), DMAP (N,N-dimethyl-4-aminopyridine) (0.20 mmol), and EDCI (1-ethyl-3-(3-dimethylaminopropyl)carbodiimide) (3.0 mmol), and reacted at room temperature overnight. After removing the solvent using a rotary evaporator, the residue was suspended in ethyl acetate, and insoluble matters were removed by filtration. The filtrate was washed by liquid separation with 0.5 N aqueous sodium oxide solution and saturated brine. Anhydrous sodium sulfate was added to the organic layer for dehydration. After filtration, the solvent was removed using a rotary evaporator to obtain a crude product. The crude product was purified by silica gel chromatography [eluting solvent; dichloromethane:methanol (continuous gradient)] to obtain CL4F6.

[Comparative Synthesis Example 2] Synthesis of CL7H6

(1) Synthesis of 2,2,3,3,19,19,20,20-octamethyl-11-(4-morpholinobutyl)-4,18-dioxa-3,19-disilahenicosane-11-ol (compound 1)

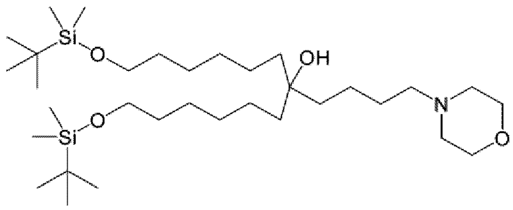

Morpholine (10.5 mL, 120 mmol) and DCM (dichloromethane, 29 mL) were added to 11-((tert-butyldimethylsilyl)oxy)-5-(6-((tert-butyldimethylsilyl)oxy)hexyl)-5-hydroxyundecyl 4-methylbenzenesulfonate (13.7 g, 20 mmol), and then the resulting reaction mixture was stirred at room temperature for 5 days. After evaporating the solvent from the reaction mixture, the residue was suspended with EtOAc and filtered. The resulting filtrate was washed with water (50 mL×2) and then brine (50 mL). The resulting organic phase was dried over $Na_2SO_4$, and then the filtrate was evaporated to obtain a crude product as a pale yellow oily residue. The residue was loaded on a silica gel column ($SiO_2$) and purified by flash chromatography with a gradient mobile phase of DCM and methanol. This gave compound 1 (10.3 g, yield 86%) as a pale yellow oil.

(2) Synthesis of 7-(4-morpholinobutyl)tridecane-1,7,13-triol (compound 2)

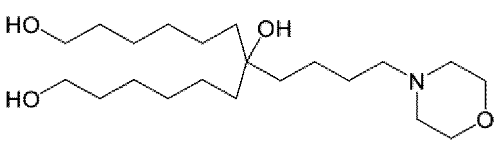

Compound 1 (10.3 g, 17.1 mmol) was dissolved in methanol (20 mL), and the resulting solution was cooled on ice. To the solution a 12 N HCl solution (5 mL) was added to obtain a reaction mixture, followed by stirring at room temperature overnight. After evaporating the solvent from the reaction mixture, the residue was diluted with an aqueous NaOH solution

(0.5 M, 100 mL) and extracted with EtOAc (2×100 mL). The resulting organic phases were combined, dried over $Na_2SO_4$, and then the solvent was evaporated to obtain a crude product as a pale yellow oily residue. The residue was loaded on a silica gel column ($SiO_2$) and purified by flash chromatography with a gradient mobile phase of DCM and methanol. This gave compound 2 (3.93 g, yield 53%) as a colorless oil.

(3) Synthesis of CL7H6

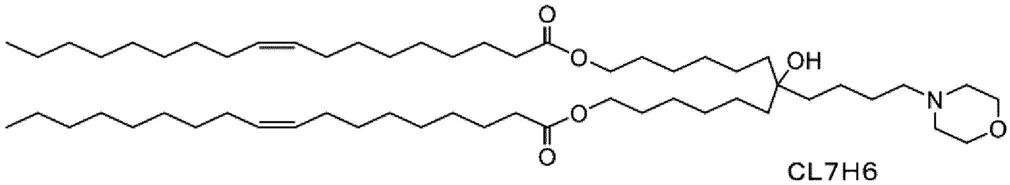

CL7H6

Compound 2 (3.93 g, 10.5 mmol) was dissolved in anhydrous DCM (30 mL) and oleic acid (6.54 g, 23.2 mmol), and then DMAP (283 mg, 2.32 mmol) and EDCI-HCl (5.05 g, 26.3 mmol) were added to the resulting mixture. The resulting reaction mixture was stirred at ambient temperature for 8 hours. After evaporating the solvent from the reaction mixture, the residue was suspended with EtOAc (100 mL), and the resulting suspension was washed with an aqueous NaOH solution (0.5 M, 100 mL) and then brine (50 mL). The resulting organic phase was dried over $Na_2SO_4$, and then the solvent was evaporated to obtain a crude product as a pale yellow oily residue. The residue was loaded on a silica gel column ($SiO_2$) and purified by flash chromatography with a gradient mobile phase of DCM, methanol, and ammonium hydroxide. This gave CL7H6 (9.17 g, yield 97%) as a pale yellow oil.

[Example 1]

An oleic acid residue was condensed to the hydroxyl group of Tris, and then 3-(dimethylamino)propionic acid was condensed to the Tris-derived amino group of the resulting ester to synthesize 2-(3-(dimethylamino)propanamido)-2-((oleoyloxy)methyl)propane-1,3-diyl dioleate (TOT-1).

(1) Synthesis of tert-butyl (1,3-dihydroxy-2-(hydroxymethyl)propan-2-yl)carbamate (Compound 1)

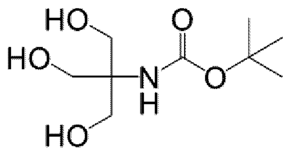

Di-tert-butyl dicarbonate (23.5 g, 107.7 mmol) dissolved in tert-butanol (75 mL) was added dropwise to a solution in which Tris (10.0 g, 82.6 mmol) was dispersed in a mixed solvent of methanol (75 mL) and tert-butanol (75 mL). The resulting reaction mixture was stirred at ambient temperature overnight and then evaporated. The resulting residue was purified by precipitation with cold EtOAc. Vacuum filtration gave compound 1 (16.7 g, yield 91%) as a white powder.

(2) Synthesis of 2-((tert-butoxycarbonyl)amino)-2-((oleoyloxy)methyl)propane-1,3-diyl dioleate (Compound 2)

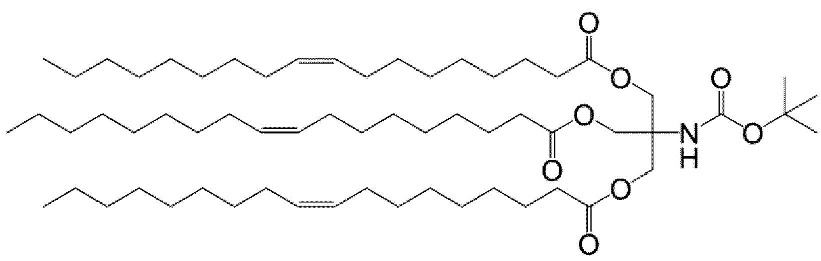

Compound 1 (2.21 g, 10.0 mmol) was dissolved in anhydrous DCM (50 mL), and further oleic acid (9.32 g, 33.0 mmol), DMAP (N,N-dimethyl-4-aminopyridine) (122 mg, 1.0 mmol), and EDCI-HCl (1-(3-dimethylaminopropyl)-3-ethylcarbodiimide hydrochloride) (7.67 g, 40.0 mmol) were dissolved in the resulting mixture. The resulting reaction mixture was stirred at ambient temperature overnight and then evaporated. The resulting residue was suspended with EtOAc, washed with saturated citric acid solution and water, and then dried over anhydrous $Na_2SO_4$. The solution was filtered, and the filtrate was evaporated. The residue was loaded on a normal-phase column (Sfär Silica HCD, manufactured by Biotage) and purified by flash chromatography with a gradient mobile phase of hexane and EtOAc. This gave compound 2 (8.50 g, yield 84%) as a colorless oil.

(3) Synthesis of 22-amino-2-((oleoyloxy)methyl)propane-1,3-diyl dioleate (TOT-0)

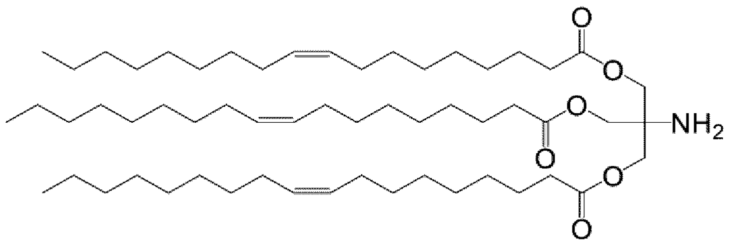

TOT-0

Compound 2 (8.50 g, 8.38 mmol) was dissolved in anhydrous DCM (44 mL), and TFA (trifluoroacetic acid) (5 mL) and TIPS (triisopropylsilane) (1 mL) were added to the resulting mixture. The resulting reaction mixture was stirred at ambient temperature for 3 hours. The reaction mixture was diluted with EtOAc, and the organic phase was washed with 1 N aqueous NaOH solution, water, and brine, and dried over anhydrous $Na_2SO_4$. The solution was filtered, and the filtrate was evaporated. The residue was loaded on a normal-phase column (Sfär Silica HCD, manufactured by Biotage) and purified by flash chromatography with a gradient mobile phase of hexane and EtOAc. This gave TOT-0 (5.00 g, yield 65%) as a colorless oil.

(4) Synthesis of 2-(3-(dimethylamino)propanamido)-2-((oleoyloxy)methyl)propane-1,3-diyl dioleate (TOT-1).

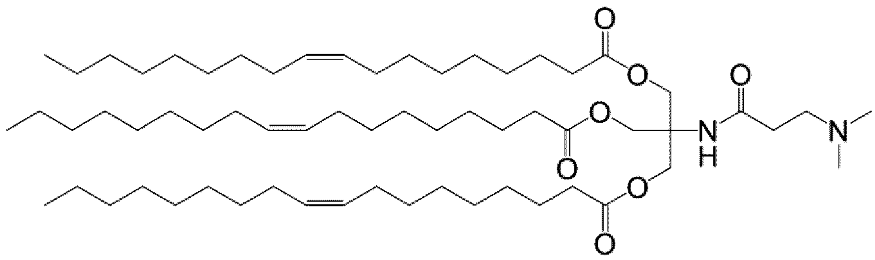

TOT-1

TOT-0 (366 mg, 0.40 mmol) was dissolved in anhydrous DCM (4 mL), and 3-(dimethylamino)propionic acid hydrochloride (76.8 mg, 0.50 mmol), DMAP (4.9 mg, 0.04 mmol), TEA (83.6 μL, 0.60 mmol), and EDCI (115 mg, 0.60 mmol) were added to the resulting mixture. The reaction mixture was stirred at 35°C overnight and then evaporated. The resulting residue was suspended with hexane/EtOAc (1 mL: 1.4 mL), washed with 0.5 N aqueous NaOH solution (5 mL), and then dried over anhydrous $Na_2SO_4$. The resulting solution was filtered, and the filtrate was evaporated. The residue was loaded on a normal-phase column (Sfär Silica HCD, manufactured by Biotage) and purified by flash chromatography with a gradient mobile phase of DCM and methanol. This gave TOT-1 (245 mg, yield 60%) as a colorless oil.

[Example 2]

4-(Dimethylamino)butyric acid was condensed to the amino group in TOT-0 synthesized in Example 1 to synthesize 2-(4-(dimethylamino)butanamido)-2-((oleoyloxy)methyl)propane-1,3-diyl dioleate (TOT-2).

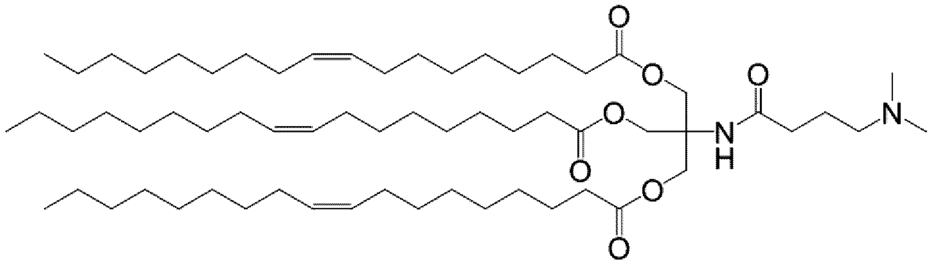

TOT-2

TOT-2 (341 mg, yield 83%) was obtained as a colorless oil in the same manner as in Example 1, except that 4-(dimethylamino)butyric acid hydrochloride (83.8 mg, 0.50 mmol) was reacted with TOT-0 instead of 3-(dimethylamino) propionic acid hydrochloride.

[Example 3]

4-(Dimethylamino)butyric acid was condensed to the amino group in TOT-0 synthesized in Example 1 to synthesize 2-(1-methylpiperidine-4-carboxamide)-2-((oleoyloxy)methyl)propane-1,3-diyl dioleate (TOT-3).

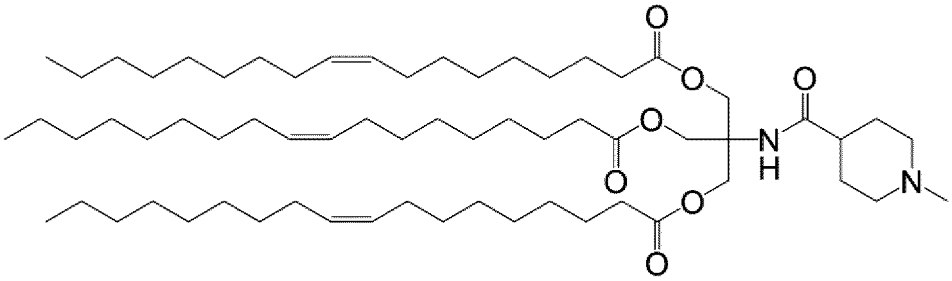

TOT-3

TOT-3 (345 mg, yield 83%) was obtained as a white wax in the same manner as in Example 1, except that 1-methyl-piperazine-4-carboxylic acid hydrochloride (89.8 mg, 0.50 mmol) was reacted with TOT-0 instead of 3-(dimethylamino)propionic acid hydrochloride.

[Example 4]

3-(Dimethylamino)propionic acid was condensed to the amino group in TOT-0 synthesized in Example 1 to synthesize 2-(3-(diethylamino)propanamido)-2-((oleoyloxy)methyl)propane-1,3-diyl dioleate (TOT-4).

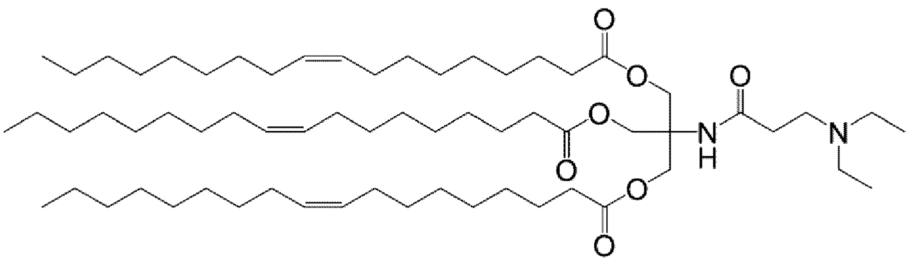

TOT-4

TOT-4 (67.6 mg, yield 16%) was obtained as a colorless oil in the same manner as in Example 1, except that 3-(dimethylamino)propionic acid hydrochloride (90.8 mg, 0.50 mmol) was reacted with TOT-0 instead of 3-(dimethylamino)propionic acid hydrochloride.

[Example 5]

3-(Dimethylamino)propionic acid was condensed to the amino group in TOT-0 synthesized in Example 1 to synthesize 2-((oleoyloxy)methyl)-2-(3-(piperidin-1-yl)propanamido)propane-1,3-diyl dioleate (TOT-5).

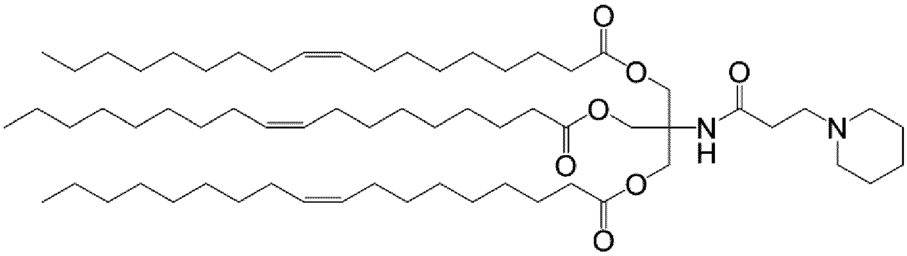

TOT-5

TOT-5 (146 mg, yield 35%) was obtained as a colorless oil in the same manner as in Example 1, except that 1-piperidinepropionic acid (78.6 mg, 0.50 mmol) was reacted with TOT-0 instead of 3-(dimethylamino)propionic acid hydrochloride.

[Example 6]

3-(Dimethylamino)propionic acid was condensed to the amino group in TOT-0 synthesized in Example 1 to synthesize 2-(2-aminoacetamido)-2-((oleoyloxy)methyl)propane-1,3-diyl dioleate (TOT-6).

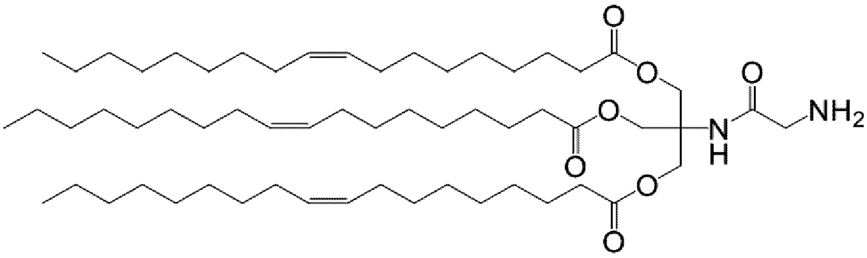

TOT-6

TOT-0 (366 mg, 0.40 mmol) was dissolved in EtOH (4 mL), and N-[(9H-fluoren-9-ylmethoxy)carbonyl]-glycine (131 mg, 0.44 mmol) and DMT-MM (4-(4,6-dimethoxy-1,3,5-triazin-2-yl)-4-methylmorpholinium chloride) (122 mg, 0.44 mmol) were added to the resulting mixture. The resulting reaction mixture was stirred at ambient temperature overnight and then evaporated. The resulting residue was suspended with EtOAc (4 mL), washed with 0.5 N aqueous NaOH solution (5 mL), and then dried over anhydrous $Na_2SO_4$. The resulting solution was filtered, the filtrate was evaporated, and then DMF (N,N-dimethylformamide) (1.8 mL) and piperidine (200 $\mu$L) were added to the mixture. The resulting reaction mixture was stirred at ambient temperature overnight and then evaporated. The residue was loaded on a normal-phase column (Sfär Silica HCD, manufactured by Biotage) and purified by flash chromatography with a gradient mobile phase of hexane and AcOEt. This gave TOT-6 (252 mg, yield 65%) as a colorless oil.

[Example 7]

N-[(9H-Fluoren-9-ylmethoxy)carbonyl]-methyl-glycine was condensed to the amino group in TOT-0 synthesized in Example 1 to synthesize 2-(2-(methylamino)acetamido)-2-((oleoyloxy)methyl)propane-1,3-diyl dioleate (TOT-7).

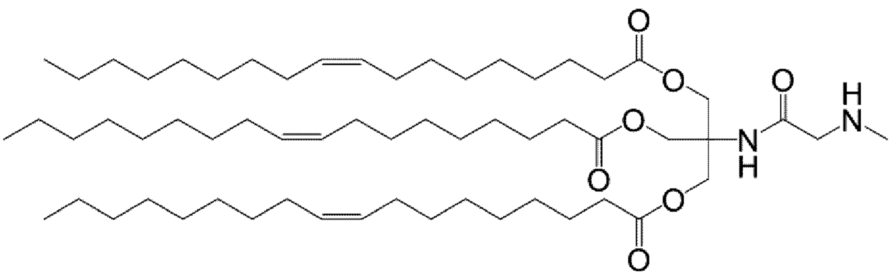

TOT-7

TOT-0 (366 mg, 0.40 mmol) was dissolved in ethanol (4 mL), and N-[(9H-fluoren-9-ylmethoxy)carbonyl]-methyl-glycine (137 mg, 0.44 mmol) and DMT-MM (122 mg, 0.44 mmol) were added to the resulting mixture. The reaction mixture was stirred at ambient temperature overnight and then evaporated. The resulting residue was suspended with EtOAc (4 mL), washed with 0.5 N aqueous NaOH solution (5 mL), and then dried over anhydrous $Na_2SO_4$. The resulting solution was filtered, and the filtrate was evaporated. DMF (1.8 mL) and piperidine (200 $\mu$L) were added to the residue, followed by

stirring at ambient temperature overnight. The resulting reaction mixture was evaporated, and then the residue was loaded on a normal-phase column (Sfär Silica HCD, manufactured by Biotage) and purified by flash chromatography with a gradient mobile phase of hexane and AcOEt. This gave TOT-7 (288 mg, yield 73%) as a colorless oil.

[Example 8]

O-tert-Butyl-N-[(9H-fluoren-9-ylmethoxy)carbonyl]-L-serine was condensed to the amino group in TOT-0 synthesized in Example 1 to synthesize 2-((S)-2-amino-3-hydroxypropanamido)-2-((oleoyloxy)methyl)propane-1,3-diyl dioleate (TOT-8).

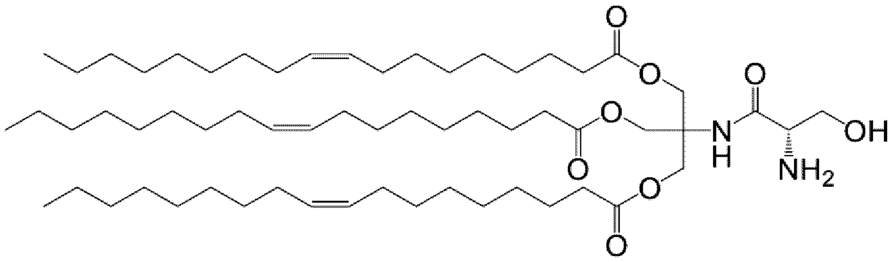

TOT-8

TOT-0 (366 mg, 0.40 mmol) was dissolved in ethanol (4 mL), and O-tert-butyl-N-[(9H-fluoren-9-ylmethoxy) carbonyl]-L-serine (169 mg, 0.44 mmol) and DMT-MM (122 mg, 0.44 mmol) were added to the resulting mixture. The reaction mixture was stirred at ambient temperature overnight and then evaporated. The resulting residue was suspended with EtOAc (4 mL), washed with 0.5 N aqueous NaOH solution (5 mL), and then dried over anhydrous $Na_2SO_4$. The resulting solution was filtered, and the filtrate was evaporated. The residue was dissolved in anhydrous DCM (2.8 mL), and TFA (0.8 mL) and TIPS (0.4 mL) were added thereto. The resulting reaction mixture was stirred at ambient temperature overnight and then diluted with EtOAc. The resulting organic phase was washed with 1 N aqueous NaOH solution, water, and brine, and then dried over anhydrous $Na_2SO_4$. The resulting solution was filtered, and the filtrate was evaporated. DMF (1.8 mL) and piperidine (200 μL) were added to the residue, followed by stirring at ambient temperature overnight. The resulting reaction mixture was evaporated, and then the residue was loaded on a normal-phase column (Sfär Silica HCD, manufactured by Biotage) and purified by flash chromatography with a gradient mobile phase of hexane and AcOEt. This gave TOT-8 (157 mg, yield 39%) as a colorless oil.

[Example 9]

N-Ethyl-N-methyl-β-alanine was condensed to the amino group in TOT-0 synthesized in Example 1 to synthesize 2-(3-(ethyl(methyl)amino)propanamido)-2-((oleoyloxy)methyl)propane-1,3-diyl dioleate (TOT-16).

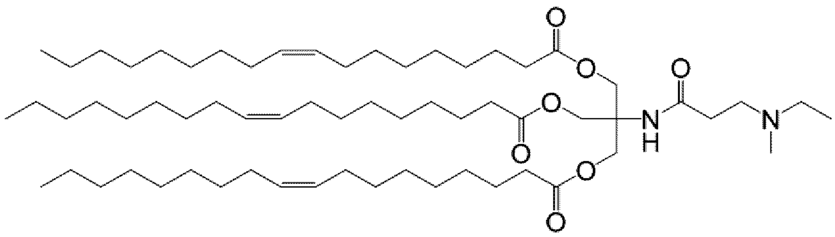

TOT-16

TOT-0 (366 mg, 0.40 mmol) was dissolved in anhydrous DCM (2 mL) and DMF (2 mL), and N-ethyl-N-methyl-β-alanine (83.8 mg, 0.50 mmol), DMAP (4.9 mg, 0.04 mmol), TEA (83.6 μL, 0.60 mmol), and EDCI (115 mg, 0.60 mmol) were added to the resulting mixture. The reaction mixture was stirred at ambient temperature overnight and then evaporated. The resulting residue was suspended with EtOAc (4 mL), washed with 0.5 N aqueous NaOH solution (5 mL), and then dried over anhydrous $Na_2SO_4$. The resulting solution was filtered, and the filtrate was evaporated. The residue was loaded on a normal-phase column (Sfär Silica HCD, manufactured by Biotage) and purified by flash chromatography with a gradient mobile phase of hexane and AcOEt. This gave TOT-16 (27.1 mg, yield 6.6%) as a colorless oil.

[Example 10]

3-(Pyrrolidin-1-yl)propanoic acid was condensed to the amino group in TOT-0 synthesized in Example 1 to synthesize 2-((oleoyloxy)methyl)-2-(3-(pyrrolidin-1-yl)propanamido)propane-1,3-diyl dioleate (TOT-17).

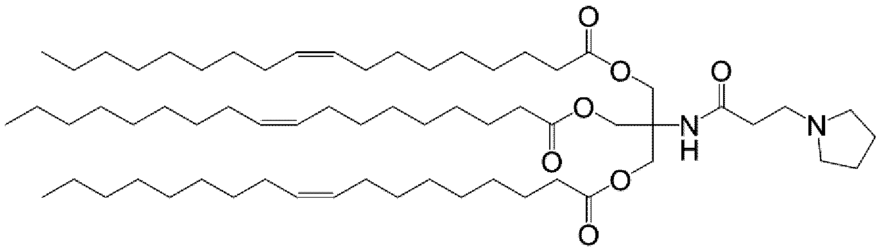

TOT-17

TOT-0 (366 mg, 0.40 mmol) was dissolved in anhydrous DCM (2 mL) and DMF (2 mL), and 3-(pyrrolidin-1-yl) propanoic acid hydrochloride (89.8 mg, 0.50 mmol), DMAP (4.9 mg, 0.04 mmol), TEA (83.6 μL, 0.60 mmol), and EDCI (115 mg, 0.60 mmol) were added to the resulting mixture. The reaction mixture was stirred at ambient temperature overnight and then evaporated. The resulting residue was suspended with EtOAc (4 mL), washed with 0.5 N aqueous NaOH solution (5 mL), and then dried over anhydrous $Na_2SO_4$. The resulting solution was filtered, and the filtrate was evaporated. The residue was loaded on a normal-phase column (Sfär Silica HCD, manufactured by Biotage) and purified by flash chromatography with a gradient mobile phase of hexane and AcOEt. This gave TOT-17 (36.3 mg, yield 8.7%) as a colorless oil.

[Test Example 1]

TLC was performed on TOT-1 to TOT-5 synthesized in Examples 1 to 5, YSK05, CL1H6, CL4H6, CL7H6, CL15H6, CL4F6, SM-102, ALC-0315, and MC3 to examine the affinity for a polar surface.

Specifically, each ionizable lipid (5 to 10 nmol, respectively) was spotted on an aluminum TLC plate (silica gel), developed using dichloromethane:methanol (19:1 or 9:1) (volume ratio) as a mobile phase, and stained with p-anisaldehyde, and the mobility (Rf value) was measured.

Fig. 1 shows a plot diagram plotting Rf values of each lipid against apparent pKa values. Fig. 1(A) shows the results of TLC using dichloromethane:methanol (9:1) as a mobile phase, and Fig. 1(B) shows the results of TLC using dichloromethane:methanol (19:1) as a mobile phase. In the figure, black circles are plots of TOT (TOT-1 to TOT-5), and white circles are plots of lipids other than TOT (YSK05, CL1H6, CL4H6, CL7H6, CL15H6, CL4F6, SM-102, ALC-0315, and MC3). As shown in Fig. 1, the mobility (Rf value) of each ionizable lipid correlated with the apparent pKa value. In particular, TOT showed a higher Rf value than the other lipids. These results suggested that, in TOT, the hydrophilic group composed of X is sterically shielded by the three hydrocarbon chains composed of $R^1$, and the interaction with highly polar silica gel is weak.

[Example 11]

Lipid nanoparticles were prepared using TOT-5 synthesized in Example 5 as a constituent lipid.

Phosphatidylethanolamine (PE), cholesterol, and PEG-DMG were used as lipids other than TOT-5. DOPE was used as PE.

Specifically, TOT-5, DOPE, cholesterol, and PEG-DMG were used in a composition of TOT-5/DOPE/chol/PEG-DMG=50 to 65/10 to 35/0 to 40/1 (mol%) to prepare lipid nanoparticles loaded with Fluc mRNA (Fluc mRNA-loaded lipid nanoparticles) by the alcohol dilution method.

Next, each of the prepared Fluc mRNA-loaded lipid nanoparticles was intravenously administered into the tail vein of BALB/c mice, and Fluc activity (RLU/mg protein) was measured. The results are shown in Fig. 2. Fluc activity was confirmed in the liver and spleen of mice administered with any of the lipid nanoparticles. From this result, it was confirmed that the lipid nanoparticles using TOT-5 as a constituent lipid are useful as gene expression carriers targeting the liver and spleen in all the compositions used this time. Among them, Fluc mRNA-loaded lipid nanoparticles having a composition ratio of constituent lipids of TOT-5/DOPE/chol/PEG-DMG=60/15/25/1 showed high Fluc activity in the liver and not so high Fluc activity in the spleen, indicating that they are excellent as liver-selective gene expression carriers.

[Example 12]

Lipid nanoparticles were prepared using TOT-5 synthesized in Example 5 as a constituent lipid.

PE, cholesterol, and PEG-DMG were used as lipids other than TOT-5. DOPE or POPE was used as PE.

Specifically, in the same manner as in Example 11, TOT-5, PE, cholesterol, and PEG-DMG were used in a composition of TOT-5/PE/chol/PEG-DMG=60/15/25/1 (mol%) to prepare Fluc mRNA-loaded lipid nanoparticles by the alcohol dilution method.

Next, each of the prepared Fluc mRNA-loaded lipid nanoparticles was intravenously administered into the tail

vein of BALB/c mice, and Fluc activity (RLU/mg protein) was measured. The results are shown in Fig. 3. Fluc activity was confirmed in the liver and spleen of mice administered with any of the lipid nanoparticles, but the Fluc mRNA-loaded lipid nanoparticles containing DOPE showed higher Fluc activity than those of the Fluc mRNA-loaded lipid nanoparticles containing POPE, indicating that they are superior as gene expression carriers.

[Example 13]

Lipid nanoparticles were prepared using TOT-4 to TOT-8 synthesized in Examples 4 to 8 as constituent lipids.

DOPE, cholesterol, and PEG-DMG were used as lipids other than TOT.

Specifically, in the same manner as in Example 11, any one of TOT-4 to TOT-8, DOPE, cholesterol, and PEG-DMG were used in a composition of TOT/DOPE/chol/PEG-DMG=60/15/25/1.0 (mol%) to prepare Fluc mRNA-loaded lipid nanoparticles by the alcohol dilution method.

Next, each of the prepared Fluc mRNA-loaded lipid nanoparticles was intravenously administered into the tail vein of BALB/c mice, and Fluc activity (RLU/mg protein) was measured. The results are shown in Fig. 4. Since Fluc activity was confirmed in the liver and spleen of mice administered with any of the lipid nanoparticles, it was confirmed that the lipid nanoparticles using TOT-4, TOT-6 to TOT-8 as constituent lipids are also useful as gene expression carriers targeting the liver and spleen, similar to the lipid nanoparticles using TOT-5 as a constituent lipid. Among them, the Fluc mRNA-loaded lipid nanoparticles containing TOT-4 or TOT-5 showed very high Fluc activity, indicating that they are excellent as gene expression carriers. Meanwhile, the Fluc mRNA-loaded lipid nanoparticles containing TOT-6 to TOT-8 showed higher Fluc activity in the spleen than in the liver, indicating that the lipid nanoparticles containing TOT-6 to TOT-8 have high spleen selectivity and are useful as gene expression carriers targeting the spleen.

[Example 14]

Lipid nanoparticles were prepared using TOT-5 synthesized in Example 5 as a constituent lipid.

Phosphatidylcholine (PC), cholesterol, and PEG-DMG were used as lipids other than TOT-5. DSPC was used as PC.

Specifically, TOT-5, DSPC, cholesterol, and PEG-DMG were used in a composition of TOT-5/DSPC/chol/PEG-DMG=55 to 70/5 to 30/10 to 35/1 (mol%) to prepare Fluc mRNA-loaded lipid nanoparticles by the alcohol dilution method.

Next, each of the prepared Fluc mRNA-loaded lipid nanoparticles was intravenously administered into the tail vein of BALB/c mice, and Fluc activity (RLU/mg protein) was measured. The results are shown in Fig. 5. Fluc activity was confirmed in the liver and spleen of mice administered with any of the lipid nanoparticles. From this result, it was confirmed that the lipid nanoparticles using TOT-5 as a constituent lipid are useful as gene expression carriers targeting the liver and spleen in all the compositions used this time. Among them, Fluc mRNA-loaded lipid nanoparticles having a composition ratio of constituent lipids of TOT-5/DSPC/chol/PEG-DMG=65/15/20/1 showed high Fluc activity in the spleen and not so high Fluc activity in the liver, indicating that they are excellent as spleen-selective gene expression carriers. Further, Fluc mRNA-loaded lipid nanoparticles having a composition ratio of constituent lipids of TOT-5/DSPC/chol/PEG-DMG=65/5/30/1 showed very high Fluc activity in the liver, indicating that they are excellent as liver-selective gene expression carriers.

[Example 15]

Lipid nanoparticles were prepared using TOT-4 synthesized in Example 4 as a constituent lipid.

PC, cholesterol, and PEG-DMG were used as lipids other than TOT-4. DSPC, DPPC, POPC, or DOPC was used as PC.

Specifically, TOT-4, PC, cholesterol, and PEG-DMG were used in a composition of TOT-4/PC/chol/PEG-DMG=65/15/20/1 (mol%) to prepare Fluc mRNA-loaded lipid nanoparticles by the alcohol dilution method.

Next, each of the prepared Fluc mRNA-loaded lipid nanoparticles was intravenously administered into the tail vein of BALB/c mice, and Fluc activity (RLU/mg protein) was measured. The results are shown in Fig. 6. Fluc activity was confirmed in the liver and spleen of mice administered with any of the lipid nanoparticles. From this result, it was confirmed that the lipid nanoparticles using TOT-4 as a constituent lipid are useful as gene expression carriers targeting the liver and spleen in all the compositions used this time. Among them, the Fluc mRNA-loaded lipid nanoparticles containing DSPC and the Fluc mRNA-loaded lipid nanoparticles containing DPPC both showed higher Fluc activity in the spleen than in the liver, indicating that they are excellent as spleen-selective gene expression carriers. In particular, since the Fluc mRNA-loaded lipid nanoparticles containing DSPC showed relatively suppressed Fluc activity in the liver, it was suggested that DSPC is optimal as a neutral lipid for the lipid nanoparticles containing TOT-4 as a spleen-selective gene expression carrier.

[Example 16]

Lipid nanoparticles were prepared using TOT-1 to TOT-8, TOT-16, and TOT-17 synthesized in Examples 1 to 10 as constituent lipids.
DSPC, cholesterol, and PEG-DMG were used as lipids other than TOT.
Specifically, TOT, DSPC, cholesterol, and PEG-DMG were used in a composition of TOT/DSPC/chol/PEG-DMG=65/15/20/1.5 (mol%) to prepare Fluc mRNA-loaded lipid nanoparticles by the alcohol dilution method. When two kinds of TOT were used, the total amount was adjusted to 65 mol% by using an equal amount of each.
Next, each of the prepared Fluc mRNA-loaded lipid nanoparticles was intravenously administered into the tail vein of BALB/c mice, and Fluc activity (RLU/mg protein) was measured. The results are shown in Fig. 7. In the figure, "TOT-1,5" shows the results of Fluc mRNA-loaded lipid nanoparticles produced using equal amounts of TOT-1 and TOT-5 as TOT. Since Fluc activity was confirmed in the spleen of mice administered with any of the lipid nanoparticles, it was confirmed that the lipid nanoparticles using TOT-1 to TOT-4, TOT-6 to TOT-8, TOT-16, TOT-17, and a mixture of TOT-1 and TOT-5 as constituent lipids are also useful as gene expression carriers targeting the spleen, similar to the lipid nanoparticles using TOT-5 as a constituent lipid. Among them, the Fluc mRNA-loaded lipid nanoparticles containing TOT-4 showed very high Fluc activity in the spleen, indicating that they are excellent as spleen-selective gene expression carriers. Further, since Fluc activity was confirmed in the liver in all the mice except for those administered with Fluc mRNA-loaded lipid nanoparticles using TOT-2 as a constituent lipid, it was also confirmed that the lipid nanoparticles using TOT-1, TOT-3 to TOT-8, TOT-16, TOT-17, and a mixture of TOT-1 and TOT-5 as constituent lipids are useful as gene expression carriers targeting the liver.

[Example 17]

The gene expression ability in the spleen was compared between Fluc mRNA-loaded lipid nanoparticles containing TOT-4, which showed the best spleen selectivity in Example 16, and Fluc mRNA-loaded lipid nanoparticles containing RNA-LPX (manufactured by BioNTech), which is a commercially available highly spleen-selective lipid.
The lipid composition (molar ratio) of RNA-LPX is DOTMA/DOPE=50/50 (mol%). Fluc mRNA-loaded lipid nanoparticles using RNA-LPX were prepared in the same manner as the Fluc mRNA-loaded lipid nanoparticles containing TOT-4 in Example 16, except that RNA-LPX was used as a constituent lipid.
Next, each of the prepared Fluc mRNA-loaded lipid nanoparticles was intravenously administered into the tail vein of BALB/c mice, and Fluc activity (RLU/mg protein) was measured. The results are shown in Fig. 8. As a result, the Fluc mRNA-loaded lipid nanoparticles containing TOT-4 showed 24 times higher gene expression in the spleen than the Fluc mRNA-loaded lipid nanoparticles using RNA-LPX.

[Example 18]

The gene expression activity of lipid nanoparticles using TOT-4 as a pH-sensitive cationic lipid and lipid nanoparticles using CL4F6 in each tissue was compared and examined.
DSPC, cholesterol, and PEG-DMG were used as lipids other than the pH-sensitive cationic lipid.
Specifically, TOT-4, DSPC, cholesterol, and PEG-DMG were used in a composition of TOT-4/DSPC/chol/PEG-DMG=65/15/20/1.5 (mol%) to prepare TOT-4-containing Fluc mRNA-loaded lipid nanoparticles by the alcohol dilution method. Further, CL4F6, DSPC, cholesterol, and PEG-DMG were used in a composition of CL4F6/DSPC/chol/PEG-DMG=50/10/40/1.5 (mol%) to prepare CL4F6-containing Fluc mRNA-loaded lipid nanoparticles by the alcohol dilution method.
Next, each of the prepared Fluc mRNA-loaded lipid nanoparticles was intravenously administered into the tail vein of BALB/c mice, and Fluc activity (RLU/mg protein) was measured. The results are shown in Fig. 9. In the figure, "NT" shows the results of mice not administered with lipid nanoparticles, "F" shows the results of mice administered with CL4F6-containing Fluc mRNA-loaded lipid nanoparticles, and "PC" shows the results of mice administered with TOT-4-containing Fluc mRNA-loaded lipid nanoparticles. As a result, the TOT-4-containing Fluc mRNA-loaded lipid nanoparticles showed high gene expression in the spleen and liver, similar to the CL4F6-containing Fluc mRNA-loaded lipid nanoparticles.

[Example 19]

The gene expression activity of lipid nanoparticles using TOT-4 as a pH-sensitive cationic lipid and lipid nanoparticles using CL4F6 in each tissue was compared and examined.
Specifically, in the same manner as in Example 18, TOT-4-containing Fluc mRNA-loaded lipid nanoparticles having a composition of TOT-4/DSPC/chol/PEG-DMG=65/15/20/1.5 (mol%) and CL4F6-containing Fluc mRNA-loaded lipid nanoparticles having a composition of CL4F6/DSPC/chol/PEG-DMG=50/10/40/1.5 (mol%) were prepared. Table 1

shows the ζ-average particle size (nm), number average particle size (nm), polydispersity index (PdI), and encapsulation rate (%) of each Fluc mRNA-loaded lipid nanoparticle.

Table 1

| Cationic Lipid | ζ-Average Particle Size [nm] | Number Average Particle Size [nm] | PdI | Encapsulation Rate [%] |
|---|---|---|---|---|
| CL4F6 | 90.7 | 59.4 | 99.0 | 0.170 |
| TOT-4 | 134.3 | 33.5 | 83.5 | 0.157 |

Next, each of the prepared Fluc mRNA-loaded lipid nanoparticles was administered to BALB/c mice, and Fluc activity (RLU/mg protein) in each tissue was measured (N=3).

Specifically, first, each lipid nanoparticle was intravenously administered into the tail vein of mice in an amount of 0.2 mg/kg of Fluc mRNA, and 6 hours after administration of the lipid nanoparticles, about 50 mg each of the liver, spleen, lung, heart, brain, and muscle was collected and stored frozen. 500 μL of passive lysis buffer (manufactured by Promega) was added to each tube containing the collected organ. The liver was homogenized at 4000 rpm for 20 seconds twice, the spleen, kidney, lung, and heart were homogenized at 5000 rpm for 20 seconds twice, and the muscle was homogenized at 5000 rpm for 20 seconds four times; then, the homogenate was centrifuged at 13000 rpm at 4°C for 10 minutes, and the supernatant was collected as a sample for measurement. The liver and spleen were diluted 100 times with passive lysis buffer. 50 μL of a measurement sample (tissue supernatant) was mixed with 50 μL of luciferase assay buffer of the Luciferase Assay System (E1500, manufactured by Promega) previously added in a test tube by pipetting, and the amount of luminescence was measured with a luminometer. Protein concentration was determined using a commercially available assay kit (BCA Protein Assay Kit, manufactured by Pierce). Fluc activity was expressed as relative light units (RLU) per mg of protein.

Fig. 10 shows the measurement results of the amount of luciferase expression (Fluc activity) in each tissue of mice administered with each Fluc mRNA-loaded lipid nanoparticle. Mice administered with TOT-4-containing Fluc mRNA-loaded lipid nanoparticles showed higher luciferase activity in the spleen, lung, brain, heart, and muscle than mice administered with CL4F6-containing Fluc mRNA-loaded lipid nanoparticles. These results suggested that the lipid nanoparticles using TOT-4 as a constituent lipid serve as gene expression carriers that can be transported to various tissues.

[Example 20]

Delivery of lipid nanoparticles using TOT-4 as a pH-sensitive cationic lipid to each tissue was examined.

DOPE or DSPC, cholesterol, and PEG-DMG were used as lipids other than TOT-4.

Specifically, in the same manner as in Example 19, TOT-4, DSPC, cholesterol, and PEG-DMG were used in a composition of TOT-4/DSPC/chol/PEG-DMG=65/15/20/1.5 (mol%) to prepare DSPC-containing Fluc mRNA-loaded lipid nanoparticles. Further, TOT-4, DOPE, cholesterol, and PEG-DMG were used in a composition of TOT-4/DOPE/chol/-PEG-DMG=60/15/25/1.5 (mol%) to prepare DOPE-containing Fluc mRNA-loaded lipid nanoparticles.

Next, each of the prepared Fluc mRNA-loaded lipid nanoparticles was intravenously administered into the tail vein of BALB/c mice so that the dose of Fluc mRNA per 1 kg body weight was 0.01 mg, and 6 hours after administration, luciferase expression was evaluated by bioluminescence imaging of live animals. Specifically, mice were anesthetized with isoflurane, and then 100 μL of a substrate solution (a solution of d-luciferin dissolved in PBS at a concentration of 30 mg/mL) was intraperitoneally injected. The anesthetized mice were placed on a black sheet, and the intensity of luciferase-derived luminescence of the whole body was measured. The intensity of luciferase-derived luminescence in each organ was measured by imaging using an imaging system (IVIS Lumina system, manufactured by Perkin Elmer). The images were processed with "Living Image software v.4.3" (64-bit, manufactured by Caliper Life Sciences).

Fig. 11 shows imaging results measuring luciferase-derived luminescence intensity of the whole body of mice. In mice administered with DOPE-containing Fluc mRNA-loaded lipid nanoparticles, luciferase expression was mainly observed in the liver, whereas in mice administered with DSPC-containing Fluc mRNA-loaded lipid nanoparticles, expression was particularly observed in the spleen. In mice administered with DSPC-containing Fluc mRNA-loaded lipid nanoparticles, expression was also observed in the heel, hand, nose, neck, and the like. These results suggested the possibility that expression was observed at the site where blood vessels gather due to delivery to blood vessels or delivery to immune cells.

[Example 21]

The usefulness of lipid nanoparticles using TOT-5 as a pH-sensitive cationic lipid as a gene expression carrier for mRNA having an antitumor effect was examined.
DSPC, cholesterol, and PEG-DMG were used as lipids other than TOT-5.
Ova mRNA was used as mRNA having an antitumor effect, and EL4 cells (E.G.7-OVA cells), a mouse lymphoma cell line stably expressing OVA antigen, were used as tumor cells.
Specifically, first, in the same manner as in Example 19, TOT-5, DSPC, cholesterol, and PEG-DMG were used in a composition of TOT-5/DSPC/chol/PEG-DMG=65/15/20/1.5 (mol%) to prepare Ova mRNA-loaded lipid nanoparticles encapsulating Ova mRNA.
$5\times10^5$ E.G.7-OVA cells were subcutaneously injected into the flank region of C57BL/6N mice (female, 6 weeks old) per mouse for inoculation. Then, the tumor volume of each mouse was measured over time.
$$[\text{Tumor volume } (\text{mm}^3)]=[\text{Major axis of tumor } (\text{mm})]\times[\text{Minor axis of tumor } (\text{mm})]^2)\times0.52$$

On days 7 and 10 after tumor cell inoculation, Ova mRNA-loaded lipid nanoparticles suspended with PBS were intravenously administered into the tail vein at 0, 0.005, 0.01, 0.05, 0.1, or 0.5 mg RNA/kg body weight.
The results of monitoring tumor volume are shown in Fig. 12. On day 18 after administration of the Ova mRNA-loaded lipid nanoparticles, an antitumor effect was observed at a dose of 0.1 mg RNA/kg or more.

[Example 22]

TOT-5 was synthesized using N-(1,3-dihydroxy-2-(hydroxymethyl)propan-2-yl)-3-(piperidin-1-yl)propanamide (compound 3).

(1) Synthesis of N-(1,3-dihydroxy-2-(hydroxymethyl)propan-2-yl)-3-(piperidin-1-yl)propanamide (compound 3)

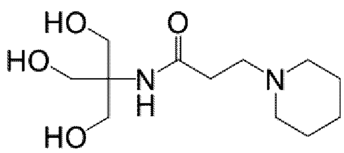

Piperidine (1.98 mL, 20.0 mmol) was added to N-[tris(hydroxymethyl)methyl]acrylamide (93%, 1.884 g, 10.0 mmol) dissolved in methanol (10 mL). The reaction mixture was stirred at ambient temperature overnight and then evaporated. This gave compound 3 (2.72 g, yield 97%) as a white powder. The obtained crude product was used for the next reaction without further purification.

(2) Synthesis of TOT-5

Oleic acid (890 mg, 3.15 mmol), DMAP (12.2 mg, 0.1 mmol), and EDCI (633 mg, 3.3 mmol) were added to a solution of compound 3 (260 mg, 1.0 mmol) dissolved in anhydrous DCM (10 mL). The reaction mixture was stirred at 40°C overnight and then evaporated. The resulting residue was suspended with EtOAc, passed through silica gel, and then the filtrate was washed with 0.1 N aqueous NaOH solution (5 mL) and brine and then dried over anhydrous $Na_2SO_4$. The obtained residue was loaded on a normal-phase column (Sfär Silica HCD, manufactured by Biotage) and purified by flash chromatography with a gradient mobile phase of hexane and AcOEt containing 0.5% TEA. This gave TOT-5 (799 mg, yield 76%) as a colorless oil.

[Example 23]

TOT-4 was synthesized using 3-(diethylamino)-N-(1,3-dihydroxy-2-(hydroxymethyl)propan-2-yl)propanamide (compound 4).

(1) Synthesis of 3-(diethylamino)-N-(1,3-dihydroxy-2-(hydroxymethyl)propan-2-yl)propanamide (compound 4)

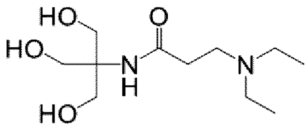

Diethylamine (6.27 mL, 60.0 mmol) was added to N-[tris(hydroxymethyl)methyl]acrylamide (93%, 1.884 g, 10.0 mmol). The reaction mixture was stirred at 40°C overnight. The obtained residue was loaded on a normal-phase column (Sfär Silica HCD, manufactured by Biotage) and purified by flash chromatography with a gradient mobile phase of DCM and methanol. This gave compound 4 (2.01 g, yield 81%) as a pale yellow solid.

(2) Synthesis of TOT-4

Oleic acid (478 mg, 1.55 mmol), DMAP (12.2 mg, 0.1 mmol), and EDCI (307 mg, 1.60 mmol) were added to a solution of compound 4 (124 mg, 0.50 mmol) dissolved in anhydrous DCM (5 mL). The reaction mixture was stirred at 40°C overnight and then evaporated. The resulting residue was suspended with EtOAc, passed through silica gel, and then the filtrate was washed with 0.1 N aqueous NaOH solution (5 mL) and brine, and then dried over anhydrous $Na_2SO_4$. The obtained residue was loaded on an amino column (Sfär Amino D, manufactured by Biotage) and purified by flash chromatography with a gradient mobile phase of hexane and AcOEt. This gave TOT-4 (235 mg, yield 45%) as a colorless oil.

[Example 24]

1-Ethylpiperidine-4-carboxylic acid was condensed to the amino group in TOT-0 synthesized in Example 1 to synthesize 2-(1-ethylpiperidine-4-carboxamide)-2-((oleoyloxy)methyl)propane-1,3-diyl dioleate (TOT-18).

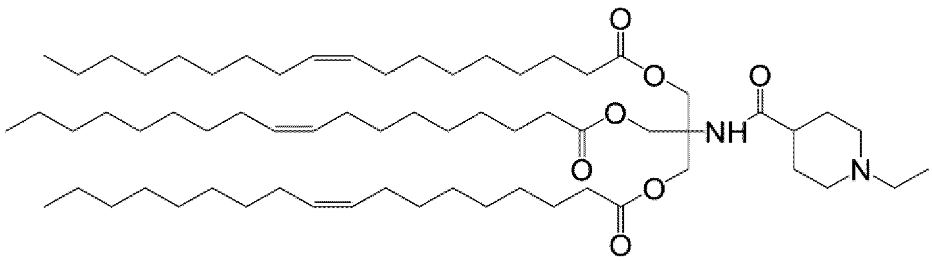

TOT-18

TOT-18 (37.4 mg, yield 8.9%) was obtained as a colorless oil in the same manner as TOT-17, except that 1-ethylpiperidine-4-carboxylic acid (78.6 mg, 0.50 mmol) was used instead of 3-(pyrrolidin-1-yl)propanoic acid.

[Example 25]

1-(Propan-2-yl)piperidine-4-carboxylic acid was condensed to the amino group in TOT-0 synthesized in Example 1 to synthesize 2-(1-isopropylpiperidine-4-carboxamide)-2-((oleoyloxy)methyl)propane-1,3-diyl dioleate (TOT-19).

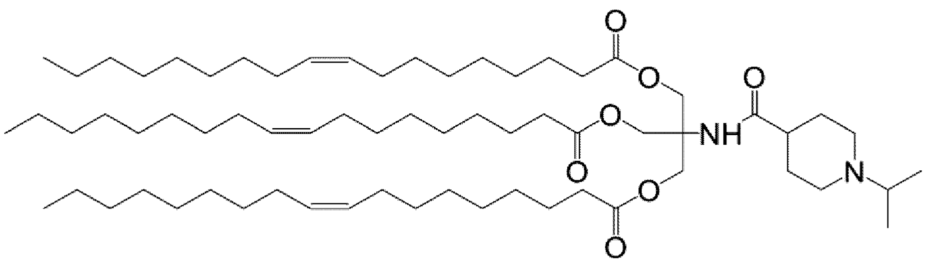

TOT-19

TOT-19 (108.4 mg, yield 25.4%) was obtained as a colorless oil in the same manner as TOT-17, except that

1-(propan-2-yl)piperidine-4-carboxylic acid (103.9 mg, 0.50 mmol) was used instead of 3-(pyrrolidin-1-yl)propanoic acid.

[Example 26]

3-(Pyrrolidin-1-yl)propanoic acid hydrochloride was condensed to the amino group in TOT-0 synthesized in Example 1 to synthesize 2-(1-methylpyrrolidine-3-carboxamide)-2-((oleoyloxy)methyl)propane-1,3-diyl dioleate (TOT-20).

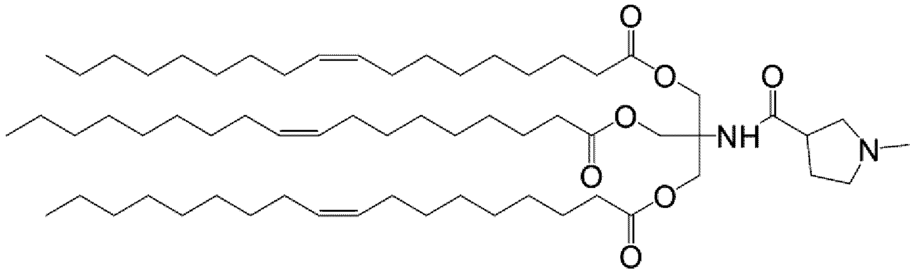

TOT-20

TOT-20 (116.6 mg, yield 28.4%) was obtained as a colorless oil in the same manner as TOT-17, except that 3-(pyrrolidin-1-yl)propanoic acid hydrochloride (89.8 mg, 0.50 mmol) was used instead of 3-(pyrrolidin-1-yl)propanoic acid.

[Example 27]

N-[Tris(hydroxymethyl)methyl]acrylamide, Nω-methyltryptamine, and oleic acid were condensed to synthesize 2-(3-((2-(1H-indol-3-yl)ethyl)(methyl)amino)propanamido)-2-((oleoyloxy)methyl)propane-1,3-diyl dioleate (TOT-21).

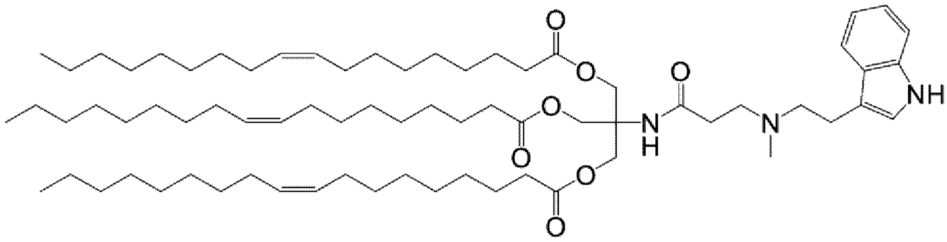

TOT-21

N-[Tris(hydroxymethyl)methyl]acrylamide (93%, 188.4 mg, 1.0 mmol) and Nω-methyltryptamine were dissolved in EtOH (1 mL) and water (0.4 mL). The resulting reaction mixture was stirred at ambient temperature overnight in dark and then evaporated. The obtained crude product was dissolved in anhydrous DCM (5 mL), and oleic acid (890 mg, 3.15 mmol), DMAP (12.2 mg, 0.1 mmol), and EDCI-HCl (633 mg, 3.30 mmol) were added. The reaction mixture was stirred at ambient temperature overnight in dark. The resulting residue was suspended with EtOAc (20 mL), washed with 0.1 N aqueous NaOH solution (20 mL), water (20 mL), and brine (20 mL), and then dried over anhydrous $Na_2SO_4$. The resulting solution was filtered, and the filtrate was evaporated. The residue was loaded on a normal-phase column (Rening, manufactured by Biotage) and purified by flash chromatography with a gradient mobile phase of hexane containing 0.5% TEA and AcOEt containing 0.5% TEA. This gave TOT-21 (993.3 mg, yield 87%) as a colorless oil.

[Example 28]

N-[Tris(hydroxymethyl)methyl]acrylamide, isonipecotamide, and oleic acid were condensed to synthesize 2-(3-(4-carbamoylpiperidin-1-yl)propanamido)-2-((oleoyloxy)methyl)propane-1,3-diyl dioleate (TOT-22).

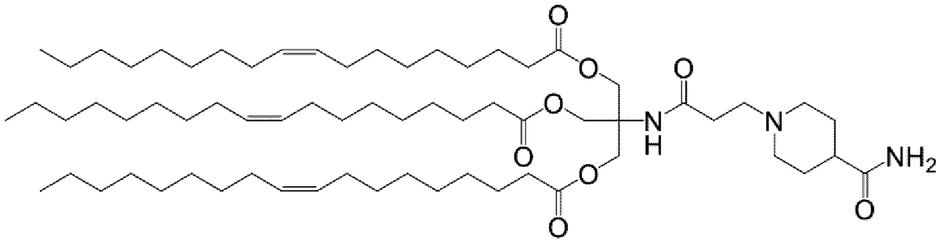

TOT-22

Isonipecotamide (38.5 mg, 0.3 mmol) dissolved in 2-propanol (600 μL) was added to N-[tris(hydroxymethyl) methyl]acrylamide (93%, 52.6 mg, 0.3 mmol) dissolved in water. The resulting reaction mixture was stirred at ambient temperature overnight, and then the solvent was removed under reduced pressure. The obtained crude product was dissolved in anhydrous DCM (3 mL), and oleic acid (282 mg, 1.0 mmol), DMAP (12.2 mg, 0.1 mmol), and EDCI-HCl (211 mg, 1.10 mmol) were added thereto. The reaction mixture was stirred at 40°C overnight. The resulting reaction mixture was diluted with hexane, passed through a celite pad, and then the filtrate was evaporated. The residue was loaded on a normal-phase column (Rening, manufactured by Biotage) and purified by flash chromatography with a gradient mobile phase of hexane containing 0.5% TEA and AcOEt containing 0.5% TEA. This gave TOT-22 (171.5 mg, yield 52%) as a colorless oil.

[Example 29]

N-[Tris(hydroxymethyl)methyl]acrylamide, N-methyl-2-pyridinemethanamine, and oleic acid were condensed to synthesize 2-(3-(methyl(pyridin-2-ylmethyl)amino)propanamido)-2-((oleoyloxy)methyl)propane-1,3-diyl dioleate (TOT-23).

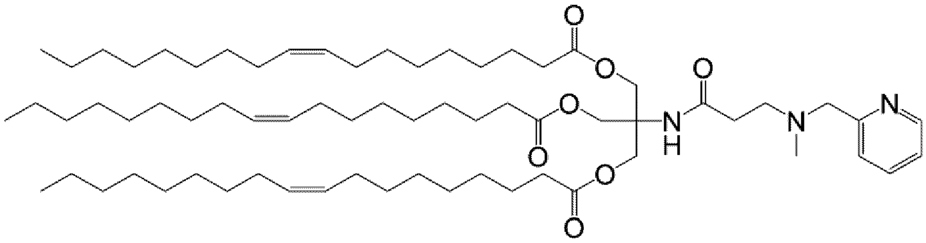

TOT-23

TOT-23 (215 mg, yield 65.7%) was obtained as a pale yellow oil in the same manner as TOT-22, except that N-methyl-2-pyridinemethanamine (36.7 mg, 0.3 mmol) was used instead of isonipecotamide.

[Example 30]

N-[Tris(hydroxymethyl)methyl]acrylamide, 1'H-spiro[piperidine-4,4'-quinazolin]-2'(3'H)-one, and oleic acid were condensed to synthesize 2-((oleoyloxy)methyl)-2-(3-(2'-oxo-2',3'-dihydro-1'H-spiro[piperidine-4,4'-quinazolin]-1-yl)propanamido)propane-1,3-diyl dioleate (TOT-24).

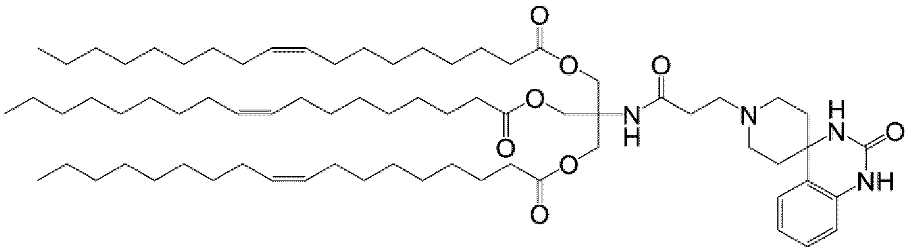

TOT-24

TOT-24 (155.8 mg, yield 43.8%) was obtained as a colorless oil in the same manner as TOT-22, except that 1'H-spiro[piperidine-4,4'-quinazolin]-2'(3'H)-one (65.2 mg, 0.3 mmol) was used instead of isonipecotamide.

[Example 31]

N-[Tris(hydroxymethyl)methyl]acrylamide, 3-(4-piperidinyl)-1,3-benzoxazol-2(3H)-one, and oleic acid were

condensed to synthesize 2-((oleoyloxy)methyl)-2-(3-(4-(2-oxobenzo[d]oxazol-3(2H)-yl)piperidin-1-yl)propanamido)propane-1,3-diyl dioleate (TOT-25).

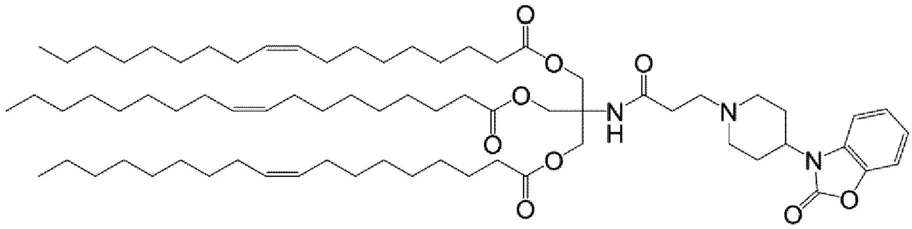

TOT-25

TOT-25 (116.2 mg, yield 32.6%) was obtained as a yellow oil in the same manner as TOT-22, except that 3-(4-piperidinyl)-1,3-benzoxazol-2(3H)-one (65.5 mg, 0.3 mmol) was used instead of isonipecotamide.

[Example 32]

N-[Tris(hydroxymethyl)methyl]acrylamide, N-methyl-4-pyridylmethylamine, and oleic acid were condensed to synthesize 2-(3-(methyl(pyridin-4-ylmethyl)amino)propanamido)-2-((oleoyloxy)methyl)propane-1,3-diyl dioleate (TOT-26).

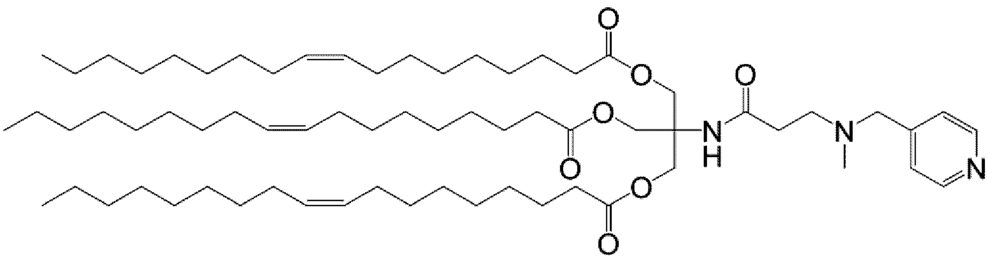

TOT-26

TOT-26 (54 mg, yield 16.5%) was obtained as a colorless oil in the same manner as TOT-22, except that N-methyl-4-pyridylmethylamine (36.7 mg, 0.3 mmol) was used instead of isonipecotamide.

[Example 33]

Lipid nanoparticles were prepared using the pH-sensitive cationic lipids TOT-1 to 8 and 16 to 20 synthesized in Examples 1 to 10 and 24 to 26 (hereinafter sometimes collectively referred to as "TOT") as constituent lipids.

DSPC, cholesterol, and PEG-DMG were used as lipids other than TOT.

Specifically, various TOTs, DSPC, cholesterol, and PEG-DMG were used in a composition of TOT/DSPC/chol/-PEG-DMG=65/15/20/1.5 (mol%) to prepare Fluc mRNA-loaded lipid nanoparticles by the alcohol dilution method. Table 2 shows the ζ-average particle size (nm), polydispersity index (PdI), pKa, and encapsulation rate (%) of each Fluc mRNA-loaded lipid nanoparticle.

Table 2

| TOT | ζ-Average Particle Size [nm] | PdI | pKa | Encapsulation Rate [%] |
|---|---|---|---|---|
| TOT-5 | 102.1 | 0.161 | 6.2 | 88.6 |
| TOT-16 | 99.2 | 0.180 | 6.6 | 87.6 |
| TOT-4 | 134.3 | 0.157 | 6.6 | 83.5 |
| TOT-1 | 138.0 | 0.324 | 7.0 | 85.9 |
| TOT-20 | 201.8 | 0.263 | 7.4 | 94.7 |
| TOT-18 | 130.0 | 0.319 | 7.5 | 92.2 |
| TOT-19 | 142.6 | 0.261 | 7.5 | 92.2 |
| TOT-2 | 166.2 | 0.347 | <7.5 | 92.3 |
| TOT-3 | 167.2 | 0.344 | <7.5 | 92.3 |

(continued)

| TOT | ζ-Average Particle Size [nm] | PdI | pKa | Encapsulation Rate [%] |
|---|---|---|---|---|
| TOT-6 | 102.5 | 0.190 | - | 95.4 |
| TOT-7 | 128.0 | 0.232 | - | 92.4 |
| TOT-8 | 87.1 | 0.213 | - | 85.7 |

As shown in Table 2, all the lipid nanoparticles had a ζ-average particle size of about 100 to about 200 nm and an encapsulation rate of about 90%, and all had sufficiently suitable physical properties as gene expression carriers. Further, it was confirmed that the pKa of the lipid nanoparticles is affected by the TOT used as a constituent lipid, and by appropriately selecting TOT, lipid nanoparticles having a desired pKa can be prepared within a very wide range of 6.2 to 7.7 or more.

Next, each of the prepared Fluc mRNA-loaded lipid nanoparticles was intravenously administered into the tail vein of BALB/c mice, and Fluc activity (RLU/mg protein) in each tissue was measured. As a control, Fluc mRNA-loaded lipid nanoparticles prepared using CL4F6 instead of TOT in a composition of CL4F6/DSPC/chol/PEG-DMG=50/10/40/1.5 (mol%) were also administered to mice in the same manner, and Fluc activity in each tissue was measured. Fluc activity in each tissue was measured in the same manner as in Example 19.

The measurement results of the amount of luciferase expression in each tissue of mice administered with each Fluc mRNA-loaded lipid nanoparticle are shown in Figs. 13 to 18. In the liver, the luciferase activity was lower in all the mice administered with the lipid nanoparticles containing TOT than in the control mice (mice administered with CL4F6-containing lipid nanoparticles) (Fig. 13). In the spleen, the luciferase activity was higher in the mice administered with the lipid nanoparticles containing TOT-5, TOT-16, or TOT-4 than in the control mice, and it was found that spleen selectivity can be enhanced by using TOT having a pKa of about 6.2 to about 6.6 as a constituent lipid (Fig. 14). In the lung, the luciferase activity was highest in the mice administered with the lipid nanoparticles containing TOT-19, and it was higher in the mice administered with the lipid nanoparticles containing TOT-18, TOT-2, TOT-3, or TOT-4 than in the control mice (Fig. 15). Since TOT-18, TOT-19, TOT-2, and TOT-3 have a pKa of 7.5 or more, it was suggested that the lung selectivity of lipid nanoparticles can be enhanced by selecting TOT having a high pKa. In the brain, the luciferase activity was remarkably high in the mice administered with the lipid nanoparticles containing TOT-4, suggesting that a carrier that can be efficiently transported to the brain can be produced by using TOT-4 as a constituent lipid (Fig. 16). In the heart, the luciferase activity was very high in the mice administered with the lipid nanoparticles containing TOT-4 or TOT-19, and it was higher in the mice administered with the lipid nanoparticles containing TOT-16 or TOT-7 than in the control mice (Fig. 17). In the muscle, the luciferase activity was very high in the mice administered with the lipid nanoparticles containing TOT-18, TOT-19, or TOT-4, and it was higher in the mice administered with the lipid nanoparticles containing TOT-16 than in the control mice (Fig. 18).

[Example 34]

As a result of statistical analysis, it was predicted that the selectivity of the liver and spleen in the uptake of lipid nanoparticles into tissues is affected by the content ratio of DSPC in the total constituent lipids of the lipid nanoparticles. Therefore, lipid nanoparticles with different DSPC contents were prepared, and the uptake into the liver and spleen was examined.

Specifically, in the same manner as in Example 19, TOT-5, DSPC, cholesterol, and PEG-DMG were used in a composition of TOT-5/DSPC/chol/PEG-DMG=65/X/35-X/1.5 (mol%) (X=3, 5, 10, 15, or 20) to prepare TOT-5-containing Fluc mRNA-loaded lipid nanoparticles. Each of the prepared Fluc mRNA-loaded lipid nanoparticles was intravenously administered into the tail vein of BALB/c mice so that the dose of Fluc mRNA was 0.01 mg/kg, the liver and spleen were collected 6 hours after administration of the lipid nanoparticles, and Fluc activity (RLU/mg protein) was measured (N=3). Fluc activity in each tissue was measured in the same manner as in Example 19.

Further, Fluc mRNA-loaded lipid nanoparticles using SM-102, ALC-0315, or MC3 instead of TOT-5 as a cationic lipid were prepared in a composition of cationic lipid/DSPC/chol/PEG-DMG=50/X/50-X/1.5 (mol%) (X=5, 10, 15, or 20), intravenously administered into the tail vein of mice, and Fluc activity (RLU/mg protein) in the liver and spleen was measured in the same manner.

The measurement results of Fluc activity in the liver and spleen of each mouse are shown in Fig. 19. Fig. 19(A) shows the results of mice administered with TOT-5-containing Fluc mRNA-loaded lipid nanoparticles, Fig. 19(B) shows the results of mice administered with MC3-containing Fluc mRNA-loaded lipid nanoparticles, Fig. 19(C) shows the results of mice administered with SM-containing Fluc mRNA-loaded lipid nanoparticles, and Fig. 19(D) shows the results of mice administered with ALC-containing Fluc mRNA-loaded lipid nanoparticles. In any cationic lipid, as the DSPC content increased, the activity in the spleen tended to increase, and the activity in the liver tended to decrease. In particular, in mice

administered with TOT-5-containing Fluc mRNA-loaded lipid nanoparticles, when the DSPC content was less than 10 mol%, the activity was predominantly in the liver, and when the DSPC content was 10 mol% or more, the activity was predominantly in the spleen.

[Example 35]

The relationship between the DSPC content-dependent organ selectivity and the pKa of the lipid nanoparticles in the uptake of the lipid nanoparticles into organs was examined.

TOT-1 to 5 and 16 were used as pH-sensitive cationic lipids. DSPC, cholesterol, and PEG-DMG were used as lipids other than TOT.

Specifically, in the same manner as in Example 19, a composition of TOT/DSPC/chol/PEG-DMG=65/3/32/1.5 (mol%) was used to prepare Fluc mRNA-loaded lipid nanoparticles having a DSPC content of 3 mol% (DSPC-3-containing Fluc mRNA-loaded lipid nanoparticles). Further, a composition of TOT/DSPC/chol/PEG-DMG=65/15/20/1.5 (mol%) was used to prepare Fluc mRNA-loaded lipid nanoparticles having a DSPC content of 15 mol% (DSPC-15-containing Fluc mRNA-loaded lipid nanoparticles).

Each of the prepared Fluc mRNA-loaded lipid nanoparticles was intravenously administered into the tail vein of BALB/c mice so that the dose of Fluc mRNA was 0.01 mg/kg, the liver and spleen were collected 6 hours after administration of the lipid nanoparticles, and Fluc activity (RLU/mg protein) was measured. Fluc activity in each tissue was measured in the same manner as in Example 19.

For each Fluc mRNA-loaded lipid nanoparticle, the ratio of Fluc activity in the spleen to Fluc activity in the liver ([Fluc activity in spleen (RLU/mg protein)]/[Fluc activity in liver (RLU/mg protein)]) was calculated as "specificity". Fig. 20(A) shows a diagram plotting specificity on the vertical axis and pKa on the horizontal axis for each Fluc mRNA-loaded lipid nanoparticle. Further, Fig. 20(B) shows a diagram plotting the relationship of the difference (specificity change) between the specificity of DSPC-15-containing Fluc mRNA-loaded lipid nanoparticles and the specificity of DSPC-3-containing Fluc mRNA-loaded lipid nanoparticles with the pKa of the lipid nanoparticles in the Fluc mRNA-loaded lipid nanoparticles prepared using the same TOT.

As shown in Fig. 20(A), the lower the pKa of the lipid nanoparticles, the higher the Fluc activity in the liver when the DSPC content was 3 mol% and the higher the Fluc activity in the spleen when the DSPC content was 15 mol%. In particular, the influence of pKa on the spleen/liver ratio (specificity) of Fluc activity was large under the condition where the DSPC content was 3 mol%, and the Fluc activity was predominantly in the spleen in the lipid nanoparticles having a pKa of more than 7. It was shown that when the pKa of the lipid nanoparticles is low, the target property for the liver and spleen greatly changes by changing the DSPC content.

[Example 36]

It has been reported that endogenous apolipoprotein E (ApoE) is involved in the uptake of lipid nanoparticles into hepatocytes. Therefore, Fluc mRNA-loaded lipid nanoparticles having a DSPC content of 3 mol% or 15 mol% were administered to wild-type mice and ApoE-deficient mice, and the uptake into the liver and spleen was examined.

First, in the same manner as in Example 35, a composition of TOT-5/DSPC/chol/PEG-DMG=65/3/32/1.5 (mol%) was used to prepare Fluc mRNA-loaded lipid nanoparticles having a DSPC content of 3 mol%. Further, a composition of TOT-5/DSPC/chol/PEG-DMG=65/15/20/1.5 (mol%) was used to prepare Fluc mRNA-loaded lipid nanoparticles having a DSPC content of 15 mol%.

Each of the prepared Fluc mRNA-loaded lipid nanoparticles was intravenously administered into the tail vein of wild-type mice (BALB/c mice) or ApoE-deficient mice (all 4 weeks old, female) so that the dose of Fluc mRNA was 0.01 mg/kg, the liver and spleen were collected 6 hours after administration of the lipid nanoparticles, and Fluc activity (RLU/mg protein) was measured (N=3). Fluc activity in each tissue was measured in the same manner as in Example 19.

The measurement results are shown in Fig. 21. When Fluc mRNA-loaded lipid nanoparticles having a DSPC content of 3 mol% were administered, the Fluc activity in ApoE-deficient mice ("3-ApoE(-)" in the figure) was lower in the liver than that in wild-type mice ("3-WT" in the figure), while the activity in the spleen did not increase. When Fluc mRNA-loaded lipid nanoparticles having a DSPC content of 15 mol% were administered, the Fluc activity in ApoE-deficient mice ("15-ApoE(-)" in the figure) was similar to that in wild-type mice ("15-WT" in the figure), and the Fluc activity in the liver and spleen did not depend on ApoE.

[Example 37]

The spleen is composed of multiple types of cells, and its function varies depending on the cell type. Therefore, in the uptake of TOT-containing lipid nanoparticles into the spleen, it was examined which cell type in the spleen takes up the lipid nanoparticles. TOT-5, DSPC, cholesterol, and PEG-DMG were used as constituent lipids of the lipid nanoparticles.

First, DiD-modified Fluc mRNA-loaded lipid nanoparticles having a composition of TOT-5/DSPC/chol/PEG-DMG=65/X/35-X/1.5 (mol%) (X=3, 5, 10, 15, or 20) were prepared in the same manner as in Example 34, except that the red fluorescent substance DiD was incorporated at 0.5 mol% for DiD modification. The obtained DiD-modified Fluc mRNA-loaded lipid nanoparticles were intravenously administered into the tail vein of BALB/c mice so that the dose of Fluc mRNA was 0.2 mg/kg, the mice were euthanized 1 hour after administration, and the spleen was collected (N=3). As a control, the spleen was also collected from BALB/c mice not administered with lipid nanoparticles.

The spleen cells were stained to distinguish B cells, T cells, NK cells, dendritic cells, and neutrophils. The collected spleen was placed on a sterile petri dish and minced with scissors, an appropriate amount of cold HBSS(-) buffer was added to the sterile petri dish to loosen the tissue, the resulting suspension was passed through a 40 μm cell strainer, and centrifuged at 4°C and 400×g for 5 minutes. After removing the supernatant, erythrocytes were removed by adding 1 mL of RBC lysis buffer, diluted with 9 mL of cold HBSS(-) buffer, and then centrifuged at 4°C and 400×g for 5 minutes. After removing the supernatant, the cells were resuspended with 5 mL of cold HBSS(-) buffer, the number of cells was counted, the suspensions were added to a 1.5 mL tube so that $3\times10^6$ cells were added, and centrifuged at 4°C and 400×g for 5 minutes. After removing the supernatant, for blocking, purified anti-mouse CD16/32 antibody (10 μg/mL, prepared with FACS buffer) was added at 100 μL/$1\times10^6$ cells and reacted on ice for 10 minutes while lightly tapping every 5 minutes. The cells were stained with antibodies for B cells as $CD19^+CD3^-$ cells, for T cells as $CD19^-CD3^+$ cells, for NK cells as $CD3^-CD49b^+$ cells, for dendritic cells as $CD11c^+I\text{-}A/I\text{-}E^+$ cells, and for neutrophils as $Ly6G^+CD11b^+$ cells, and reacted on ice for 30 minutes while lightly tapping every 15 minutes. After the reaction, the volume was adjusted to 1 mL/tube with FACS buffer, centrifuged at 4°C and 400×g for 5 minutes, and washed twice with 1 mL of FACS buffer to obtain a sample. 6 μL of propidium iodide solution was added to the sample, each cell group was extracted by each surface antigen, and then DiD fluorescence intensity was measured.

The FACS buffer (1×PBS, 0.5% BSA, 0.1% $NaN_3$) was prepared by dissolving 10 PBS tablets in 1 L of deuterium-depleted water (DDW), autoclaving, and then adding 5.0 g of albumin (derived from bovine serum, fraction V, pH 7.0) and 1.0 g of sodium azide and dissolving them. The buffer was stored at 4°C until use.

DiD-positive cells (cells emitting DiD fluorescence) are cells that have taken up DiD-modified Fluc mRNA-loaded lipid nanoparticles. Therefore, the DiD fluorescence intensity of each spleen cell type was determined as the uptake amount of DiD-modified Fluc mRNA-loaded lipid nanoparticles. The measurement results are shown in Fig. 22. In the figure, "DC" shows the results of dendritic cells, and "neu" shows the results of neutrophils. As the DSPC content of the lipid nanoparticles increased, the number of cells in splenic B cells that took up DiD-modified Fluc mRNA-loaded lipid nanoparticles increased. Further, as shown in Fig. 22, the lipid nanoparticles containing TOT-5 were highly taken up by B cells and dendritic cells, and in particular, the uptake by B cells was improved by about 10 times by increasing the DSPC content of the lipid nanoparticles.

[Example 38]

For splenic B cells, which showed the highest uptake in Example 37, lipid nanoparticles with different DSPC contents were taken up, and the influence of Fluc activity and DSPC content was examined. The DiD-modified Fluc mRNA-loaded lipid nanoparticles prepared in Example 37 were used.

DiD-modified Fluc mRNA-loaded lipid nanoparticles were intravenously administered into the tail vein of BALB/c mice so that the dose of Fluc mRNA was 0.1 mg/kg, the mice were euthanized 6 hours after administration, and the spleen was collected (N=3). The collected spleen was placed on a sterile petri dish and minced with scissors, an appropriate amount of cold HBSS(-) buffer was added to the sterile petri dish to loosen the tissue, the resulting suspension was passed through a 40 μm cell strainer, and centrifuged at 4°C and 400×g for 5 minutes. After removing the supernatant, erythrocytes were removed by adding 1 mL of RBC lysis buffer, diluted with 9 mL of cold HBSS(-) buffer, and then centrifuged at 4°C and 400×g for 5 minutes. After removing the supernatant, the cells were resuspended with 5 mL of cold HBSS(-) buffer, the number of cells was counted, and centrifuged at 4°C and 400×g for 5 minutes. After removing the supernatant, the cells were suspended with MACS buffer (a solution of FACS buffer containing 2 mM EDTA) to $5\times10^7$ cells/mL/tube, centrifuged at 4°C and 400×g for 5 minutes, and the supernatant was removed. The precipitated cells were suspended in 450 μL of MACS buffer, and 50 μL of CD19 MicroBeads was added to obtain a cell suspension having a total volume of 500 μL. The entire amount of the cell suspension was added to an MS column, and after the suspension flowed out of the column, 500 μL of MACS buffer was added to the column three times. Then, the column was removed from the MACS separator, placed on a collection tube, 1 mL of MACS buffer was added, and a plunger was pushed to collect B cells.

Fluc activity (RLU/mg protein) of the collected B cells was measured in the same manner as in Example 19 (N=3). The measurement results are shown in Fig. 23. As the DSPC content in the lipid nanoparticles increased, the Fluc activity of splenic B cells was improved by about 20 times.

[Example 39]

It is known that the complement system is involved in the uptake of nanoparticles into immune cells, and in particular, complement receptor CD21/35 is known to be involved in nanoparticle uptake into B cells (Limei Shen, et al, Journal of Allergy, 2018, vol. 142, pp. 1558-1570; Monika Bednarczyk, et al, Molecular Sciences, 2021, vol. 22, p. 2869). Therefore, it was examined whether the complement system is involved in the uptake of TOT-containing lipid nanoparticles into B cells.

Among the DiD-modified Fluc mRNA-loaded lipid nanoparticles prepared in Example 37, those having a DSPC content of 3 mol% and 15 mol% were used.

The spleen was collected from euthanized mice in the same manner as in Example 38, and splenic B cells (primary B cells) were isolated and collected from the collected spleen. The number of cells was counted, $1\times10^7$ cells/tube were dispensed, and centrifuged at 4°C and 400×g for 5 minutes. After removing the supernatant, 1 ×passive lysis buffer was added at 100 μL/tube. Since a white aggregate was observed, the mixture was centrifuged at 4°C and 13000 rpm for 10 minutes, and the supernatant was collected as a sample for measurement. 50 μL of a measurement sample (tissue supernatant) was mixed with 50 μL of luciferase assay buffer of the Luciferase Assay System (E1500, manufactured by Promega) previously added to a test tube by pipetting, and the amount of luminescence was measured with a luminometer.

Inactivated serum was prepared as follows. Blood was collected from the heart of mice under anesthesia and allowed to stand at room temperature for 30 minutes. The blood clot was peeled off by tapping, and the mixture was centrifuged at room temperature and 1500×g for 15 minutes, and then the supernatant was collected as serum. Mouse serum heated at 56°C for 30 minutes was used as inactivated serum.

Primary B cells were resuspended with a basal medium to $2.5\times10^6$ cells/mL and stored on ice. In a 96-well plate, a solution was prepared by mixing 5 μL of DiD-modified Fluc mRNA-loaded lipid nanoparticles (8 ng RNA/μL), 5 μL of PBS, and 10 μL of mouse serum. As an EDTA addition group, a part of D-PBS(-) was replaced with 2 μL of a 0.5 M EDTA solution. Further, as an inactivated group, mouse serum was replaced with heat-treated one. The solution was allowed to stand at 37°C under 5% $CO_2$ for 1 hour, and then 80 μL of a cell suspension of $2.5\times10^6$ cells/mL was added to the well, and allowed to stand at 37°C under 5% $CO_2$ for 1 hour (final number of cells: $2\times10^5$ cells/well, final RNA amount: 40 ng/well, final serum concentration: 10%, final EDTA concentration: 10 mM). The entire amount of the solution in the well was collected, 400 μL of FACS buffer was added thereto, and DiD fluorescence intensity was measured with a cell sorter (CytoFLEX, manufactured by Beckman Coulter).

The measurement results of DiD fluorescence intensity in each primary B cell taken up with DiD-modified Fluc mRNA-loaded lipid nanoparticles are shown in Fig. 24(A). Further, Fig. 24(B) shows the results of FACS sorting by DiD uptake amount for primary B cells taken up by culturing DiD-modified Fluc mRNA-loaded lipid nanoparticles having a DSPC content of 15 mol% in the presence of non-inactivated serum under the condition of no EDTA. As shown in Fig. 24(A), the amount of DiD-modified Fluc mRNA-loaded lipid nanoparticles taken up into primary B cells was clearly lower in the presence of inactivated serum or in the presence of EDTA than under the condition of untreated serum and no EDTA. Further, under the condition of untreated serum and no EDTA, the DiD-modified Fluc mRNA-loaded lipid nanoparticles having a DSPC content of 15 mol% were taken up more than those having a DSPC content of 3 mol%, but in the presence of inactivated serum or in the presence of EDTA, the DiD-modified Fluc mRNA-loaded lipid nanoparticles having a DSPC content of 3 mol% were taken up more. These results suggested that any complement system may be involved in the uptake of TOT-containing lipid nanoparticles into splenic B cells.

[Example 40]

For splenic B cells, which showed the highest uptake in Example 37, it was examined which cell type in splenic B cells takes up the lipid nanoparticles.

Among the DiD-modified Fluc mRNA-loaded lipid nanoparticles prepared in Example 37, those having a DSPC content of 3 mol% and 15 mol% were used.

DiD-modified Fluc mRNA-loaded lipid nanoparticles were intravenously administered into the tail vein of BALB/c mice so that the dose of Fluc mRNA was 0.1 mg/kg, the mice were euthanized 1 hour after administration, and the spleen was collected (N=3). As a control, the spleen was also collected from BALB/c mice not administered with lipid nanoparticles.

Spleen cells were collected in the same manner as in Example 37 and blocked with purified anti-mouse CD16/32 antibody (10 μg/mL, prepared with FACS buffer). Then, the cells were stained with antibodies for B cells as $CD19^+$ cells, for MZB cells as $CD21/35^{high}CD23^{mid}$ cells, and for FOB cells as $CD21/35^{mid}CD23^{high}$ cells, and reacted on ice for 30 minutes while lightly tapping every 15 minutes. After the reaction, the volume was adjusted to 1 mL/tube with FACS buffer, centrifuged at 4°C and 400×g for 5 minutes, and washed twice with 1 mL of FACS buffer to obtain a sample. 6 μL of propidium iodide solution was added to the sample, each cell group was extracted by each surface antigen, and then DiD fluorescence intensity was measured.

Fig. 25 shows the results of measurement of DiD fluorescence intensity in B cells, MZB cells, and FOB cells of mice not administered with lipid nanoparticles (NT), mice administered with DiD-modified Fluc mRNA-loaded lipid nanoparticles having a DSPC content of 3 mol% (3% DSPC), and mice administered with DiD-modified Fluc mRNA-loaded lipid nanoparticles having a DSPC content of 15 mol% (15% DSPC). As shown in Fig. 25, the uptake of DiD-modified Fluc mRNA-loaded lipid nanoparticles containing TOT was clearly higher in MZB cells highly expressing CD21/35 than in FOB cells. Further, the uptake into MZB cells was higher in the lipid nanoparticles having a DSPC content of 15 mol% than in the lipid nanoparticles having a DSPC content of 3 mol%.

Fluc mRNA-loaded lipid nanoparticles having a composition of TOT-5/DSPC/chol/PEG-DMG=65/3/32/1.5 (mol%) (DSPC-3-containing Fluc mRNA-loaded lipid nanoparticles) prepared in Example 35 and Fluc mRNA-loaded lipid nanoparticles having a composition of TOT-5/DSPC/chol/PEG-DMG=65/15/20/1.5 (mol%) (DSPC-15-containing Fluc mRNA-loaded lipid nanoparticles) were incubated with untreated mouse serum prepared in Example 39, respectively, and then the proteins bound to each lipid nanoparticle were identified. As a result, fibrinogen, vitronectin, IgM, mannose-binding protein C (MBP), C1q, and the like were detected as proteins that bind more to lipid nanoparticles having a DSPC content of 15 mol% than to lipid nanoparticles having a DSPC content of 3 mol%.

MBP and C1q are proteins located upstream of the lectin pathway and classical pathway of complement, respectively, have similar structures to each other, and have been reported to be recognized by C1q receptor (Ehrnthaller, et al, Mol Med, 2010, vol. 17(3-4), pp. 317-329). IgM is recognized by the globular heads of C1q. It has been reported that fibrinogen, which is a blood coagulation-related factor, and vitronectin, which is a cell adhesion factor, become ligands for gC1q receptor when denatured (Ghebrehiwet, et al, Immunol Rev, 2002, vol. 180, pp. 65-77). Based on these findings, it is presumed that lipid nanoparticles having a relatively high DSPC content are taken up into spleen cells through the complement pathway, particularly, the pathway mediated by C1q receptor, and may be useful as drug delivery carriers to animals in which the pathway is activated.

## Claims

1. A pH-sensitive cationic lipid comprising a compound represented by the following formula (I):

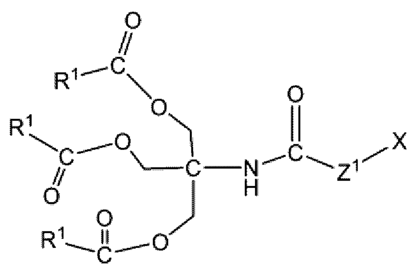

(I)

wherein, in formula (I), $R^1$ is a hydrocarbon group having 1 to 22 carbon atoms; three $R^1$ groups in one molecule may be the same as or different from each other; $Z^1$ is an alkylene group having 1 to 6 carbon atoms which may be substituted with one hydroxyalkyl group having 1 to 3 carbon atoms, or a single bond; X is $-N(R^2)(R^3)$ or a 5-to 7-membered nonaromatic heterocyclic group, wherein the group is bonded to $Z^1$ via a carbon atom, and the ring may be substituted with one or two hydrocarbon groups having 1 to 4 carbon atoms or amide groups; and $R^2$ and $R^3$ are each independently a hydrogen atom or a hydrocarbon group having 1 to 4 carbon atoms in which one or two hydrogen atoms may be substituted with a phenyl group, a pyrrolyl group, a pyrimidyl group, or an indolyl group, or $R^2$ and $R^3$ may be bonded to each other to form a 5- to 7-membered nonaromatic heterocycle in which one or two hydrogen atoms of the ring may be substituted with a hydrocarbon group having 1 to 4 carbon atoms, an amide group, or a nitrogen-containing cyclic group, and one carbon atom of the ring may be a carbon atom constituting other nitrogen-containing cyclic group.

2. The pH-sensitive cationic lipid according to claim 1, wherein $-Z^1$-X in the general formula (I) is any one of the following formulas (H-1) to (H-8) and (H-16) to (H-26):

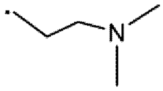
(H-1)
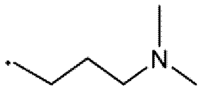
(H-2)
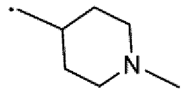
(H-3)
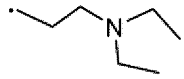
(H-4)

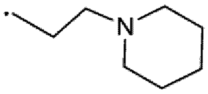
(H-5)
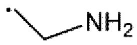
(H-6)
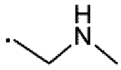
(H-7)
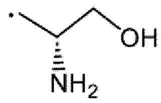
(H-8)
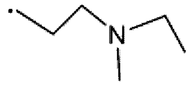
(H-16)

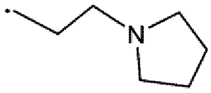
(H-17)
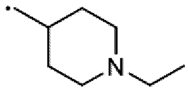
(H-18)
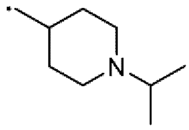
(H-19)
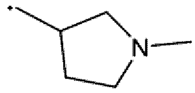
(H-20)

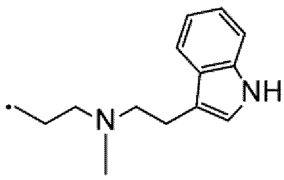
(H-21)
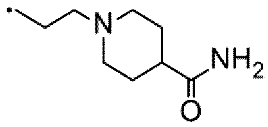
(H-22)
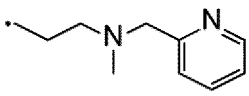
(H-23)

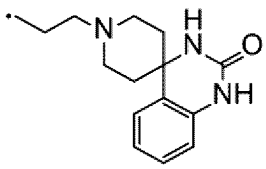
(H-24)
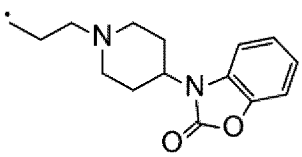
(H-25)
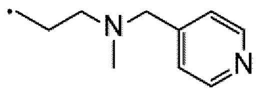
(H-26)

wherein a black circle represents a bonding arm.

**3.** The pH-sensitive cationic lipid according to claim 1, wherein the three $R^1$ groups in one molecule are all the same.

**4.** The pH-sensitive cationic lipid according to claim 1, wherein $R^1$ is an alkyl group having 15 to 22 carbon atoms or an alkenyl group having 15 to 22 carbon atoms.

**5.** The pH-sensitive cationic lipid according to claim 1, which is a compound represented by any one of the following general formulas (I-1) to (I-8) and (I-16) to (I-26):

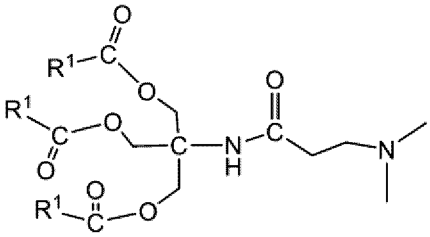

(I-1)

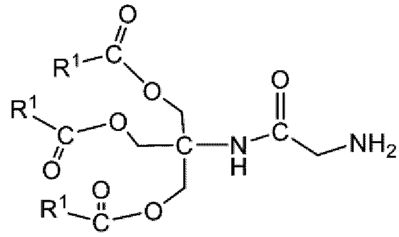

(I-6)

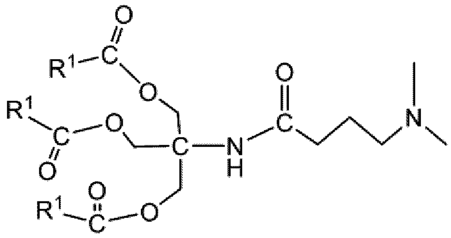

(I-2)

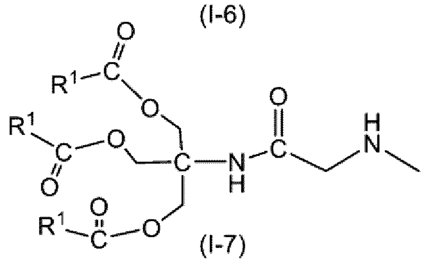

(I-7)

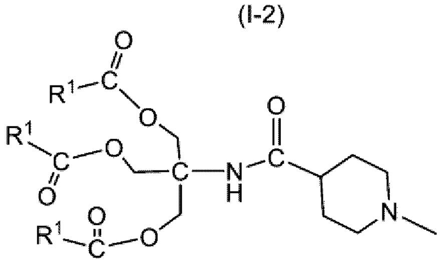

(I-3)

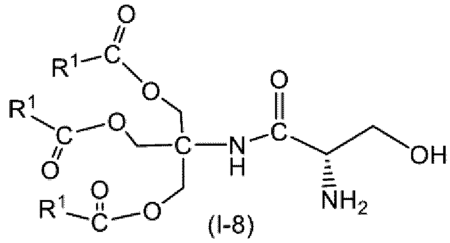

(I-8)

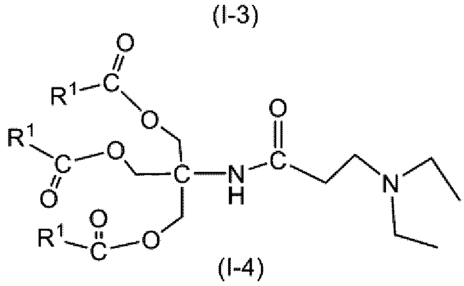

(I-4)

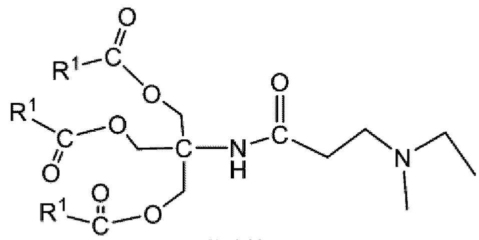

(I-16)

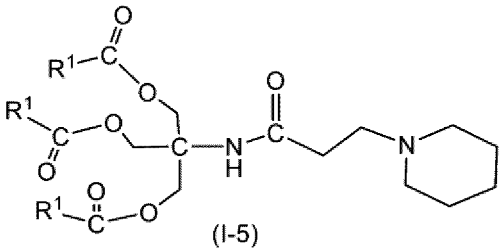

(I-5)

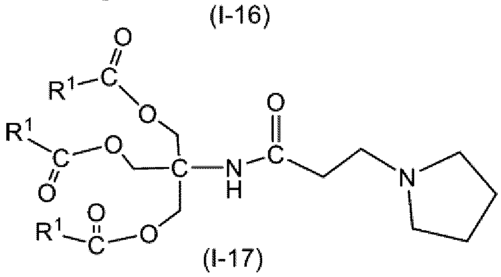

(I-17)

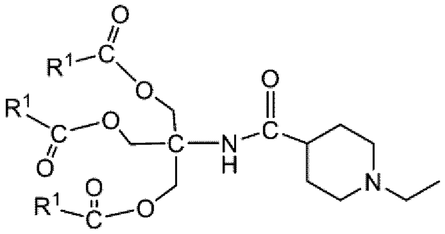

(I-18)

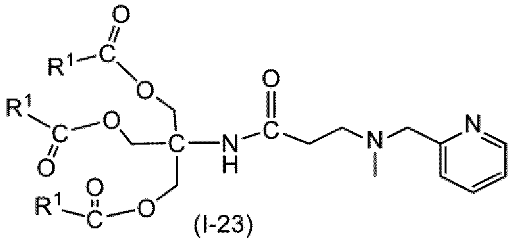

(I-23)

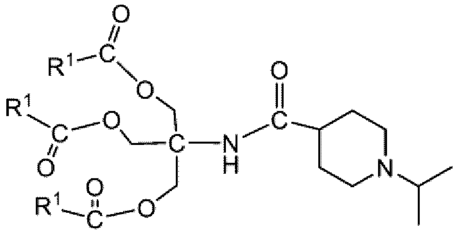

(I-19)

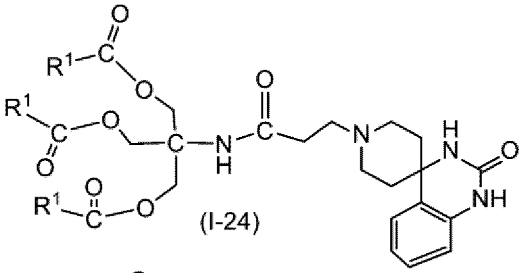

(I-24)

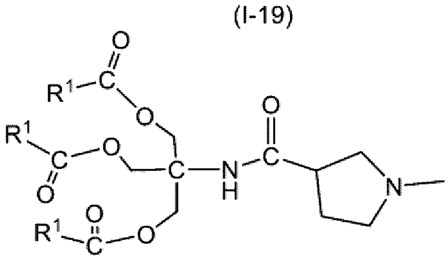

(I-20)

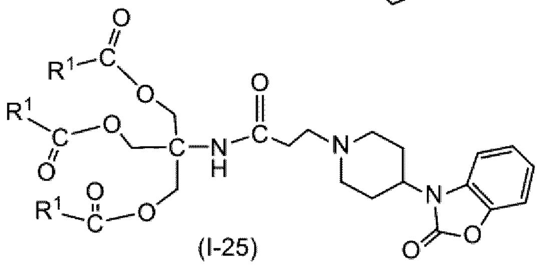

(I-25)

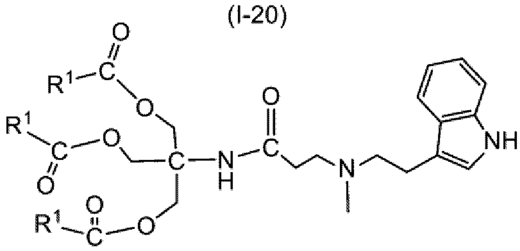

(I-21)

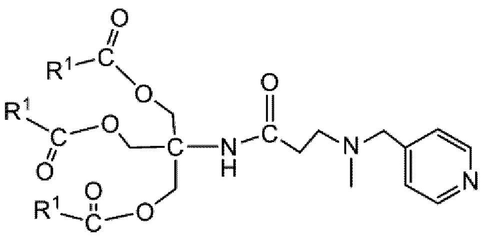

(I-26)

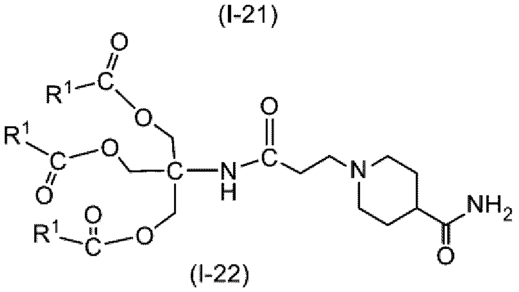

(I-22)

wherein $R^1$ is the same as defined above.

**6.** A pH-sensitive cationic lipid comprising a compound having a Tris skeleton, wherein

a fatty acid residue is bonded to each of three oxygen atoms of the Tris skeleton, the fatty acid residue has 1 to 22 carbon atoms in a hydrocarbon chain moiety thereof and may have one or more unsaturated bonds, and the three fatty acid residues in one molecule may be the same group or two or more different groups,
a primary to tertiary amine is linked to one nitrogen atom of the Tris skeleton via a carbonyl group or a carbonyl group followed by an alkylene group having 1 to 6 carbon atoms, and the alkylene group may be substituted with one hydroxyalkyl group having 1 to 3 carbon atoms, and
the compound has a pKa of 4.0 to 9.0.

**7.** A method for producing a pH-sensitive cationic lipid, the method comprising condensing a compound represented by the following general formula (A):

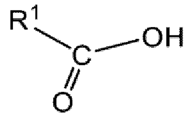

(A)

wherein $R^1$ is an alkyl group having 1 to 22 carbon atoms or an alkenyl group having 2 to 22 carbon atoms, a compound represented by the following general formula (B):

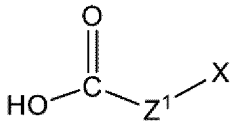

(B)

wherein $Z^1$ is an alkylene group having 1 to 6 carbon atoms which may be substituted with one hydroxyalkyl group having 1 to 3 carbon atoms, or a single bond; X is -N($R^2$)($R^3$) or a 5- to 7-membered nonaromatic heterocyclic group, wherein the group is bonded to $Z^1$ via a carbon atom, and the ring may be substituted with one or two hydrocarbon groups having 1 to 4 carbon atoms or amide groups; and $R^2$ and $R^3$ are each independently a hydrogen atom or a hydrocarbon group having 1 to 4 carbon atoms in which one or two hydrogen atoms may be substituted with a phenyl group, a pyrrolyl group, a pyrimidyl group, or an indolyl group, or $R^2$ and $R^3$ may be bonded to each other to form a 5- to 7-membered nonaromatic heterocycle in which one or two hydrogen atoms of the ring may be substituted with a hydrocarbon group having 1 to 4 carbon atoms, an amide group, or a nitrogen-containing cyclic group, and one carbon atom of the ring may be a carbon atom constituting other nitrogen-containing cyclic group,
and tris(hydroxymethyl)aminomethane to produce a pH-sensitive cationic lipid comprising a compound represented by the following general formula (I):

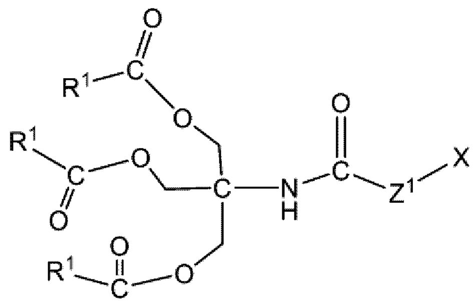

(I)

wherein $R^1$, $Z^1$, and X are the same as defined above, and three $R^1$s groups in one molecule may be the same as or different from each other.

**8.** A method for producing a pH-sensitive cationic lipid, the method comprising adding N-[tris(hydroxymethyl)methyl] acrylamide to a compound represented by the following general formula (C):

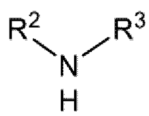

(C)

wherein $R^2$ and $R^3$ are each independently a hydrogen atom or a hydrocarbon group having 1 to 4 carbon atoms in which one or two hydrogen atoms may be substituted with a phenyl group, a pyrrolyl group, a pyrimidyl group, or an indolyl group, or $R^2$ and $R^3$ may be bonded to each other to form a 5- to 7-membered nonaromatic heterocycle in which one or two hydrogen atoms of the ring may be substituted with a hydrocarbon group having 1 to 4 carbon atoms, an amide group, or a nitrogen-containing cyclic group, and one carbon atom of the ring may be a carbon atom constituting other nitrogen-containing cyclic group,
and then further condensing a resulting compound with a compound represented by the following general formula (A):

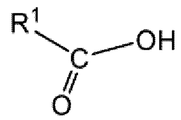

(A)

wherein $R^1$ is an alkyl group having 1 to 22 carbon atoms or an alkenyl group having 2 to 22 carbon atoms to produce a pH-sensitive cationic lipid comprising a compound represented by the following general formula (I'):

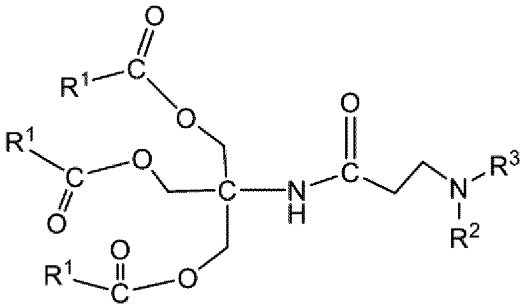

(I')

wherein $R^1$, $R^2$, and $R^3$ are the same as defined above; and three $R^1$ groups in one molecule may be the same as or different from each other.

**9.** A lipid nanoparticle comprising the pH-sensitive cationic lipid according to any one of claims 1 to 6.

**10.** The lipid nanoparticle according to claim 9, further comprising a sterol and a polyalkylene glycol-modified lipid.

**11.** The lipid nanoparticle according to claim 9, further comprising phosphatidylcholine.

**12.** The lipid nanoparticle according to claim 9, containing a nucleic acid.

**13.** The lipid nanoparticle according to claim 12, wherein the nucleic acid is siRNA, mRNA, or plasmid DNA.

**14.** A pharmaceutical composition comprising the lipid nanoparticle containing the pH-sensitive cationic lipid according to any one of claims 1 to 6 as an active ingredient.

**15.** A pharmaceutical composition comprising, as an active ingredient, the lipid nanoparticle containing the pH-sensitive cationic lipid according to any one of claims 1 to 6 and a nucleic acid.

**16.** The pharmaceutical composition according to claim 15, which is used for gene therapy.

**17.** A method for expressing a foreign gene, comprising administering to a subject animal (excluding human), the lipid nanoparticle according to claim 12, which encapsulates a foreign gene of interest to be expressed in a target cell, and expressing the foreign gene in the target cell of the subject animal.

FIG.1

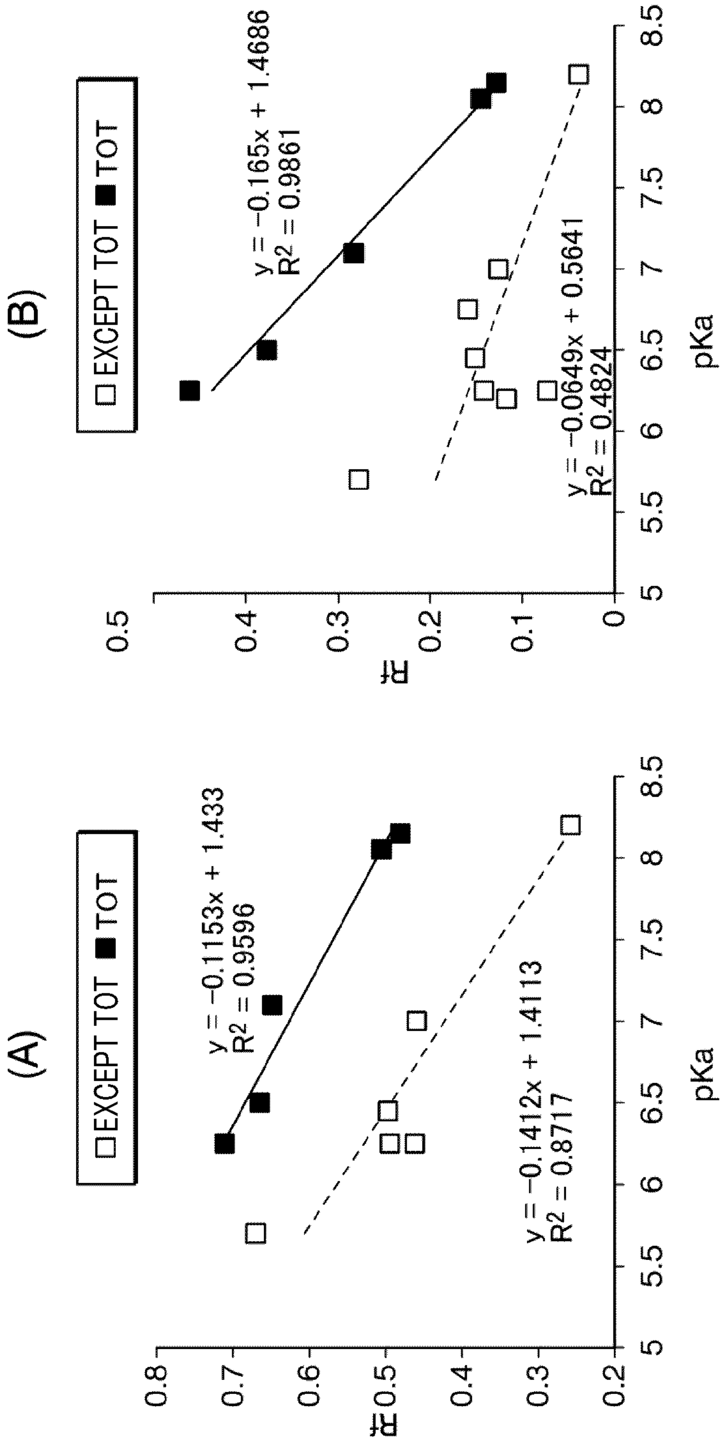
(A)
□EXCEPT TOT ■TOT
y = −0.1153x + 1.433
R2 = 0.9596
y = −0.1412x + 1.4113
R2 = 0.8717
Rf
pKa
0.8
0.7
0.6
0.5
0.4
0.3
0.2
5
5.5
6
6.5
7
7.5
8
8.5
(B)
□ EXCEPT TOT ■ TOT
y = −0.165x + 1.4686
R2 = 0.9861
y = −0.0649x + 0.5641
R2 = 0.4824
Rf
pKa
0.5
0.4
0.3
0.2
0.1
0
5
5.5
6
6.5
7
7.5
8
8.5

FIG.2

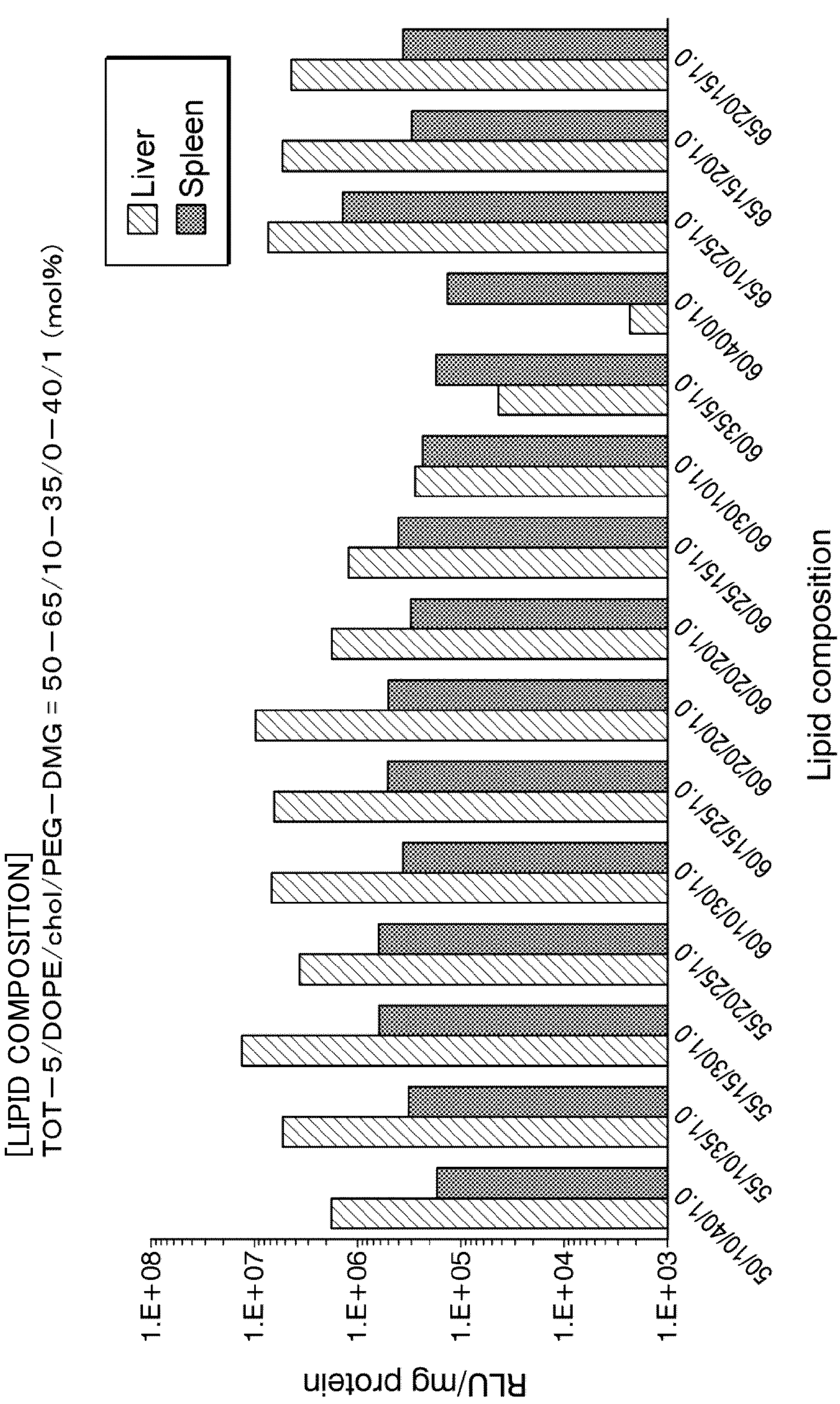
[LIPID COMPOSITION]
TOT−5/DOPE/chol/PEG−DMG = 50−65/10−35/0−40/1 (mol%)
Liver
Spleen
RLU/mg protein
1.E+08
1.E+07
1.E+06
1.E+05
1.E+04
1.E+03
50/10/40/1.0
55/10/35/1.0
55/15/30/1.0
55/20/25/1.0
60/10/30/1.0
60/15/25/1.0
60/20/20/1.0
60/20/20/1.0
60/25/15/1.0
60/30/10/1.0
60/35/5/1.0
60/40/0/1.0
65/10/25/1.0
65/15/20/1.0
65/20/15/1.0
Lipid composition

# FIG.3

[LIPID COMPOSITION]
TOT−5/PE/chol/PEG−DMG = 60/15/25/1 (mol%)

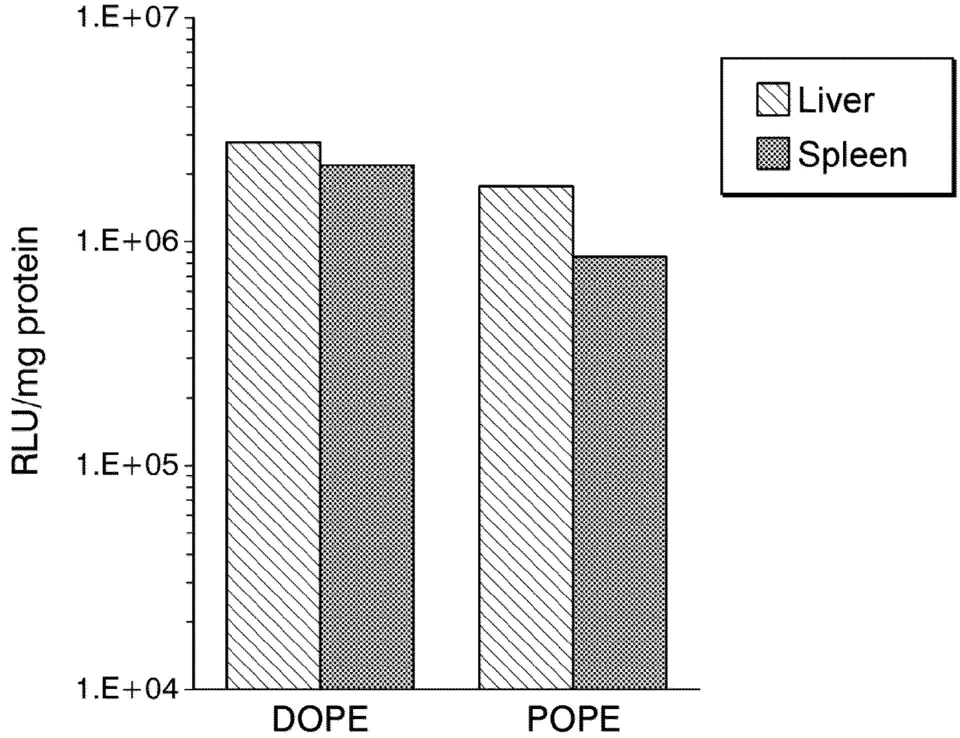


# FIG.4

[LIPID COMPOSITION]
TOT/DOPE/chol/PEG−DMG = 60/15/25/1 (mol%)

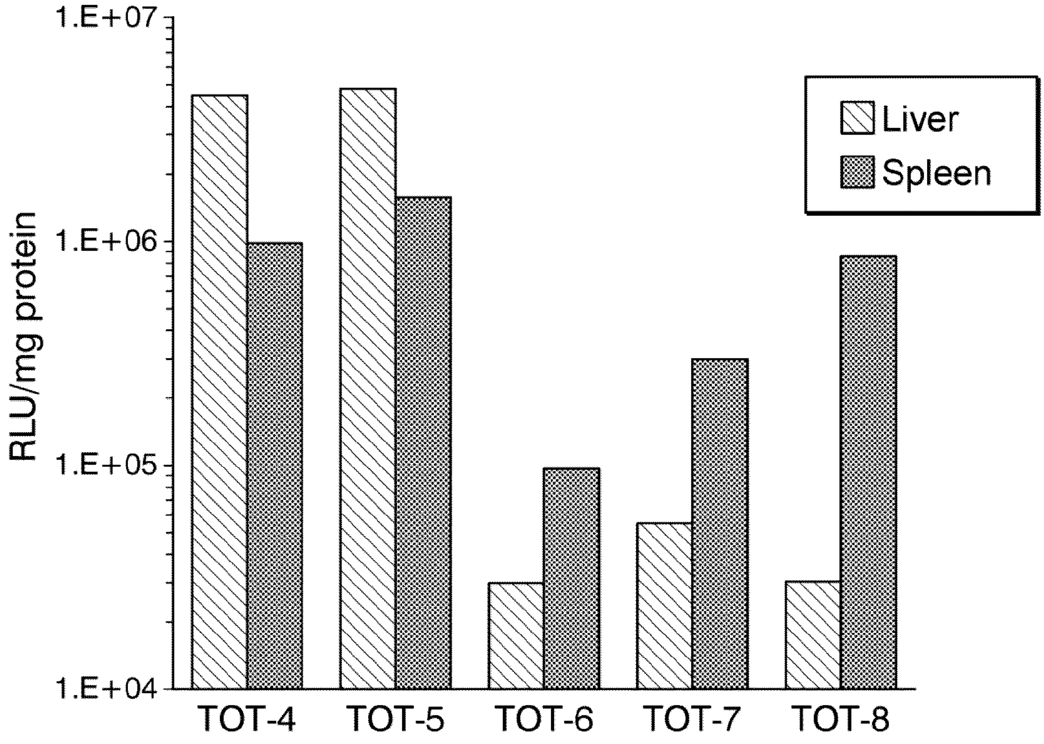

FIG.5

[LIPID COMPOSITION]
TOT−5/DSPC/chol/PEG−DMG = 55−70/5−30/10−35/1 (mol%)

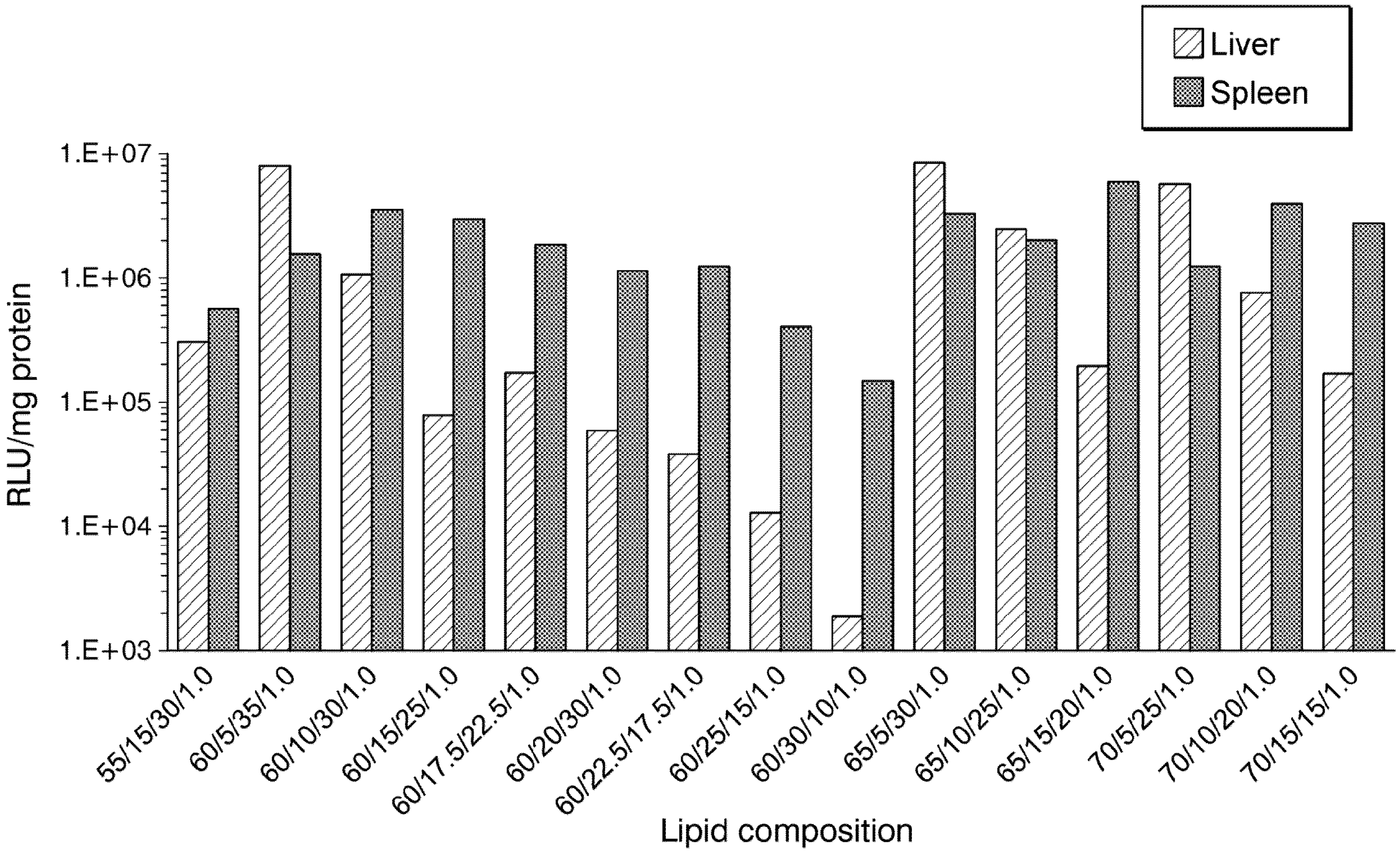

FIG.6

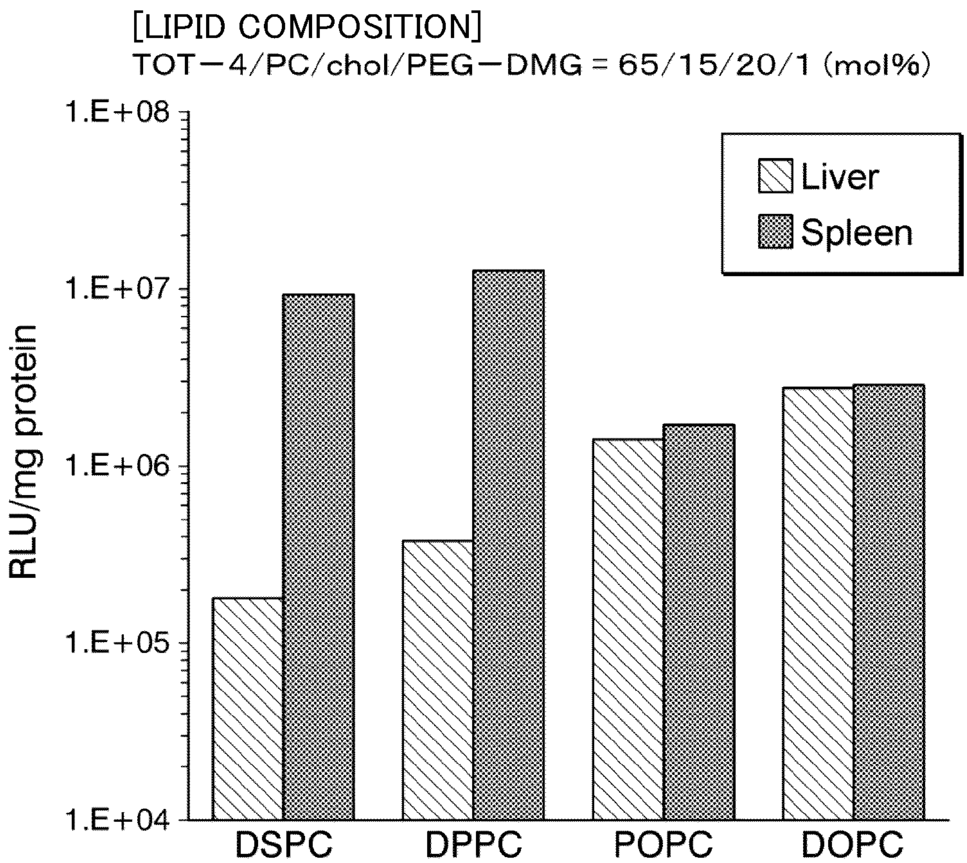
[LIPID COMPOSITION]
TOT−4/PC/chol/PEG−DMG = 65/15/20/1 (mol%)
RLU/mg protein
1.E+08
1.E+07
1.E+06
1.E+05
1.E+04
Liver
Spleen
DSPC
DPPC
POPC
DOPC

FIG.7

[LIPID COMPOSITION]
TOT/DSPC/chol/PEG−DMG = 65/15/20/1.5 (mol%)

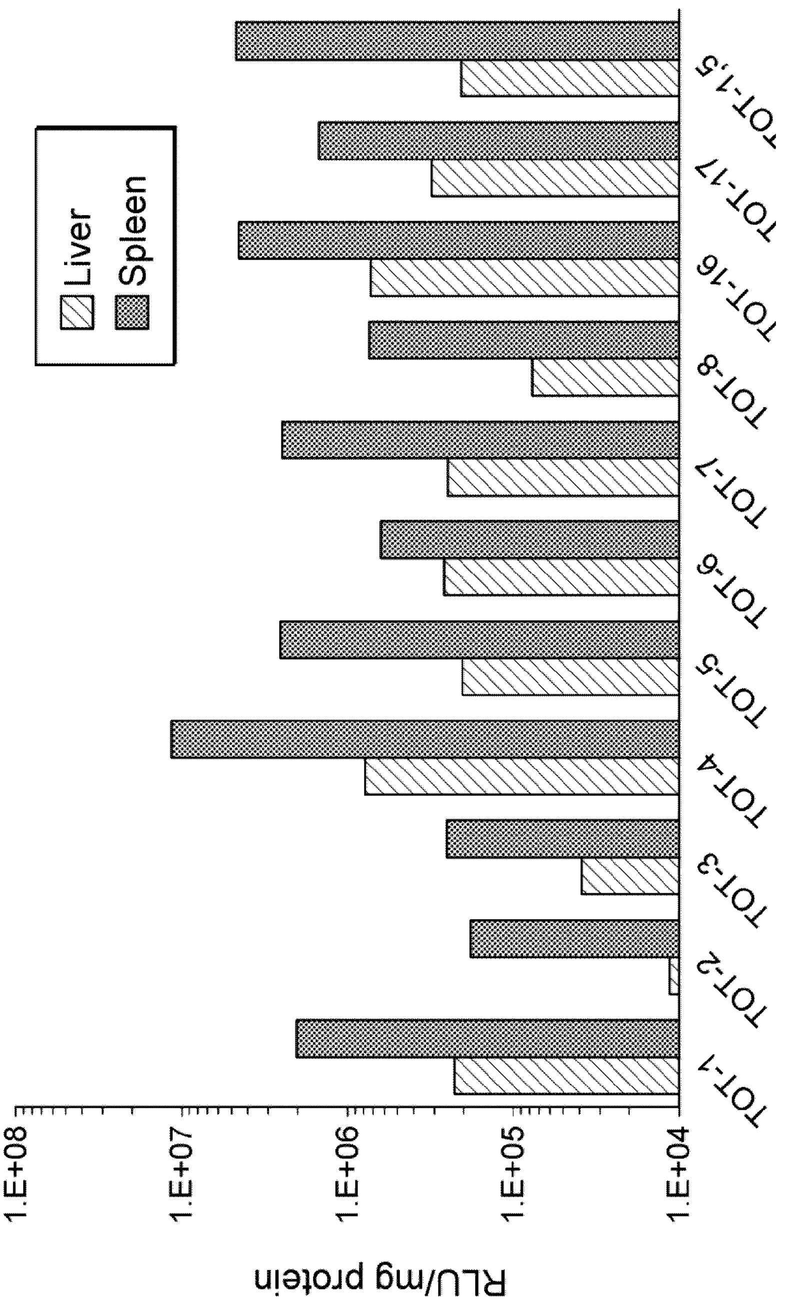

# FIG.8

[LIPID COMPOSITION]
TOT−4:TOT−4/DSPC/chol/PEG−DMG = 65/15/20/1.5 (mol%)
RNA−LPX: DOTMA/DOPE = 50/50 (mol%), N/P = 0.65

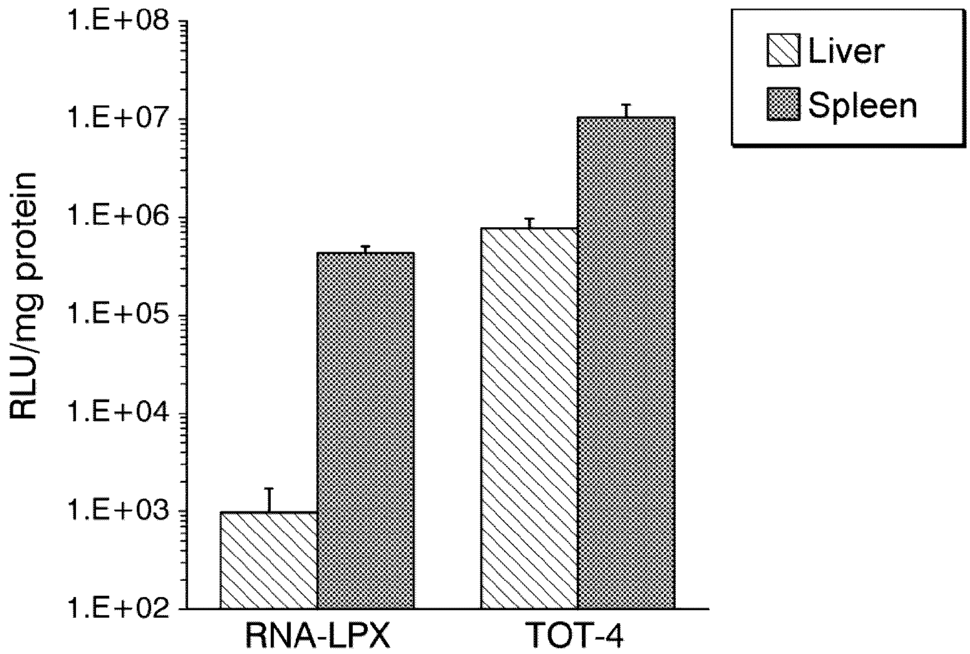


# FIG.9

[LIPID COMPOSITION]
CL4F6/DSPC/chol/PEG−DMG = 50/10/40/1.5 (mol%)
TOT−4/DSPC/chol/PEG−DMG = 65/15/20/1.5 (mol%)

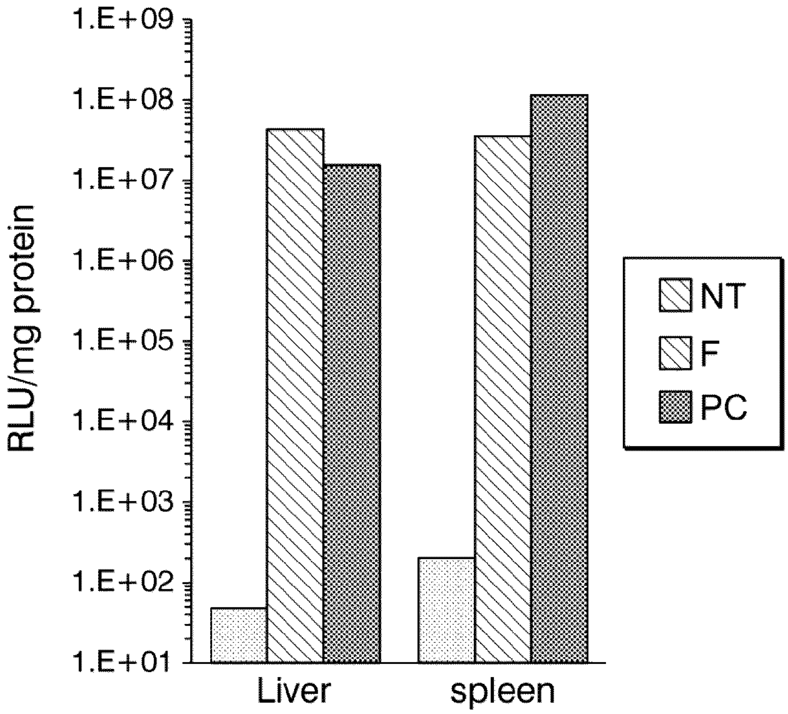

FIG.10

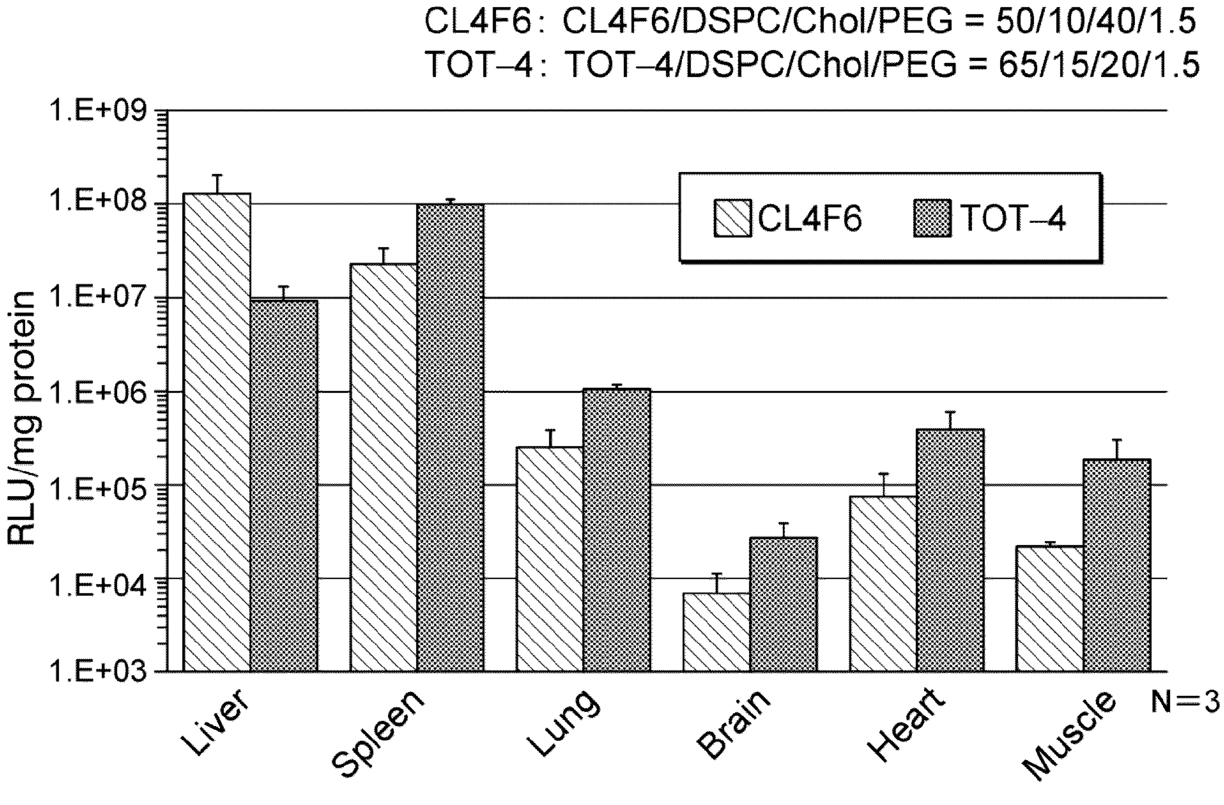
CL4F6 : CL4F6/DSPC/Chol/PEG = 50/10/40/1.5
TOT–4 : TOT–4/DSPC/Chol/PEG = 65/15/20/1.5
RLU/mg protein
1.E+09
1.E+08
1.E+07
1.E+06
1.E+05
1.E+04
1.E+03
CL4F6
TOT–4
Liver
Spleen
Lung
Brain
Heart
Muscle
N=3

# FIG.11

DSPC: TOT-4/DSPC/Chol/PEG = 65/15/20/1.5
DOPE: TOT-4/DOPE/Chol/PEG = 60/15/25/1.5

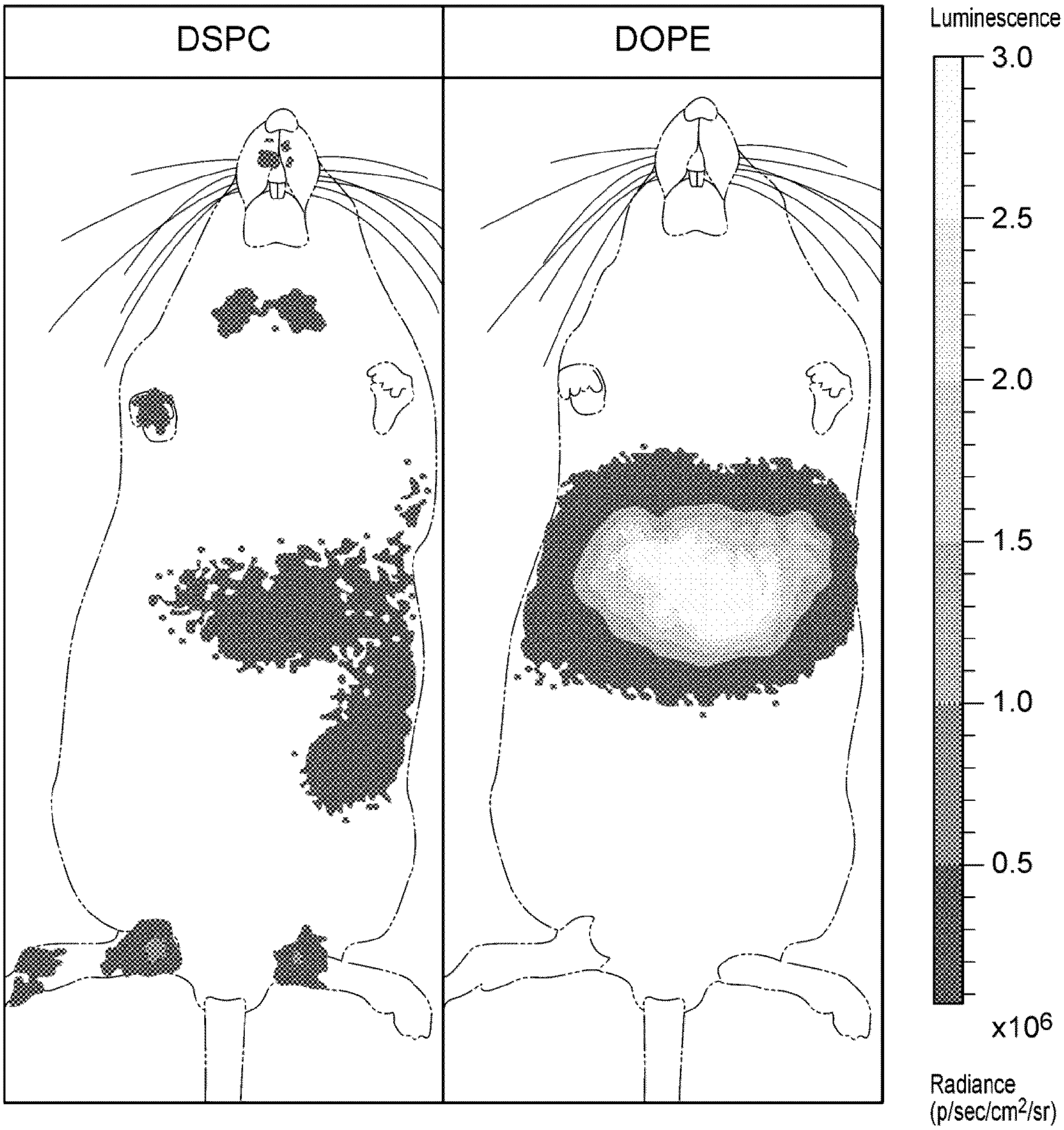

FIG.12

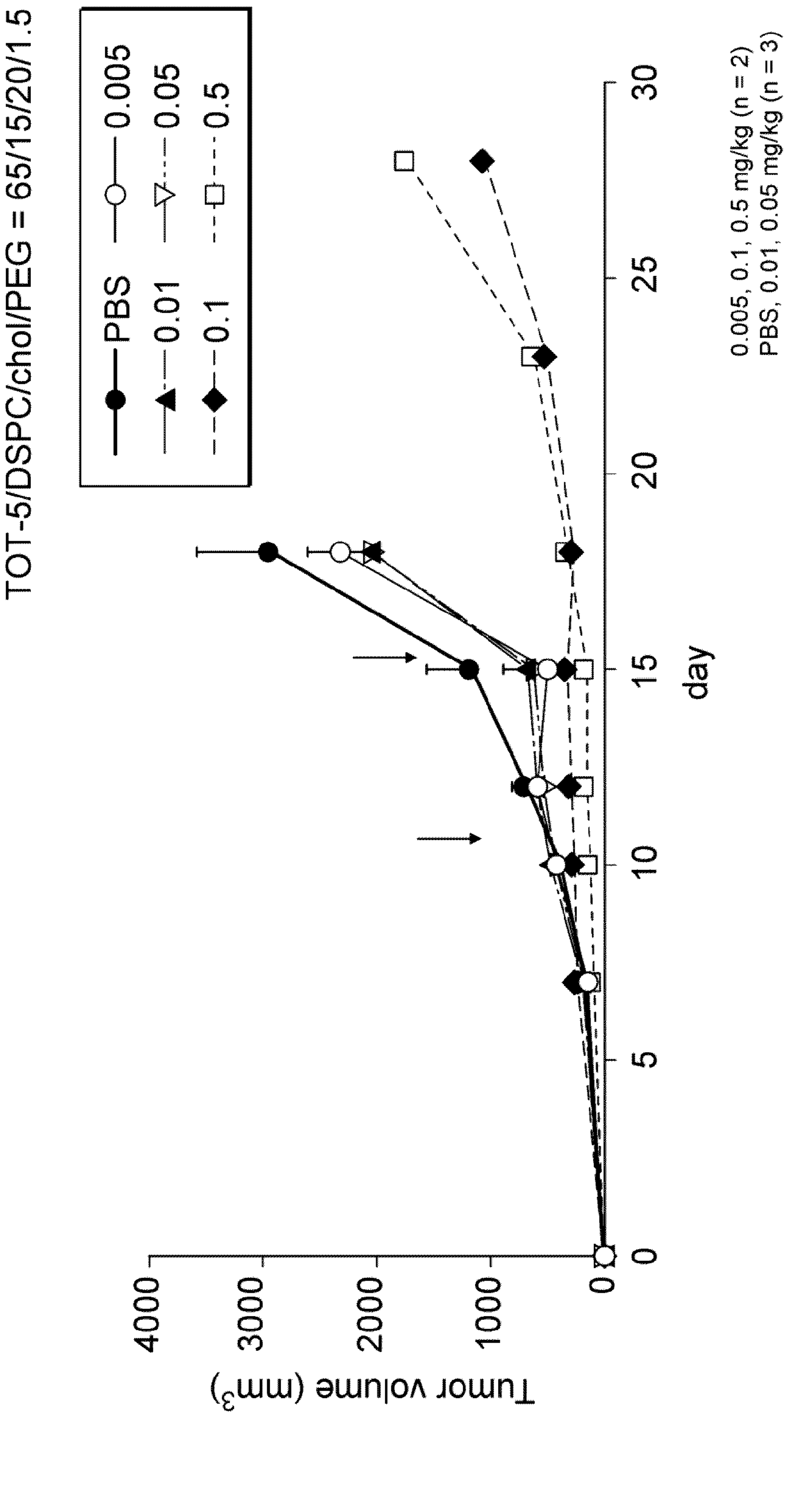
TOT-5/DSPC/chol/PEG = 65/15/20/1.5
PBS
0.005
0.01
0.05
0.1
0.5
Tumor volume (mm3)
4000
3000
2000
1000
0
0
5
10
15
20
25
30
day
0.005, 0.1, 0.5 mg/kg (n = 2)
PBS, 0.01, 0.05 mg/kg (n = 3)

FIG.13

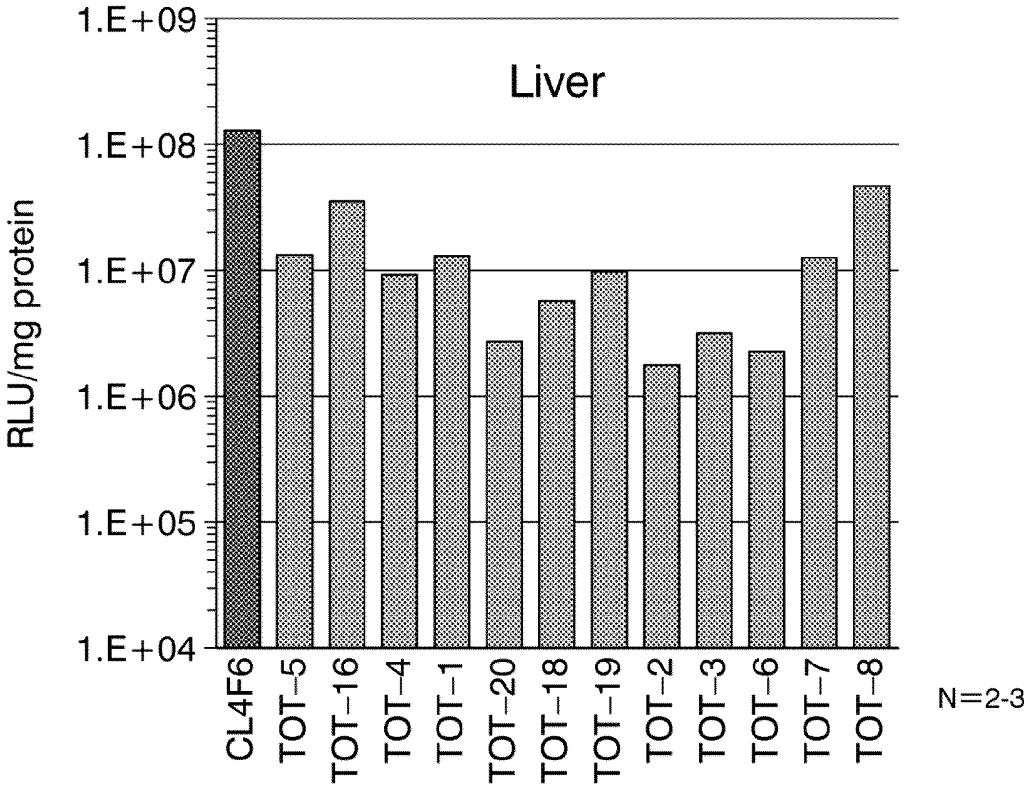
1.E+09
1.E+08
1.E+07
1.E+06
1.E+05
1.E+04
RLU/mg protein
Liver
CL4F6
TOT–5
TOT–16
TOT–4
TOT–1
TOT–20
TOT–18
TOT–19
TOT–2
TOT–3
TOT–6
TOT–7
TOT–8
N=2-3

FIG.14

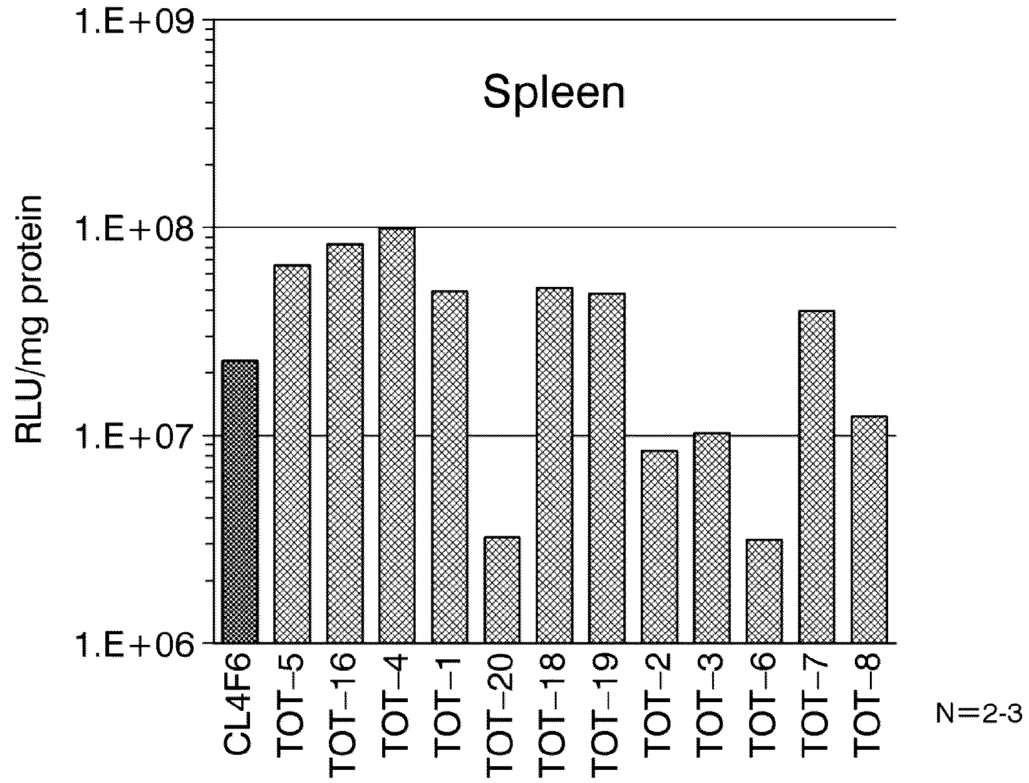
1.E+09
1.E+08
1.E+07
1.E+06
RLU/mg protein
Spleen
CL4F6
TOT–5
TOT–16
TOT–4
TOT–1
TOT–20
TOT–18
TOT–19
TOT–2
TOT–3
TOT–6
TOT–7
TOT–8
N=2-3

FIG.15

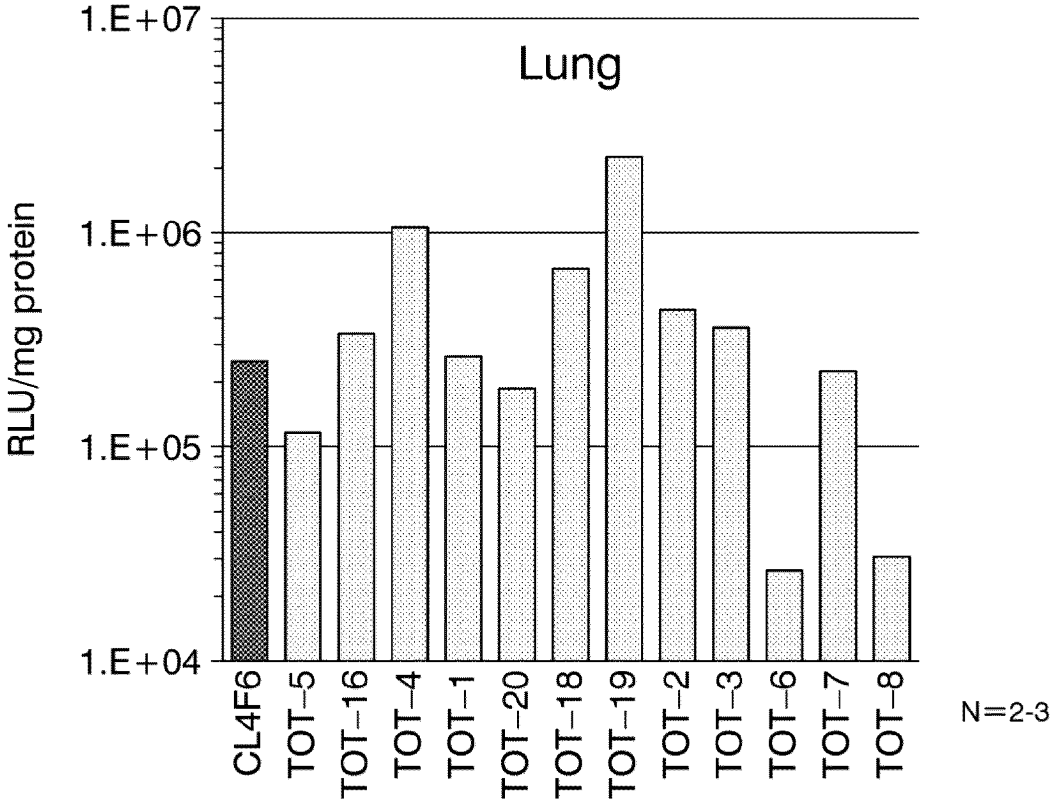
Lung
RLU/mg protein
1.E+07
1.E+06
1.E+05
1.E+04
CL4F6
TOT−5
TOT−16
TOT−4
TOT−1
TOT−20
TOT−18
TOT−19
TOT−2
TOT−3
TOT−6
TOT−7
TOT−8
N=2-3

FIG.16

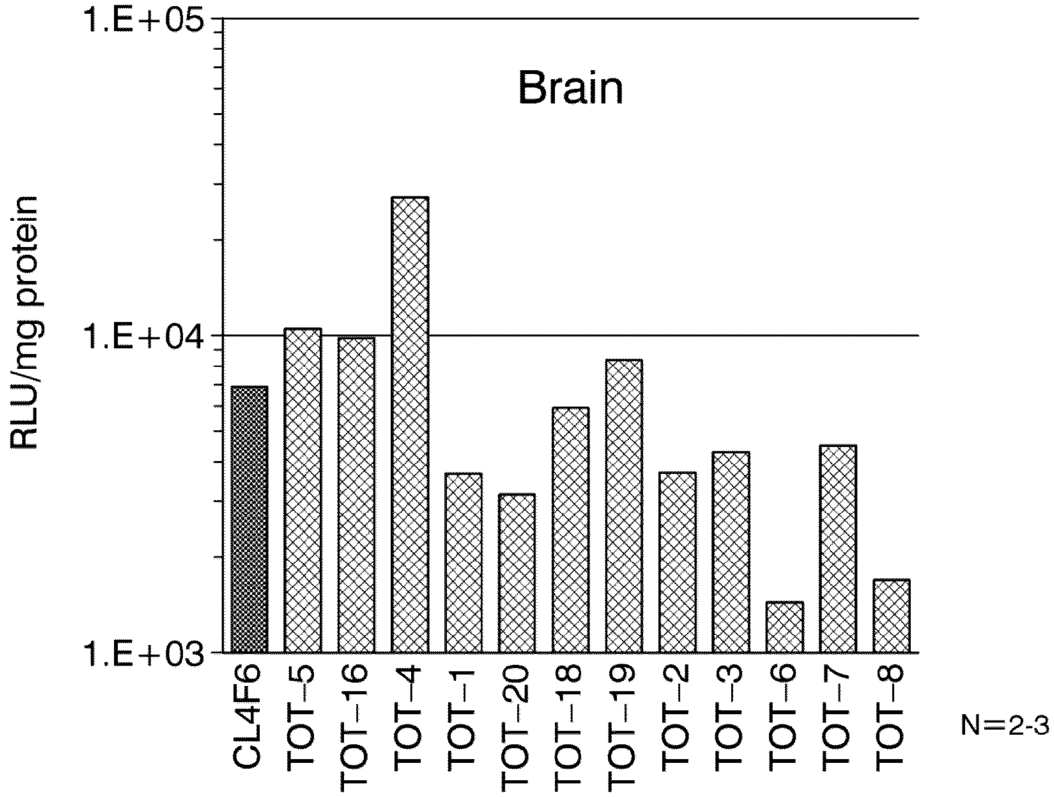
Brain
RLU/mg protein
1.E+05
1.E+04
1.E+03
CL4F6
TOT−5
TOT−16
TOT−4
TOT−1
TOT−20
TOT−18
TOT−19
TOT−2
TOT−3
TOT−6
TOT−7
TOT−8
N=2-3

FIG.17

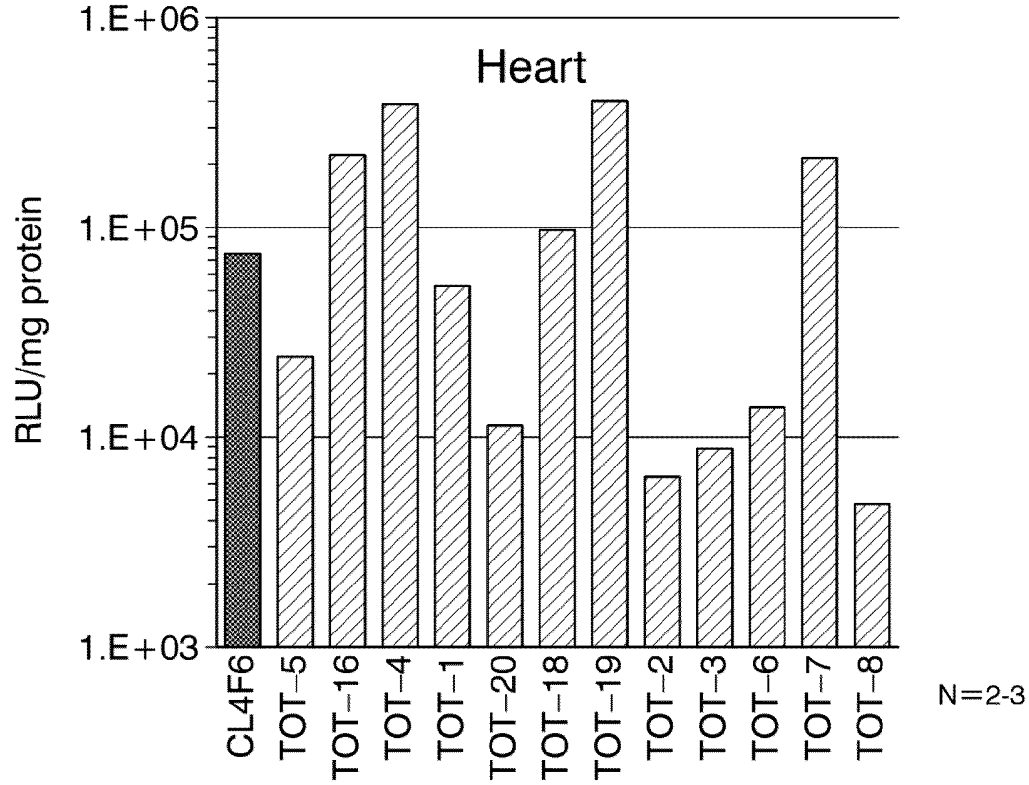
Heart
RLU/mg protein
1.E+06
1.E+05
1.E+04
1.E+03
CL4F6
TOT-5
TOT-16
TOT-4
TOT-1
TOT-20
TOT-18
TOT-19
TOT-2
TOT-3
TOT-6
TOT-7
TOT-8
N=2-3

FIG.18

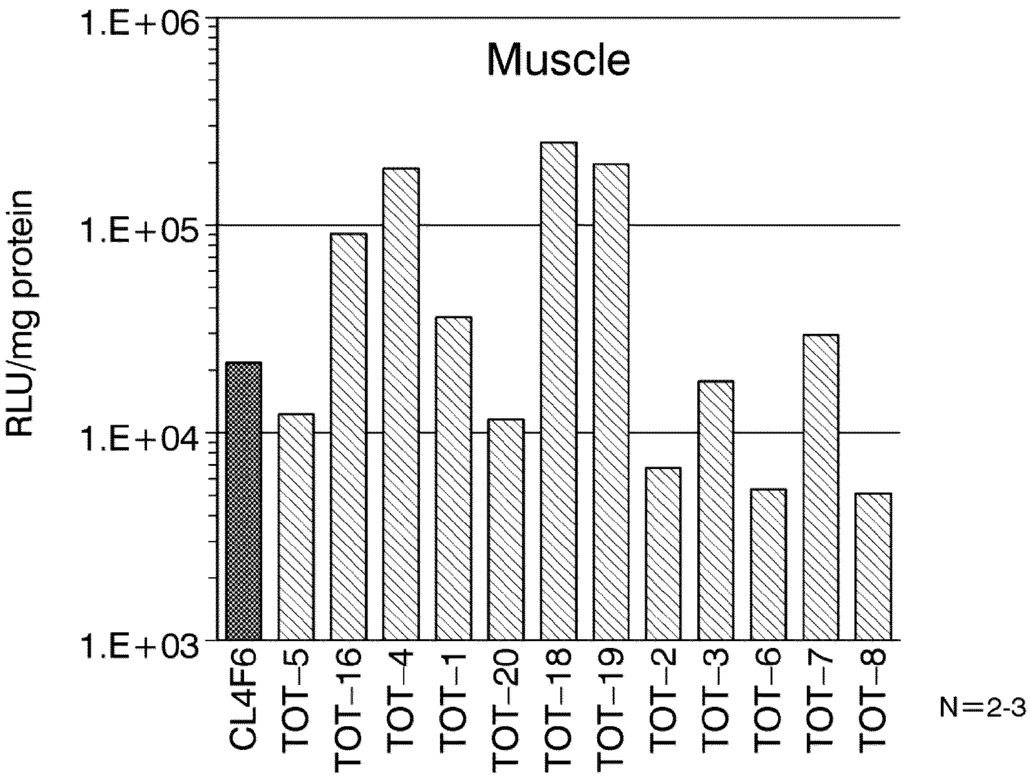
Muscle
RLU/mg protein
1.E+06
1.E+05
1.E+04
1.E+03
CL4F6
TOT-5
TOT-16
TOT-4
TOT-1
TOT-20
TOT-18
TOT-19
TOT-2
TOT-3
TOT-6
TOT-7
TOT-8
N=2-3

FIG.19

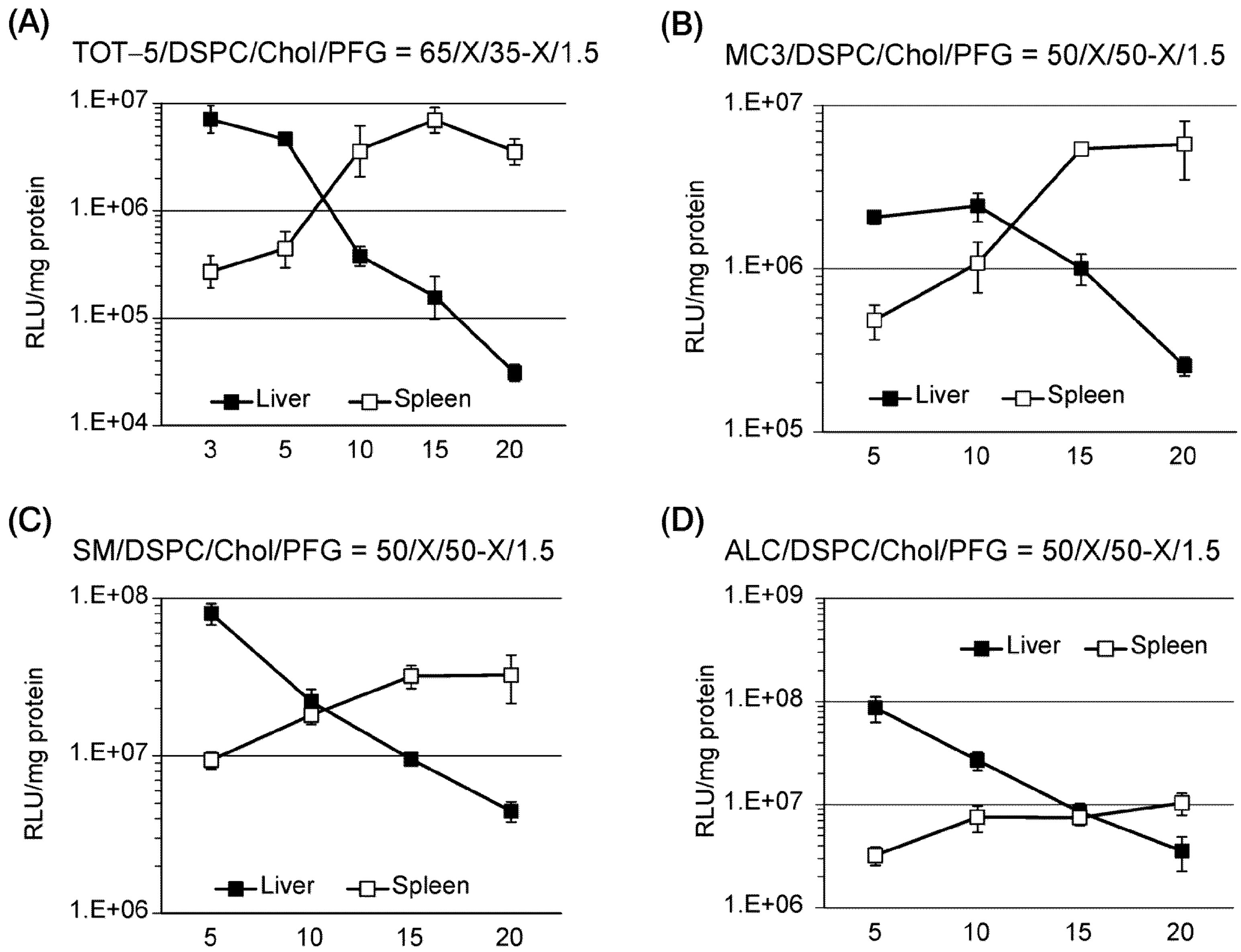
(A)
TOT–5/DSPC/Chol/PFG = 65/X/35-X/1.5
RLU/mg protein
1.E+07
1.E+06
1.E+05
1.E+04
3
5
10
15
20
Liver
Spleen
(B)
MC3/DSPC/Chol/PFG = 50/X/50-X/1.5
RLU/mg protein
1.E+07
1.E+06
1.E+05
5
10
15
20
Liver
Spleen
(C)
SM/DSPC/Chol/PFG = 50/X/50-X/1.5
RLU/mg protein
1.E+08
1.E+07
1.E+06
5
10
15
20
Liver
Spleen
(D)
ALC/DSPC/Chol/PFG = 50/X/50-X/1.5
RLU/mg protein
1.E+09
1.E+08
1.E+07
1.E+06
5
10
15
20
Liver
Spleen

# FIG.20

TOT/DSPC/Chol/PEG = 65/X/35-X/1.5

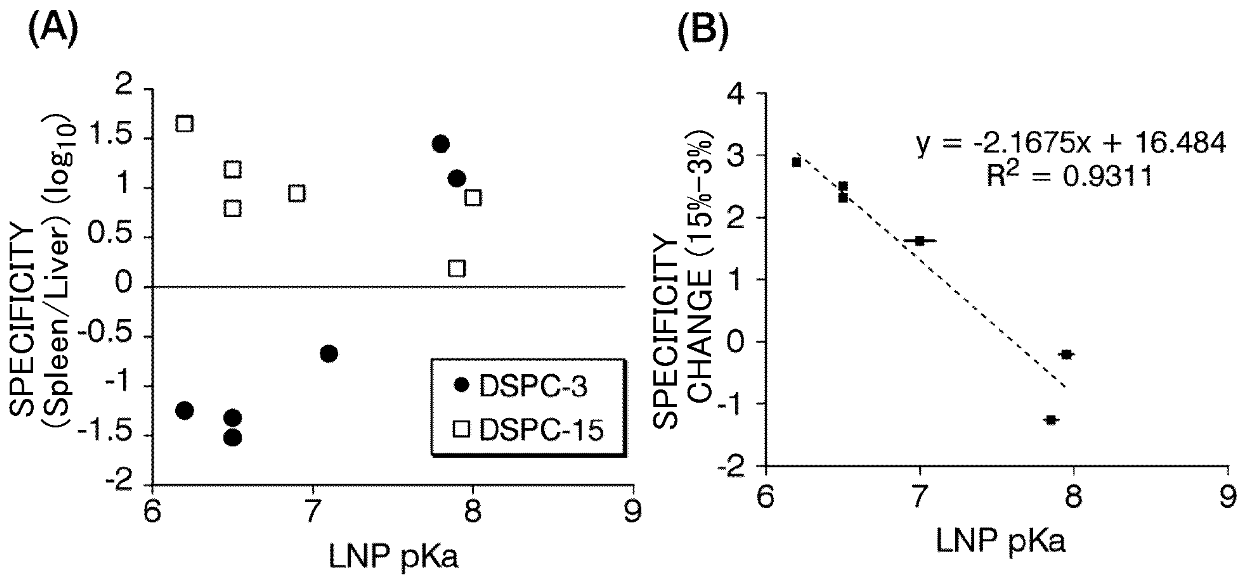


# FIG.21

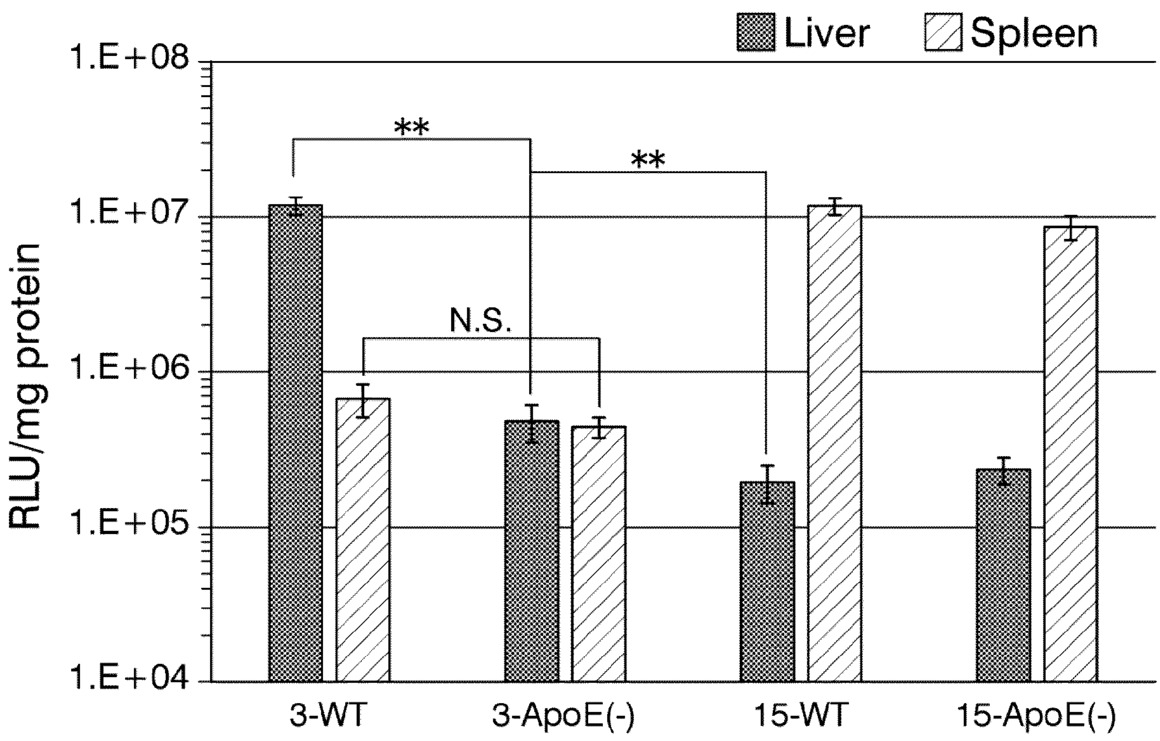

FIG.22

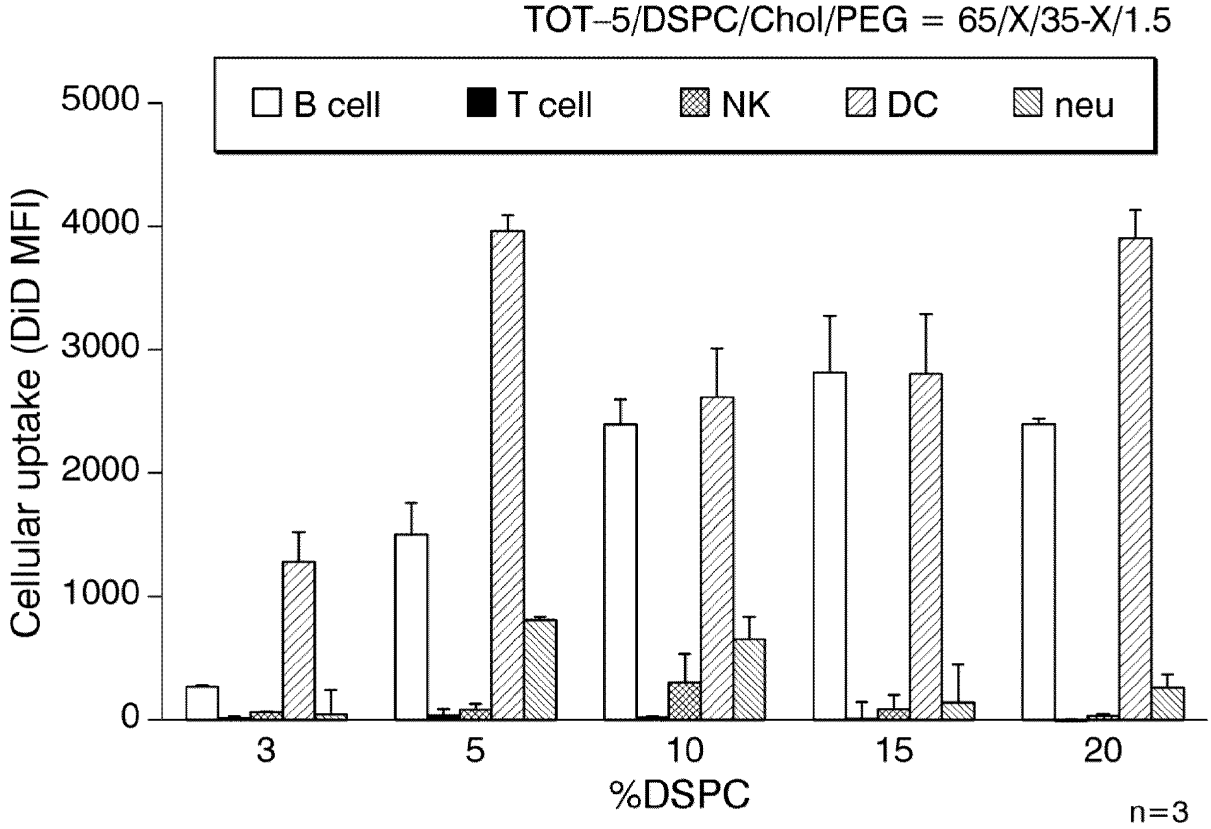
TOT–5/DSPC/Chol/PEG = 65/X/35-X/1.5
B cell
T cell
NK
DC
neu
5000
4000
3000
2000
1000
0
Cellular uptake (DiD MFI)
3
5
10
15
20
%DSPC
n=3

FIG.23

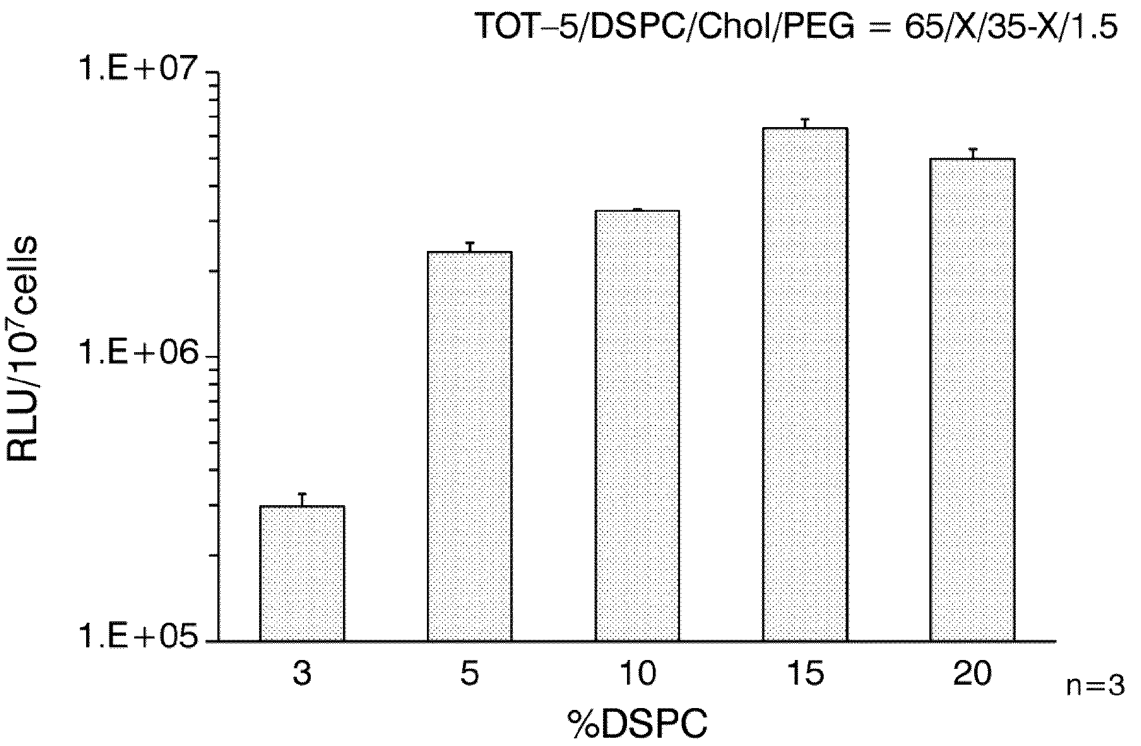
TOT–5/DSPC/Chol/PEG = 65/X/35-X/1.5
1.E+07
1.E+06
1.E+05
RLU/10^7cells
3
5
10
15
20
n=3
%DSPC

FIG.24

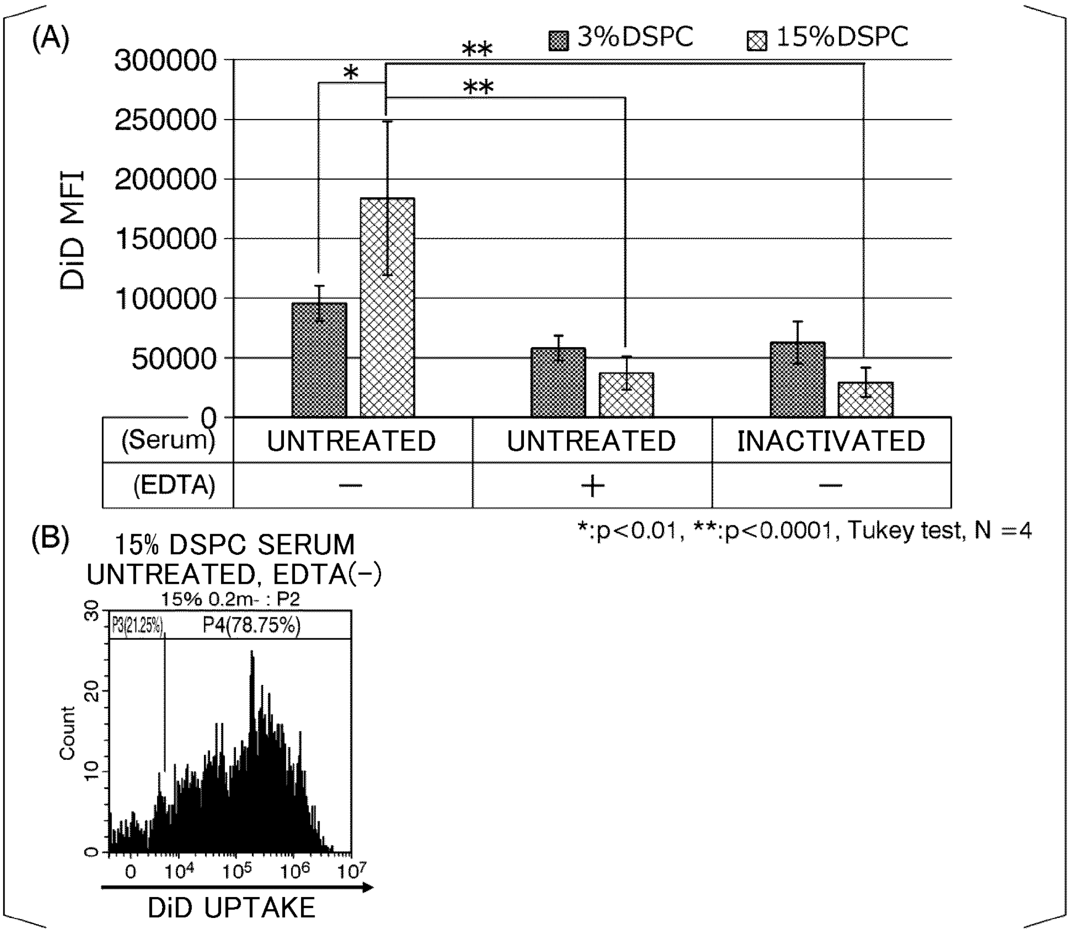
(A)
3%DSPC
15%DSPC
300000
250000
200000
150000
100000
50000
0
DiD MFI
*
**
**
(Serum)
UNTREATED
UNTREATED
INACTIVATED
(EDTA)
–
+
–
*:p<0.01, **:p<0.0001, Tukey test, N =4
(B)
15% DSPC SERUM
UNTREATED, EDTA(-)
15% 0.2m- : P2
P3(21.25%)
P4(78.75%)
Count
0
10
20
30
0
$10^4$
$10^5$
$10^6$
$10^7$
DiD UPTAKE

FIG.25

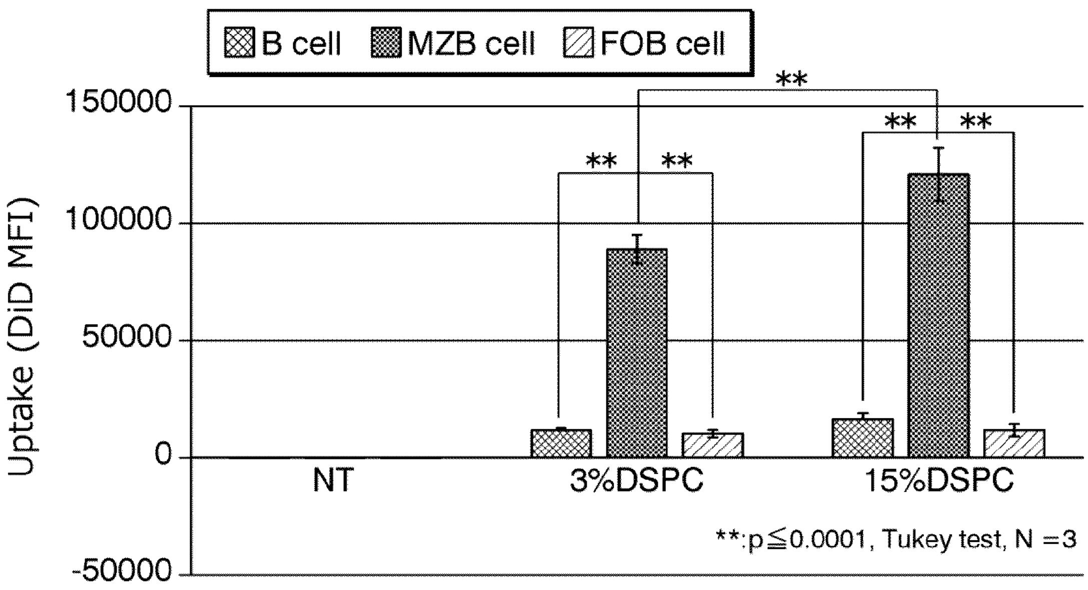
B cell
MZB cell
FOB cell
Uptake (DiD MFI)
150000
100000
50000
0
-50000
**
**
**
**
**
NT
3%DSPC
15%DSPC
**:p≦0.0001, Tukey test, N =3

**INTERNATIONAL SEARCH REPORT**

International application No. **PCT/JP2023/020385**

**A. CLASSIFICATION OF SUBJECT MATTER**

***C07C 237/08***(2006.01)i; ***A61K 9/51***(2006.01)i; ***A61K 31/7088***(2006.01)i; ***A61K 31/7105***(2006.01)i; ***A61K 47/10***(2017.01)i; ***A61K 47/18***(2017.01)i; ***A61K 47/28***(2006.01)i; ***A61K 47/34***(2017.01)i; ***A61K 48/00***(2006.01)i; ***C07C 231/02***(2006.01)i; ***C12N 15/88***(2006.01)i

FI: C07C237/08; A61K9/51; A61K31/7088; A61K31/7105; A61K47/10; A61K47/18; A61K47/28; A61K47/34; A61K48/00; C07C231/02 CSP; C12N15/88 Z

According to International Patent Classification (IPC) or to both national classification and IPC

**B. FIELDS SEARCHED**

Minimum documentation searched (classification system followed by classification symbols)

C07C237/08; A61K9/51; A61K31/7088; A61K31/7105; A61K47/10; A61K47/18; A61K47/28; A61K47/34; A61K48/00; C07C231/02; C12N15/88

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 9-501655 A (COMMONWEALTH SCIENTIFIC AND INDUSTRIAL RESEARCH OR.) 18 February 1997 (1997-02-18)<br>page 15 | 1-5 |
| A | claims, page 15 | 6-17 |
| X | DURAND, Gregory et al. Fine-Tuning the Amphiphilicity: A Crucial Parameter in the Design of Potent α-Phenyl-N-tert-butylnitrone Analogues. Journal of Medicinal Chemistry. 2007, vol. 50, no. 17, pages 3976-3979, scheme 1.<br>scheme 1 | 1-3, 5 |
| A | scheme 1 | 4, 6-17 |
| X | WO 2018/062413 A1 (KYOWA HAKKO KIRIN CO., LTD.) 05 April 2018 (2018-04-05)<br>example 29, table 15 | 1-6 |
| A | example 29, table 15 | 7-17 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 August 2023** | **22 August 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No. **PCT/JP2023/020385**

**C. DOCUMENTS CONSIDERED TO BE RELEVANT**

| Category* | Citation of document, with indication, where appropriate, of the relevant passages | Relevant to claim No. |
|---|---|---|
| X | WO 2022/060871 A1 (VERVE THERAPEUTICS, INC.) 24 March 2022 (2022-03-24) claims, page 105, example 35, paragraphs [0206], [0240] | 1-6, 9-17 |
| A | claims, page 105, example 35, paragraphs [0206], [0240] | 7-8 |
| A | JP 10-509862 A (COMMONWEALTH SCIENTIFIC AND INDUSTRIAL RESEARCH OR.) 29 September 1998 (1998-09-29) claims, examples | 1-17 |
| A | WO 2020/257716 A1 (TARANSLATE BIO, INC.) 24 December 2020 (2020-12-24) claims, examples | 1-17 |
| A | JP 10-501822 A (PASTEUR MERIEUX SERUMS ET VACCINS) 17 February 1998 (1998-02-17) claims, examples | 1-17 |

Form PCT/ISA/210 (second sheet) (January 2015)

**INTERNATIONAL SEARCH REPORT**
**Information on patent family members**

International application No.
**PCT/JP2023/020385**

| Patent document cited in search report | Publication date (day/month/year) | Patent family member(s) | Publication date (day/month/year) |
|---|---|---|---|
| JP 9-501655 A | 18 February 1997 | US 5792786 A<br>claims, columns 4, 5<br>WO 1995/004030 A1<br>EP 712389 A1<br>NZ 269773 A<br>AU 7342094 A<br>FI 960504 A<br>NO 313227 B<br>AU 683289 B<br>CA 2167818 A1<br>RU 2137755 C<br>AT 198880 T<br>DK 712389 T<br>ES 2156156 T<br>CN 1128531 A | |
| WO 2018/062413 A1 | 05 April 2018 | TW 201813632 A | |
| WO 2022/060871 A1 | 24 March 2022 | (Family: none) | |
| JP 10-509862 A | 29 September 1998 | US 5906922 A<br>claims, examples<br>WO 1996/005218 A1<br>EP 777679 A1<br>AU PM747694 A<br>AU PM767794 A<br>NO 970651 D<br>FI 970648 A<br>AU 3158595 A<br>CA 2197653 A<br>RU 2160278 C<br>NZ 290757 A<br>AT 253588 T<br>DK 777679 T<br>CN 1158619 A<br>AU PM747694 A0 | |
| WO 2020/257716 A1 | 24 December 2020 | JP 2022-537580 A<br>US 2023/0092238 A1<br>EP 3986858 A1<br>CN 114401942 A<br>AU 2020297606 A<br>CA 3144457 A1 | |
| JP 10-501822 A | 17 February 1998 | US 6124270 A<br>claims, examples<br>WO 1996/032102 A1<br>EP 769942 A1<br>EP 1245249 A2<br>FR 2732895 A<br>AU 5651796 A<br>CA 2192597 A1<br>AT 223716 T<br>DK 769942 T | |

Form PCT/ISA/210 (patent family annex) (January 2015)

**INTERNATIONAL SEARCH REPORT**
**Information on patent family members**

International application No.
**PCT/JP2023/020385**

| Patent document cited in search report | Publication date (day/month/year) | Patent family member(s) | Publication date (day/month/year) |
|---|---|---|---|
| | | ES 2181885 T | |
| | | PT 769942 E | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022115015 A
- WO 2018230710 A
- WO 2018190423 A
- WO 2007102481 A


### Non-patent literature cited in the description


- **JAYARAMAN et al.** *Angewandte Chemie International Edition*, 2012, vol. 51, 8529-8533
- **WATANABE et al.** *Scientific Reports*, 2014, vol. 4, 4750
- **YAMAMOTO et al.** *Journal of Hepatology*, 2016, vol. 64, 547-555
- **SATO et al.** *Molecular Therapy*, 2016, vol. 24, 788-795
- **LEUNG et al.** *Journal of Physical Chemistry C Nanomater Interfaces*, 2012, vol. 116 (34), 18440-18450
- *CHEMICAL ABSTRACTS*, 77-86-1
- **LIMEI SHEN et al.** *Journal of Allergy*, 2018, vol. 142, 1558-1570
- **MONIKA BEDNARCZYK et al.** *Molecular Sciences*, vol. 22
- **EHRNTHALLER et al.** *Mol Med*, 2010, vol. 17 (3-4), 317-329
- **GHEBREHIWET et al.** *Immunol Rev*, 2002, vol. 180, 65-77